# EUROPEAN PATENT APPLICATION

(11) **EP 4 234 585 A1**
(43) Date of publication of application: **30.08.2023**
(21) Application number: 21882172.6
(22) Date of filing: 22.10.2021
(51) Int. Cl.: C07K 19/00, C12N 15/62, C12N 15/50, A61K 39/215, G01N 33/53, A61P 31/14

(54) **FUSION PROTEIN AND APPLICATION THEREOF**

(30) Priority: 23.10.2020 CN 202011149020
(71) Applicant: Jiangsu Provincial Center for Disease Control and Prevention (Public Health Research Institute of Jiangsu Province), Nanjing, Jiangsu 210009 (CN); Jiangsu Recbio Technology Co., Ltd., Taizhou, Jiangsu 225300 (CN); Beijing Abzymo Biosciences Co., Ltd., Beijing 100086 (CN)
(72) Inventor: ZHU, Fengcai, Nanjing, Jiangsu 210009 (CN); YU, Jiaping, Taizhou, Jiangsu 225300 (CN); YAO, Wenrong, Beijing 100086 (CN); YUAN, Chuxiao, Beijing 100086 (CN); WU, Shuang, Beijing 100086 (CN); ZHANG, Yanyan, Taizhou, Jiangsu 225300 (CN); HU, Yingsong, Beijing 100086 (CN); GUO, Xiling, Nanjing, Jiangsu 210009 (CN); ZHANG, Ling, Beijing 100086 (CN); XIA, Li, Beijing 100086 (CN); ZHANG, Li, Nanjing, Jiangsu 210009 (CN); LI, Jingxin, Nanjing, Jiangsu 210009 (CN); PAN, Hongxing, Nanjing, Jiangsu 210009 (CN); CHEN, Jianping, Beijing 100086 (CN); LIU, Yong, Beijing 100086 (CN)
(74) Representative: Weickmann, Hans
(86) International application number: PCT/CN2021/125834
(87) International publication number: WO 2022/083760

(57) **Abstract**

The present application relates to one or more fusion proteins, comprising a structural domain of SARS-CoV-2 S(Spike) protein. The present application also relates to an immunogenic composition comprising the fusion protein and an application thereof.

## Description

### ***FIELD OF THE INVENTION

The present application relates to the field of biomedicine, specifically to the fusion protein comprising the -receptor-binding domain (RBD) of the SARS-CoV-2 spike protein (S protein) or functionally active fragment thereof and/or the N-terminal domain (NTD) of the SARS-CoV-2 spike protein (S protein) or functionally active fragment of fusion protein, and its application thereof.

### ***BACKGROUND OF THE INVENTION

Quarantine, isolation and physical distancing are the current strategies to control COVID-19. An effective vaccine against COVID-19 is therefore urgently needed to reduce the enormous burden of mortality and morbidity associated with SARS-CoV-2 infection.

The -receptor-binding domain (RBD) of the spike protein (S protein) of SARS-CoV-2 is considered the most important antigen target region to induce the production of neutralizing antibodies. RBD as a vaccine can stimulate the body to produce neutralizing antibodies that can be more efficiently directed to the virus receptor binding, which can improve the immunogenicity and immunization efficiency of the vaccine.

The N-terminal domain (NTD) of SARS-CoV-2 spike protein (S protein) is a sequence at the N-terminal of the viral S protein, which can bind to the protein or glycoprotein of the host cell to mediate the invasion of the virus into the host cell, so this region may contain epitopes that induce the production of neutralizing antibodies.

### SUMMARY OF THE INVENTION

The present application provides a fusion protein comprising the receptor-binding domain (RBD) of SARS-CoV-2 spike protein (S protein) or functionally active fragment thereof and/or the N-terminal domain (NTD) of SARS-CoV-2 spike protein (S protein) or functionally active fragment thereof, its immunogenic compositions and applications thereof, which has one or more of the following properties: 1) the fusion protein is immunogenic; 2) the COVID-19 subunit vaccine comprising the fusion protein is more immunogenic and can induce more neutralizing antibodies.

In one aspect, the present application provides a fusion protein comprising: 1) the receptor-binding domain (RBD) of the SARS-CoV-2 spike protein (S protein) or functionally active fragment thereof; and 2) one or more polypeptides selected from the group consisting of P2 or functionally active fragment thereof, the foldon domain or functionally active fragment thereof, ferritin or functionally active fragment thereof, and hepatitis B surface antigen (HBsAg) or functionally active fragment thereof.

In some embodiments, said RBD is derived from wild-type SARS-CoV-2 or a variant thereof.

In some embodiments, said SARS-CoV-2 variant is selected from any one of the groups consisting of a Gamma variant, a Beta variant, a Delta variant, and an Alpha variant.

In some embodiments, said RBD has a mutation at one or more amino acid sites selected from the group consisting of K417, L452, T478, E484, and N501 compared to the RBD derived from wild-type SAR-CoV-2.

In some embodiments, said RBD comprises one or more of the amino acid mutations of K417T/N, L452R, T478K, E484K, and N501Y

In some embodiments, said RBD comprises K417T, E484K and N501Y amino acid mutations.

In some embodiments, said RBD comprises K417N, E484K, and N501Y amino acid mutations.

In some embodiments, said RBD comprises an amino acid sequence as set forth in any one of SEQ ID NOs. 18, 19, 76, 83, 97-108.

In some embodiments, said P2 or functionally active fragment thereof comprises an epitope peptide of tetanus toxin.

In some embodiments, the epitope peptide of said tetanus toxin comprises an amino acid sequence as set forth in any one of SEQ ID NOs. 64-66.

In some embodiments, said P2 or functionally active fragment thereof is directly or indirectly linked to the N-terminal or the C-terminal of said RBD or functionally active fragment thereof.

In some embodiments, said RBD or functionally active fragment thereof is fused in ORF with the P2 or functionally active fragment thereof.

In some embodiments, said foldon domain or functionally active fragment thereof comprises amino acid residues at the C-terminal domain of bacteriophage T4 fibrin.

In some embodiments, said foldon domain or functionally active fragment thereof comprises an amino acid sequence as set forth in any one of SEQ ID NOs. 67-69 and 78.

In some embodiments, said foldon domain or functionally active fragment thereof is directly or indirectly linked to the N-terminal or C-terminal of the RBD or functionally active fragment thereof. In some embodiments, said RBD or functionally active fragment thereof is fused in ORF with the foldon domain or functionally active fragment thereof.

In some embodiments, said ferritin or functionally active fragment thereof comprises *trichoplusia ni* ferritin or functionally active fragment thereof.

In some embodiments, said *trichoplusia ni* ferritin or functionally active fragment thereof comprises a heavy chain or a light chain of *trichoplusia ni* ferritin.

In some embodiments, said heavy chain of the *trichoplusia ni* ferritin comprises the amino acid sequence as set forth in SEQ ID NO. 70.

In some embodiments, said light chain of the *trichoplusia ni* ferritin comprises the amino acid sequence as set forth in SEQ ID NO. 71.

In some embodiments, said ferritin or functionally active fragment thereof comprises *Helicobacter pylori* ferritin or functionally active fragment thereof.

In some embodiments, said ferritin or functionally active fragment thereof comprises an amino acid sequence as set forth in any one of SEQ ID NOs. 70-72.

In some embodiments, said ferritin or functionally active fragment thereof is directly or indirectly linked to the N-terminal or the C-terminal of the RBD or functionally active fragment thereof.

In some embodiments, said RBD or functionally active fragment thereof is fused in ORF with the ferritin or functionally active fragment thereof.

In some embodiments, said hepatitis B surface antigen or functionally active fragment thereof comprises the amino acid sequence as set forth in SEQ ID NO. 73.

In some embodiments, said hepatitis B surface antigen or functionally active fragment thereof is directly or indirectly linked to the N-terminal or the C-terminal of said RBD or functionally active fragment thereof.

In some embodiments, said RBD or functionally active fragment thereof is fused in ORF with the hepatitis B surface antigen or functionally active fragment thereof.

In some embodiments, said fusion protein comprises an amino acid sequence as set forth in any one of SEQ ID NOs. 1-17, 80 and 86.

In another aspect, the present application provides a fusion protein comprising: 1) the N-terminal domain (NTD) of the SARS-CoV-2 spike protein (S protein) or functionally active fragment thereof; and 2) one or more polypeptides selected from the group consisting of P2 or functionally active fragment thereof, the foldon domain or functionally active fragment thereof, ferritin or functionally active fragment thereof, and hepatitis B surface antigen (HBsAg) or functionally active fragment thereof.

In some embodiments, said NTD is derived from wild-type SARS-CoV-2 or a variant thereof.

In some embodiments, said SARS-CoV-2 variant is selected from any one of the groups consisting of a Gamma variant, a Beta variant, a Delta variant, and an Alpha variant.

In some embodiments, said NTD comprises a mutation at one or more amino acid sites selected from the group consisting of L18, T19, T20, P26, D80, D138, R190, D215, L242-244, and R246.

In some embodiments, said NTD comprises the mutations of one or more amino acids selected from the groups consisting of L18F, T19R, T20N, P26S, D80A, D138Y, R190S, D215G, L242-244del, and R246I.

In some embodiments, said NTD comprises the mutations at L18F, D80A, D215G, L242-244del, and R246I.

In some embodiments, said NTD comprises L18F, T20N, P26S, D138Y, and R246I amino acid mutations.

In some embodiments, said NTD comprises an amino acid sequence as set forth in any one of SEQ ID NOs. 37, 38, 77, 84 and 109-111.

In some embodiments, said P2 or functionally active fragment thereof comprises an epitope peptide of tetanus toxin.

In some embodiments, said epitope peptide of the said tetanus toxin comprises an amino acid sequence shown in any one of SEQ ID NOs: 64-66.

In some embodiments, said P2 or functionally active fragment thereof is directly or indirectly linked to the N-terminal or the C-terminal of said NTD or functionally active fragment thereof.

In some embodiments, said NTD or functionally active fragment thereof is fused in ORF with said P2 or functionally active fragment thereof.

In some embodiments, said foldon domain or functionally active fragment thereof comprises amino acid residues at the C-terminal domain of bacteriophage T4 fibrin.

In some embodiments, said foldon domain or functionally active fragment thereof comprises an amino acid sequence as set forth in any one of SEQ ID NOs. 67-69 and 78.

In some embodiments, said foldon domain or functionally active fragment thereof is directly or indirectly linked to the N-terminal or C-terminal of the said NTD or functionally active fragment thereof.

In some embodiments, said NTD or functionally active fragment thereof is fused in ORF with the foldon domain or functionally active fragment thereof.

In some embodiments, said ferritin or functionally active fragment thereof comprises *trichoplusia ni* ferritin or functionally active fragment thereof.

In some embodiments, said *trichoplusia ni* ferritin or functionally active fragment thereof comprises a heavy chain or a light chain of *trichoplusia ni* ferritin.

In some embodiments, said heavy chain of the *trichoplusia ni* ferritin comprises the amino acid sequence as set forth in SEQ ID NO. 70.

In some embodiments, said light chain of the *trichoplusia ni* ferritin comprises the amino acid sequence as set forth in SEQ ID NO. 71.

In some embodiments, said ferritin or functionally active fragment thereof comprises *Helicobacter pylori* ferritin or functionally active fragment thereof.

In some embodiments, said ferritin or functionally active fragment thereof comprises an amino acid sequence as set forth in any one of SEQ ID NOs. 70-72.

In some embodiments, said ferritin or functionally active fragment thereof is directly or indirectly linked to the N-terminal or C-terminal of said NTD or functionally active fragment thereof.

In some embodiments, said NTD or functionally active fragment thereof is fused in ORF with the ferritin or functionally active fragment thereof.

In some embodiments, said hepatitis B surface antigen or functionally active fragment thereof comprises the amino acid sequence as set forth in SEQ ID NO. 73.

In some embodiments, said hepatitis B surface antigen or functionally active fragment thereof is directly or indirectly linked to the N-terminal or C-terminal of the said NTD or functionally active fragment thereof.

In some embodiments, said NTD or functionally active fragment thereof is fused in ORF with the hepatitis B surface antigen or functionally active fragment thereof.

In some embodiments, said fusion protein comprises an amino acid sequence as set forth in any one of SEQ ID NOs. 20-36, 81 and 87.

In another aspect, the present application provides a fusion protein comprising: 1) the receptor-binding domain (RBD) of the SARS-CoV-2 spike protein (S protein) or functionally active fragment thereof and 2) the N-terminal domain (NTD) of the SARS-CoV-2 spike protein (S protein) or functionally active fragment thereof.

In some embodiments, said fusion protein further includes one or more polypeptides selected from the following groups: P2 or functionally active fragment thereof, a foldon domain or functionally active fragment thereof, ferritin or functionally active fragment thereof, and hepatitis B surface antigen (HBsAg) or functionally active fragment thereof.

In some embodiments, said RBD is derived from wild-type SARS-CoV-2 or its variant thereof.

In some embodiments, said SARS-CoV-2 variant is selected from any one of the groups consisting of a Gamma variant, a Beta variant, a Delta variant, and an Alpha variant.

In some embodiments, said RBD has a mutation at one or more amino acid sites selected from the groups consisting of K417, L452, T478, E484, and N501 compared to said RBD derived from wild-type SAR-CoV-2.

In some embodiments, said RBD comprises one or more of the amino acid mutations of K417T/N, L452R, T478K, E484K, and N501Y

In some embodiments, said RBD comprises K417T, E484K and N501Y amino acid mutations.

In some embodiments, said RBD comprises K417N, E484K, and N501Y amino acid mutations.

In some embodiments, said RBD comprises an amino acid sequence as set forth in any one of SEQ ID NOs. 18, 19, 76, 83, 97-108.

In some embodiments, said NTD is derived from wild-type SARS-CoV-2 or its variant thereof.

In some embodiments, said SARS-CoV-2 variant is selected from any one of the groups consisting of a Gamma variant, a Beta variant, a Delta variant, and an Alpha variant.

In some embodiments, said NTD comprises a mutation at one or more amino acid sites selected from the groups consisting of L18, T19, T20, P26, D80, D138, R190, D215, L242-244, and R246.

In some embodiments, said NTD comprises the mutations of one or more amino acids selected from the groups consisting of L18F, T19R, T20N, P26S, D80A, D138Y, R190S, D215G, L242-244del, and R246I.

In some embodiments, said NTD comprises the mutations at L18F, D80A, D215G, L242-244del, and R246I.

In some embodiments, said NTD comprises L18F, T20N, P26S, D138Y, and R246I amino acid mutations.

In some embodiments, said NTD comprises an amino acid sequence as set forth in any one of SEQ ID NOs. 37, 38, 77, 84 and 109-111.

In some embodiments, said RBD or functionally active fragment thereof is directly or indirectly linked to said NTD or functionally active fragment thereof.

In some embodiments, said RBD or functionally active fragment thereof is fused in ORF with the NTD or functionally active fragment thereof.

In some embodiments, said P2 or functionally active fragment thereof comprises an epitope peptide of tetanus toxin.

In some embodiments, said epitope peptide of the said tetanus toxin comprises an amino acid sequence shown in any one of SEQ ID NOs. 64-66.

In some embodiments, said P2 or functionally active fragment thereof is directly or indirectly linked to said RBD or its functional fragment thereof and/or said NTD or functionally active fragment thereof.

In some embodiments, said P2 or functionally active fragment thereof is fused in ORF with said RBD or functionally active fragment thereof and/or said NTD or functionally active fragment thereof.

In some embodiments, said N-terminal of said P2 or functionally active fragment thereof is directly or indirectly linked to the C-terminal of said RBD or functionally active fragment thereof, and said N- terminal of said RBD or functionally active fragment thereof is directly or indirectly linked to the C-terminal of said NTD or functionally active fragment thereof.

In some embodiments, said foldon domain or functionally active fragment thereof comprises amino acid residues at the C-terminal domain of bacteriophage T4 fibrin.

In some embodiments, said amino acid residue at the C-terminal domain of bacteriophage T4 fibrin comprises the amino acid sequence as set forth in SEQ ID NO. 78.

In some embodiments, said foldon domain or functionally active fragment thereof comprises an amino acid sequence as set forth in any one of SEQ ID NOs. 67-69 and 78.

In some embodiments, said foldon domain or functionally active fragment thereof is directly or indirectly linked to said RBD or functionally active fragment thereof and/or said NTD or functionally active fragment thereof.

In some embodiments, said foldon domain or functionally active fragment thereof is fused in ORF with said RBD or functionally active fragment thereof and/or said NTD or functionally active fragment thereof.

In some embodiments, the N-terminal of the said folden domain or functionally active fragment thereof is directly or indirectly linked to the C-terminal of the RBD or functionally active fragment thereof, and the N-terminal of said RBD or functionally active N-terminal thereof is directly or indirectly linked to the C-terminal of said NTD or functionally active fragment thereof.

In some embodiments, the N-terminal of said foldon domain or functionally active fragment thereof is directly or indirectly linked to the C-terminal of said P2 or functionally active fragment thereof. The N-terminal of said P2 or functionally active fragment thereof is directly or indirectly linked to the C-terminal of said RBD or functionally active fragment thereof. The N-terminal of said RBD or functionally active fragment thereof is directly or indirectly linked to the C-terminal of the said NTD or functionally active fragment thereof.

In some embodiments, said ferritin or functionally active fragment thereof comprises *trichoplusia ni* ferritin or functionally active fragment thereof.

In some embodiments, said *trichoplusia ni* ferritin or functionally active fragment thereof comprises a heavy chain or a light chain of *trichoplusia ni* ferritin.

In some embodiments, said heavy chain of the *trichoplusia ni* ferritin comprises the amino acid sequence as set forth in SEQ ID NO. 70.

In some embodiments, said light chain of the *trichoplusia ni* ferritin comprises the amino acid sequence as set forth in SEQ ID NOs. 71.

In some embodiments, said ferritin or functionally active fragment thereof comprises *Helicobacter pylori* ferritin or functionally active fragment thereof.

In some embodiments, said ferritin or functionally active fragment thereof comprises an amino acid sequence as set forth in any one of SEQ ID NOs. 70-72.

In some embodiments, said ferritin or functionally active fragment thereof is directly or indirectly linked to said RBD or functionally active fragment thereof and/or said NTD or functionally active fragment thereof.

In some embodiments, said ferritin or functionally active fragment thereof is fused in ORF with said RBD or functionally active fragment thereof and/or said NTD or functionally active fragment thereof. In some embodiments, the N-terminal of said ferritin or functionally active fragment thereof is directly or indirectly linked to the C-terminal of said RBD or functionally active fragment thereof, and the N-terminal of the said RBD or functionally active fragment thereof is directly or indirectly linked to the C-terminal of said NTD or functionally active fragment thereof.

In some embodiments, said hepatitis B surface antigen or functionally active fragment thereof comprises the amino acid sequence as set forth in SEQ ID NO. 73.

In some embodiments, said hepatitis B surface antigen or functionally active fragment thereof is directly or indirectly linked to said RBD or functionally active fragment thereof and/or said NTD or functionally active fragment thereof.

In some embodiments, said hepatitis B surface antigen or functionally active fragment thereof is fused in ORF to said RBD or functionally active fragment thereof and/or said NTD or functionally active fragment thereof.

In some embodiments, the N-terminal of said the hepatitis B surface antigen or functionally active fragment thereof is directly or indirectly linked to the C-terminal of said RBD or functionally active fragment thereof, and the N-terminal of said RBD or functionally active fragment thereof is directly or indirectly linked to the C-terminal of said NTD or functionally active fragment thereof.

In some embodiments, said fusion protein comprises an amino acid sequence as set forth in any one of SEQ ID NOs. 39-44, 79, 85 and 96.

In another aspect, the present application provides an immunogenic composition comprising said fusion protein.

In some embodiments, said immunogenic composition comprises the first component, wherein said first component comprises the receptor-binding domain (RBD) of the SARS-CoV-2 spike protein (S protein) or functionally active fragment thereof, and the second component comprises a fusion protein. In some embodiments, said immunogenic composition comprises the first component, wherein said first component comprises a fusion protein, and the second component comprises a N-terminal domain (NTD) of SARS-CoV-2 spike protein (S protein) or functionally active fragment thereof.

In some embodiments, said immunogenic composition comprises the first component, wherein said first component comprises said fusion protein, and the second component, wherein said second component comprises said fusion protein.

In some embodiments, said immunogenic composition is formulated in the following weight ratios: 1) the first component (1-15 portions in weight ratio); and/or 2) the second component (1-15 portions in weight ratio).

In some embodiments, said immunogenic composition comprises: 1) 5-60 µg of the first component and/or 2) 5-60 µg of the second component.

In another aspect, the present application provides an immunogenic composition comprising the first component, wherein said first component comprises the receptor-binding domain (RBD) of the SARS-CoV-2 spike protein (S protein) or functionally active fragment thereof and a second component, wherein said second component comprise a N-terminal domain (NTD) of SARS-CoV-2 spike protein (S protein) or functionally active fragment thereof.

In some embodiments, said RBD is derived from wild-type SARS-CoV-2 or its variant thereof.

In some embodiments, said SARS-CoV-2 variant is selected from any one of the groups consisting of a Gamma variant, a Beta variant, a Delta variant, and an Alpha variant.

In some embodiments, said RBD has a mutation at one or more amino acid sites selected from the groups consisting of K417, L452, T478, E484, and N501 compared to the RBD derived from wild-type SAR-CoV-2.

In some embodiments, said RBD comprises one or more of the amino acid mutations K417T/N, L452R, T478K, E484K, and N501Y

In some embodiments, said RBD comprises K417T, E484K and N501Y amino acid mutations.

In some embodiments, said RBD comprises K417N, E484K, and N501Y amino acid mutations.

In some embodiments, said RBD comprises an amino acid sequence as set forth in any one of SEQ ID NOs. 18, 19, 76, 83, 97-108.

In some embodiments, said RBD is derived from wild-type SARS-CoV-2 or its variant thereof.

In some embodiments, said SARS-CoV-2 variant is selected from any one of the groups consisting of a Gamma variant, a Beta variant, a Delta variant, and an Alpha variant.

In some embodiments, said NTD comprises a mutation at one or more amino acid sites selected from the groups consisting of L18, T19, T20, P26, D80, D138, R190, D215, L242-244, and R246.

In some embodiments, said NTD comprises the mutations of one or more amino acids selected from the groups consisting of L18F, T19R, T20N, P26S, D80A, D138Y, R190S, D215G, L242-244del, and R246I.

In some embodiments, said NTD comprises the mutations at L18F, D80A, D215G, L242-244del, and R246I.

In some embodiments, said NTD comprises L18F, T20N, P26S, D138Y, and R246I amino acid mutations.

In some embodiments, said NTD comprises an amino acid sequence as set forth in any one of SEQ ID NOs. 37, 38, 77, 84 and 109-111.

In some embodiments, said immunogenic composition is formulated in the following weight ratios: 1) the first component (1-15 portions in weight ratio); and/or 2) the second component (1-15 portions in weight ratio).

In some embodiments, said immunogenic composition comprises: 1) 5-60 µg of the first component and/or 2) 5-60 µg of the second component.

In some embodiments, said RBD comprises an amino acid sequence as set forth in any one of SEQ ID NOs. 18, 19, 76, 83, 97-108.

In some embodiments, said NTD comprises an amino acid sequence as set forth in any one of SEQ ID NOs. 37-38, 77, 84 and 109-111.

In another aspect, the present application provides a pharmaceutical composition comprising said fusion protein, or said immunogenic composition, and optionally a pharmaceutically acceptable excipient.

In another aspect, the present application provides the use of said fusion protein or said immunogenic composition in the preparation of a vaccine.

In some embodiments, said vaccine is used to prevent and/or treat COVID-19.

In another aspect, the present application provides said fusion protein or said immunogenic composition for the treatment and/or prevention of COVID-19.

In another aspect, the present application provides a method for preparing a COVID-19 subunit vaccine which comprises:
1) providing said fusion protein or said immunogenic composition; and
2) mixing said fusion protein or immunogenic composition described in 1) with a pharmaceutically acceptable adjuvant.

In another aspect, the present application provides a method for detecting SARS-CoV-2 neutralizing antibodies which comprises:
1) administering the COVID-19 subunit vaccine to the subjects; and
2) detecting the neutralizing antibodies produced in said subjects described in 1) after receiving the COVID-19 subunit vaccine.

In another aspect, the present application provides a method of treating and/or preventing COVID-19 which comprises administering said fusion protein or said immunogenic composition or said COVID-19 subunit vaccine to the subjects.

Other aspects and advantages of the application can be readily appreciated by those skilled in the art from the detailed description below. Only examples of the present application are shown and described in the detailed description below. As those skilled in the art will perceive, the contents of the present application enable those skilled in the art to make modifications to the disclosed embodiments without departing from the spirit and scope of the invention contemplated in the present application. Accordingly, the descriptions in the drawings and specification of the present application are only exemplary and not limiting.

### ***BRIEF DESCRIPTION OF THE DRAWING

The specific features of the present invention to which the present application relates are shown in the attached claims. The features and advantages of the present invention can be better understood with reference to the examples and drawings described in detail below. A brief description of the attached figures is as follows:
Figure 1 shows the results of verification of RBD-P2-6*HIS protein by SDS-PAGE electrophoresis and Western blot.
Figure 2 shows the results of the verification of NTD-P2-6*HIS (GP101150-8) protein by SDS-PAGE electrophoresis and Western blot.
Figure 3 shows the results of verification of NTD-RBD-foldon-8*HIS protein by SDS-PAGE electrophoresis and Western blot.

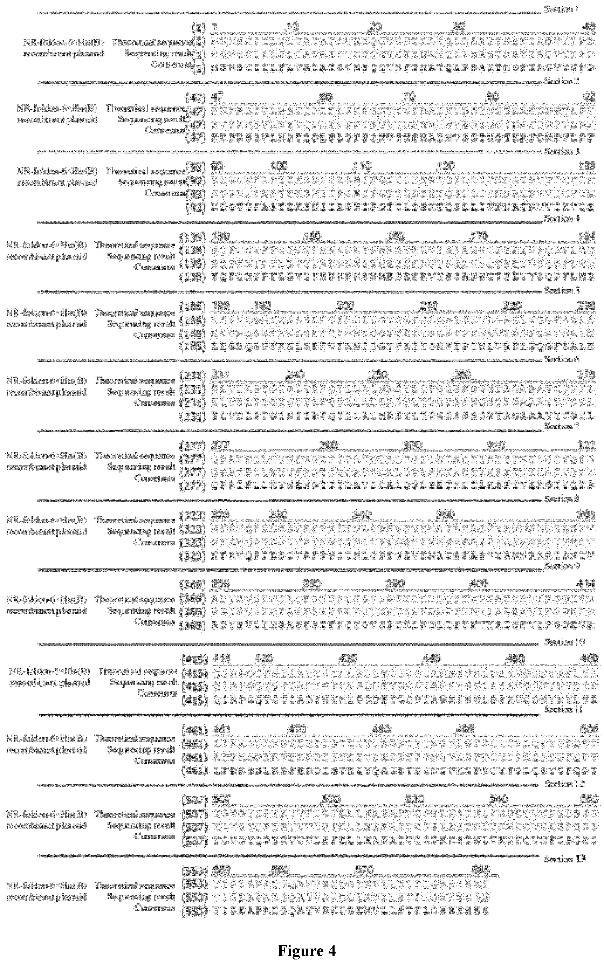

Figure 4 shows the amino acids sequence alignment of NTD-RBD-foldon-6 × His (Gamma variant) sequencing results.
Figure 5 shows the Size-exclusion chromatogram of the NTD-RBD-foldon (Gamma variant) protein.
Figure 6 shows the results of the verification of NTD-RBD-foldon (Gamma variant) protein by SDS-PAGE electrophoresis and Western blot.
Figure 7 shows the amino acids sequence alignment of NTD-RBD-foldon-6 × His (Beta variant) sequencing results.
Figure 8 shows the Size-exclusion chromatogram of the NTD-RBD-foldon (Beta variant) protein.
Figure 9 shows the results of the verification of NTD-RBD-foldon (Beta variant) protein by SDS-PAGE and Western blot.

### DETAILED DESCRIPTION

The embodiments of the present invention are described below by the specific examples, and other advantages and effects of the present invention can be readily appreciated by those familiar with the art from the contents disclosed in this invention.

### ***Definitions of Terms

In the present application, the term "fusion protein" generally refers to the protein molecule with biological functional activity obtained through genetic engineering techniques. In the present application, said fusion protein may be a fusion protein consisting of RBD or functionally active fragment thereof and any of the following sequences: P2 or functionally active fragment thereof, foldon domain or functionally active fragment thereof, ferritin or functionally active fragment thereof, hepatitis B surface antigen HBsAg or functionally active fragment thereof. In the present application, said fusion protein may be a fusion protein consisting of NTD or functionally active fragment thereof and any of the following sequences: P2 or functionally active fragment thereof, foldon domain or functionally active fragment thereof, ferritin or functionally active fragment thereof, hepatitis B surface antigen HBsAg or functionally active fragment thereof. In the present application, the fusion protein may be a fusion protein consisting of NTD or functionally active fragment thereof and RBD or functionally active fragment thereof. In the present application, said fusion protein may be a fusion protein consisting of NTD or functionally active fragment thereof, RBD or functionally active fragment, and any of the following sequences: P2 or functionally active fragment thereof, foldon domain or functionally active fragment thereof, ferritin or functionally active fragment thereof, hepatitis B surface antigen HBsAg or functionally active fragment thereof.

The whole genome sequence of the hCoV-19/WIV04/2019 (WIV04) virus strain (EPI_ISL_402124) is used as the formal reference sequence by the GISAID dataset. The WIV04 strain is generally defined as a wild-type or original virus strain. In the present application, the term "wild-type SARS-CoV-2" generally refers to the WIV04 virus strain.

In the present application, the term "P2" generally refers to epitope peptides of tetanus toxin. For example, P2 may be a peptide segment of the epitope peptides 830 to 844 of tetanus toxin or a variant thereof. For example, P2 may be a peptide segment of the epitope peptides 830 to 845 of tetanus toxin or a variant thereof. For example, P2 may be a peptide segment of the epitope peptides 829 to 844 of tetanus toxin or a variant thereof. For example, P2 may be a peptide segment obtained by truncating 1, 2, 3, 4, 5, or 6 or 10 amino acids of the N-terminal or C-terminal from the epitope peptides 829 to 844 of tetanus toxin or by adding those acids to the epitope peptides. For example, P2 may be a peptide segment obtained by truncating 1, 2, 3, 4, 5, or 6 or 10 amino acids of the N-terminal or C-terminal from the epitope peptides 830 to 845 of tetanus toxin or by adding those acids to the epitope peptides. For example, P2 may be a peptide segment obtained by truncating 1, 2, 3, 4, 5, or 6 or 10 amino acids of the N-terminal or C-terminal from the epitope peptides 830 to 844 of tetanus toxin or by adding those acids to the epitope peptides. In the present application, the term "variant" generally refers to a sequence that differs from the reference sequence by containing one or more differences (mutations). This difference may be a substitution, deletion, or insertion of one or more amino acids. In the present application, the term "foldon domain" generally refers to the residues at the C-terminal of bacteriophage T4 fibritin. In the present application, the foldon domain may be 27 residues or variants of the C-terminal of bacteriophage T4 fibritin. In the present application, the foldon domain may be a truncated or elongated form obtained by truncating or adding 1, 2, 3, 4, 5, or 6 or 10 amino acids at the N-terminal or C-terminal of 27 residues of bacteriophage T4 fibritin. In the present application, the term "variant" generally refers to a sequence that differs from the reference sequence by containing one or more differences (mutations). This difference may be a substitution, deletion, or insertion of one or more amino acids.

In the present application, the term "ferritin" generally refers to the ferritin from *trichoplusia ni* or *Helicobacter pylori.* In the present application, the ferritin from *trichoplusia ni* has a heavy chain and a light chain (ferritin HC and ferritin LC). In the present application, the ferritin from Helicobacter pylori has a single-chain structure. In the present application, the ferritin may be a variant of the ferritin from *trichoplusia ni* or *Helicobacter pylori.* In the present application, the term "variant" generally refers to a sequence that differs from the reference sequence by containing one or more differences (mutations). This difference may be a substitution, deletion, or insertion of one or more amino acids. In the present application, the "light chain of ferritin from *trichoplusia ni"* and "LC" can be interchangeably used with "ferritin LC". In the present application, the "heavy chain of ferritin from *trichoplusia ni"* and "HC" can be interchangeably used with "ferritin HC".

In the present application, the term "hepatitis B surface antigen (HBsAg)" generally refers to a capsid protein located in the outermost part of the envelope of hepatitis B virus. For example, the amino acid sequence of the hepatitis B surface antigen may be the sequence corresponding to the protein sequence accession number AAA45524, ANJ76941, CAA24234 or AAC34729 in the NCBI, or the sequence obtained by truncating or adding 1, 2, 3, 4, 5, 6 or 10 amino acids at the N-terminal or C-terminal, or a mutation in the protein, such as a deletion, substitution, or insertion of one or more amino acids as appropriate.

In the present application, the term "functionally active fragment" generally refers to a fragment with similar biological activity to the RBD, NTD, P2, foldon domain, ferritin or hepatitis B surface antigen HBsAg.

In the present application, the term "immunogenic composition" generally refers to a subunit composition. In the present application, the subunit composition refers to a composition in which the components have been isolated and purified to a purity of at least 50%, 60%, 70%, 80% or 90% prior to mixing the components to form an antigenic composition. For example, the subunit composition may be an aqueous solution of a water-soluble protein. For example, the subunit composition may comprise a detergent. For example, the subunit composition may comprise a non-ionic, zwitterionic, or ionic detergent. For example, the subunit composition may comprise a lipid. In some cases, the immunogenic composition may include an RBD or functionally active fragment thereof and an NTD or functionally active fragment thereof. In some cases, the immunogenic composition may include an RBD or functionally active fragment thereof and a fusion protein comprising an NTD or functionally active fragment thereof. In some cases, the immunogenic composition may include a fusion protein comprising an RBD or functionally active fragment thereof and an NTD or functionally active fragment thereof. In some cases, the immunogenic composition may include a fusion protein comprising an RBD or functionally active fragment thereof and a fusion protein comprising an NTD or functionally active fragment thereof. In the present application, the immunogenic composition may further include an adjuvant. For example, the said adjuvant may comprise an aluminum salt, such as aluminium hydroxide gel (alum) or aluminium phosphate, but it may also be a calcium salt, iron salt, or zinc salt, or it may be an insoluble suspension of an acylated tyrosine or an acylated sugar, a cationically or anionically derivatized polysaccharide, or a polyphosphazene. For example, the immunogenic composition may also be a selected preferential inducer of the Th1-type response. For example, the preferential inducer of Th1-type response may include monophosphoryl lipid A or a derivative thereof. For example, the adjuvant may be a composition of monophosphoryl lipid A (e.g., 3-de-O-acylated monophosphoryl lipid A (3D-MPL)) and an aluminium salt. An adjuvant enhancing system may comprise a composition of monophosphoryl lipid A and a saponin derivative, particularly the combination of QS21 and 3D-MPL as disclosed in WO 94/00153, or a composite in which QS21 is quenched with cholesterol to reduce the reactogenicity as disclosed in WO 96/33739. For example, the said adjuvant may also be the adjuvant disclosed in WO 95/17210, which contains QS21, 3D-MPL and tocopherol in an oil-in-water emulsion. For example, the said adjuvant may be a homogeneous droplet-like emulsion formed by mixing Tween 80, sorbitan trioleate and squalene and then microfluidizing under the high pressure condition. For example, the said adjuvant may comprise the unmethylated CpG of an oligonucleotide (WO96/02555).

In the present application, the term "weight ratio" generally refers to the ratio of the weight of the first component to the second component of the immunogenic composition. In the present application, the immunogenic composition may contain the following components per dose: 5-60 µg of the first component and 5-60 µg of the second component. For example, the immunogenic composition may comprise 5 µg, 10 µg, 20 µg, 30 µg or 40 µg of the first component. For example, the immunogenic composition may comprise 5 µg, 10 µg, 20 µg, 30 µg or 40 µg of the second component.

In the present application, the term "RBD" refers to the receptor-binding domain of the SARS-CoV-2 spike protein (S protein). In the present application, the RBD may be a peptide segment with the amino acids 310-560 of the SARS-CoV-2 spike protein (S protein), or a variant thereof, and it may also be a peptide segment obtained by truncating 1, 2, 3, 4, 5, 6, 7, 8, 9, 10, 11, 12, 13, 14, 15, 16, 17, 20, 25 or 30 amino acids at the N-terminal or C-terminal. In the present application, the said RBD may be a peptide segment with the amino acids 319-541 of the SARS-CoV-2 spike protein (S protein), or a variant thereof, and it may also be a peptide segment obtained by truncating the amino acids 1, 2, 3, 4, 5, 6, 7, 8, 9, 10, 11, 12, 13, 14, 15, 16, 17, 20, 25 or 30 at the N-terminal or C-terminal as appropriate. In the present application, the said RBD may be a peptide segment with the amino acids 331-524 of the SARS-CoV-2 spike protein (S protein), or a variant thereof, and it may also be a peptide segment obtained by truncating 1, 2, 3, 4, 5, 6, 7, 8, 9, 10, 11, 12, 13, 14, 15, 16, 17, 20, 25 or 30 amino acids at the N-terminal or C-terminal as appropriate. In the present application, the term "variant" generally refers to a sequence that differs from the reference sequence by containing one or more differences (mutations). This difference may be a substitution, deletion, or insertion of one or more amino acids.

In some embodiments, said RBD may comprise the RBD of SARS-CoV-2 (Gamma variant).

In some embodiments, said RBD may comprise the RBD of SARS-CoV-2 (Beta variant).

In some embodiments, said RBD may comprise the RBD of SARS-CoV-2 (WIV04-1).

In the present application, the term "NTD" refers to the domain at the N-terminal of the SARS-CoV-2 spike protein (S protein). In the present application, said NTD may be a peptide segment with the amino acids 13-353 of the SARS-CoV-2 spike protein (S protein), or a variant thereof, and it may also be a peptide segment obtained by truncating 1, 2, 3, 4, 5, 6, 7, 8, 9, 10, 11, 12, 13, 14, 15, 16, 17, 20, 25 or 30 amino acids at the N-terminal or C-terminal as appropriate. For example, the NTD may be a peptide segment with amino acids 13-303 of the SARS-CoV-2 spike protein (S protein), or a variant thereof. In the present application, the said NTD may be a peptide segment with amino acids 14-304 of SARS-CoV-2 spike protein (S protein), or a variant thereof. In the present application, said NTD may be a peptide segment with amino acids 18-353 of the SARS-CoV-2 spike protein (S protein), or a variant thereof. In the present application, the term "variant" generally refers to a sequence that differs from the reference sequence by containing one or more differences (mutations). This difference may be a substitution, deletion, or insertion of one or more amino acids. In some embodiments, said NTD may comprise the NTD of SARS-CoV-2 (Gamma variant). In some embodiments, said NTD may comprise the NTD of SARS-CoV-2 (Beta variant). In some embodiments, the NTD may comprise the NTD of SARS-CoV-2 (WIV04-1).

In the present application, the term "S protein", also known as "Spike protein", generally refers to the glycoprotein on the capsid surface of a coronavirus. SARS-CoV-2 invades cells by binding its S protein to ACE2. The S protein is composed of 1213 amino acids and contains a transmembrane domain, including the peptide segment with the amino acids from the N-terminal or the 14^{th} position to at least 1213^{th} position of the coronavirus capsid surface glycoprotein, or the corresponding region from other SARS viruses. The S1 protein is the subunit 1 of S protein, which mainly refers to the segment with the amino acids from the N-terminal or the 14^{th} position to the 685^{th} position amino acid.

In the present application, said variant may comprise any one or several currently known SAR-CoV-2 variants (mutations). For example, for the said Gamma variant, the amino acid sequence of glycoprotein on the surface of SARS-CoV-2 of the lineage B.1.1.28 with GenBank accession number QRN46961.1 can be referred to. For example, for the said Gamma variant, the amino acid sequence of glycoprotein on the surface of SARS-CoV-2 of the lineage B.1.1.28 with GenBank accession number QLF80256.1 can be referred to. For example, for the said Gamma variant, the amino acid sequence of glycoprotein on the surface of SARS-CoV-2 of the lineage P.1 with GenBank accession number QVE55289.1 can be referred to. For example, the said Gamma variant may be a Gamma variant 501YV3, with the amino acid sequence of the glycoprotein on its surface as set forth in SEQ ID NO. 88.

For example, for the said Beta variant, the amino acid sequence of glycoprotein on the surface of SA RS-CoV-2 of the lineage B.1.351 with GenBank accession number QUA12570.1 can be referred to. For example, for the said Beta variant, the amino acid sequence of glycoprotein on the surface of SA RS-CoV-2 of the lineage B.1 with GenBank accession number QIZ15537.1 can be referred to. For e xample, for the said Beta variant, the amino acid sequence of glycoprotein on the surface of SARS-CoV-2 of the lineage B.1.351 with GenBank accession number QVI03430.1 can be referred to. For example, the said Beta variant may be a Beta variant 501YV2, with the amino acid sequence of the glycoprotein on its surface as set forth in SEQ ID NO. 82.

The term "hemagglutinin/hemagglutinin protein/HA" generally refers to antibodies or other substan ces that can agglutinate red blood cells, which can be found on the surface of influenza viruses, mea sles viruses (as well as many other bacteria and viruses), and they can attach to red blood cells of va rious animals, causing red blood cells to agglutinate and, in severe cases, causing death. Herein the t erm refers to hemaglutinin (HA), the surface glycoprotein of the influenza virus. The WHO official website will publish the forecast of the sequence of the influenza virus of the current year on a yearl y basis. The sequence of the influenza virus referenced in this article is that of the Northern hemisph ere published in 2021-2022, namely influenza virus type A (H1N1) EPI1661231|HA|A/Wisconsin/5 88/2019|EPI_ISL_404460, type A(H3N2)EPI1843589|HA|A/Cambodia/e0826360/2020|EPI_ISL_9 44639, type B EPI1394970|HA|B/Washington/02/2019|EPI_ISL_347829 and type B EPI529345|HA |B/PHUKET/3073/2013|EPI_ISL_161843 which can be accessed on the GISAID official website. In the present application, the term "comprising" generally refers to the meaning of including, summing up, containing and inclusive. In some cases, it also means "as" and "consisting of".

In the present application, the term "approximately" generally refers to the variation that changes within the range of 0.5% to 10% above or below a specified value, such as the variations within the ranges of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

In the application, the term "vector" generally refers to a nucleic acid molecule capable of self-replicating in a suitable host, which transfers the inserted nucleic acid molecule into and/or between the host cells. The said vector may include vectors primarily for inserting DNA or RNA into a cell, vectors primarily for replicating DNA or RNA, and vectors primarily for the transcription and/or translation of the expression of DNA or RNA. The said vector may further comprise a vector having a plurality of the above functions. The said vector may be a polynucleotide capable of being transcribed and translated into a polypeptide when introduced into a suitable host cell. Typically, the said vector can produce the desired expression product by culturing a suitable host cell comprising the said vector.

In the present application, the term "host cell" generally refers to a plasmid or vector of the nucleic acid molecules described in the present application or the individual cells, cell line or cell culture that are capable of expressing the said antibodies or antigen-binding fragment thereof described in the present application. The said host cell may comprise the progeny of a single host cell. The progeny cell may not necessarily be morphologically or genomically identical to the original parent cell as a result of a natural, accidental or deliberate mutation, but is capable of expressing the fusion protein described in the present application. The host cells can be obtained by in vitro transfection of the cells using the vectors described in the present application. The host cell may be a prokaryotic cell (e.g., Escherichia coli) or a eukaryotic cell (e.g., a yeast cell, e.g., a COS cell, a Chinese Hamster Ovary (CHO) cell, a HeLa cell, a HEK293 cell, a COS-1 cell, an NS0 cell, or a myeloma cell). In some embodiments, the said host cell is a mammalian cell. For example, the said mammalian cell may be a CHO cell.

### Detailed Description of the Invention

### Fusion protein

In one aspect, the present application provides a fusion protein comprising: 1) the receptor-binding domain (RBD) of the SARS-CoV-2 spike protein (S protein) or functionally active fragment thereof; and 2) one or more polypeptides selected from the groups consisting of P2 or functionally active fragment thereof, foldon domain or functionally active fragment thereof, ferritin or functionally active fragment thereof, and hepatitis B surface antigen (HBsAg) or functionally active fragment thereof. In the present application, said RBD or functionally active fragment thereof may be derived from the RBD of the wild-type S protein of the SARS-CoV-2 or from the RBD of a variant S protein of SARS-CoV-2. In some embodiments, said RBD or functionally active fragment thereof may comprise an amino acid variant at one or more of the amino acid sites of K417, L452, T478, E484 and N501.

In the present application, said RBD or functionally active fragment thereof may comprise the RBD of the S protein of the Gamma variant. In some embodiments, said RBD or functionally active fragment thereof may comprise one or more amino acid variants of K417T, L452R, T478K, E484K and N501Y. For example, said RBD may comprise the amino acid sequence as set forth in SEQ ID NO. 83. In the present application, said RBD or functionally active fragment thereof may comprise the RBD of the S protein of the Beta variant. In some embodiments, the RBD or functionally active fragment thereof may comprise one or more amino acid variants of K417N, E484K and N501Y. For example, said RBD may comprise the amino acid sequence as set forth in SEQ ID NO. 76.

In the present application, said P2 or functionally active fragment thereof may comprise an epitope peptide of tetanus toxin.

For example, P2 may be a peptide segment of the epitope peptides 830 to 844 of tetanus toxin or a variant thereof. For example, P2 may be a peptide segment of the epitope peptides 830 to 845 of tetanus toxin or a variant thereof. For example, P2 may be a peptide segment of the epitope peptides 829 to 844 of tetanus toxin or a variant thereof. For example, P2 may be a peptide segment obtained by truncating 1, 2, 3, 4, 5, or 6 or 10 amino acids of the N-terminal or C-terminal from the epitope peptides 829 to 844 of tetanus toxin or by adding those acids to the epitope peptides. For example, P2 may be a peptide segment obtained by truncating 1, 2, 3, 4, 5, or 6 or 10 amino acids of the N-terminal or C-terminal from the epitope peptides 830 to 845 of tetanus toxin or by adding those acids to the epitope peptides. For example, P2 may be a peptide segment obtained by truncating 1, 2, 3, 4, 5, or 6 or 10 amino acids of the N-terminal or C-terminal from the epitope peptides 830 to 844 of tetanus toxin or by adding those acids to the epitope peptides. In the present application, the term "variant" generally refers to a sequence that differs from the reference sequence by containing one or more differences (mutations). This difference may be the substitution, deletion, or insertion of one or more amino acids. For example, the epitope peptide of said tetanus toxin comprises an amino acid sequence as set forth in any one of SEQ ID NOs: 64-66.

In the present application, said P2 or functionally active fragment thereof is directly or indirectly linked to the N-terminal or the C-terminal of the RBD or functionally active fragment thereof.

In the present application, said P2 or functionally active fragment thereof may be directly or indirectly linked to the N-terminal of the RBD or functionally active fragment thereof.

In the present application, said P2 or functionally active fragment thereof is directly or indirectly linked to the C-terminal of the RBD or functionally active fragment thereof.

In the present application, said RBD or functionally active fragment thereof is fused in ORF with the said P2 or functionally active fragment thereof.

In the present application, said foldon domain or functionally active fragment thereof may comprise amino acid residues at the C-terminal domain of bacteriophage T4 fibrin.

In the present application, said foldon domain or functionally active fragment thereof may comprise 27 amino acid residues of the C-terminal domain of bacteriophage T4 fibrin or variants thereof. In the present application, the foldon domain may be the truncated or elongated forms obtained by truncating or adding 1, 2, 3, 4, 5, or 6 or 10 amino acids at the N-terminal or C-terminal of 27 residues of bacteriophage T4 fibritin. In the present application, the term "variant" generally refers to a sequence that differs from the reference sequence by containing one or more differences (mutations). This difference may be the substitution, deletion, or insertion of one or more amino acids.

For example, said foldon domain or functionally active fragment thereof may comprise an amino acid sequence as set forth in any one of SEQ ID NOs. 67-69 and 78.

In the present application, said foldon domain or functionally active fragment thereof may be directly or indirectly linked to the N-terminal or C-terminal of said RBD or functionally active fragment thereof.

In the present application, said foldon domain or functionally active fragment thereof may be directly or indirectly linked to the N-terminal of said RBD or functionally active fragment thereof.

In the present application, said foldon domain or functionally active fragment thereof may be directly or indirectly linked to the C-terminal of said RBD or functionally active fragment thereof.

In the present application, said RBD or functionally active fragment thereof may be fused in the ORF to said foldon domain or functionally active fragment thereof.

In the present application, said ferritin or functionally active fragment thereof comprises *trichoplusia ni* ferritin or functionally active fragment thereof.

In the present application, said *trichoplusia ni* ferritin or functionally active fragment thereof may comprise a heavy chain or a light chain of *trichoplusia ni* ferritin.

In the present application, the ferritin may be a variant of the ferritin from *trichoplusia ni* or *Helicobacter pylori.* In the present application, the term "variant" generally refers to a sequence that differs from the reference sequence by containing one or more differences (mutations). This difference may be the substitution, deletion, or insertion of one or more amino acids. In the present application, the "light chain of ferritin from *trichoplusia ni"* and "LC" can be interchangeably used with "ferritin LC". In the present application, the "heavy chain of ferritin from *trichoplusia ni"* and "HC" can be interchangeably used with "ferritin HC". In the present application, "*Helicobacter pylori* ferritin" can be used interchangeably with "HP ferritin".

In the present application, said heavy chain of the *trichoplusia ni* ferritin may comprise an amino acid sequence as set forth in SEQ ID NO. 70.

In the present application, said light chain of the *trichoplusia ni* ferritin may comprise an amino acid sequence as set forth in SEQ ID NO. 71.

In the present application, said ferritin or functionally active fragment thereof may comprise the *Helicobacter pylori* ferritin or a functionally active fragment thereof.

In the present application, said *Helicobacter pylori* ferritin or functionally active fragment thereof may comprise the amino acid sequence shown in SEQ ID NO. 72.

In the present application, said ferritin or functionally active fragment thereof comprises an amino acid sequence shown in any one of SEQ ID NOs. 70-72.

In the present application, said ferritin or functionally active fragment thereof may be directly or indirectly linked to the N-terminal or C-terminal of said RBD or functionally active fragment thereof. For example, said ferritin or functionally active fragment thereof may be directly or indirectly linked to the N-terminal of said RBD or functionally active fragment thereof.

For example, said ferritin or functionally active fragment thereof may be directly or indirectly linked to the C-terminal of said RBD or functionally active fragment thereof.

In the present application, said RBD or functionally active fragment thereof may be fused in the ORF to the ferritin or functionally active fragment thereof.

In the present application, the term " hepatitis B surface antigen (HBsAg) " generally refers to a capsid protein located in the outermost part of the envelope of hepatitis B virus. For example, the amino acid sequence of the hepatitis B surface antigen may be the sequence corresponding to the protein sequence accession number AAA45524, ANJ76941, CAA24234 or AAC34729 in the NCBI, or the sequence obtained by truncating or adding 1, 2, 3, 4, 5, 6 or 10 amino acids at the N-terminal or C-terminal, or a variant in the protein, such as the deletion, substitution, or insertion of one or more amino acids as appropriate.

For example, said hepatitis B surface antigen (HBsAg) or functionally active fragment thereof comprises the amino acid sequence as set forth in SEQ ID NO. 73.

In the present application, said hepatitis B surface antigen (HBsAg) or functionally active fragment thereof may be directly or indirectly linked to the N-terminal or C-terminal of said RBD or functionally active fragment thereof.

In the present application, said hepatitis B surface antigen (HBsAg) or functionally active fragment thereof may be directly or indirectly linked to the N-terminal of said RBD or functionally active fragment thereof.

In the present application, said hepatitis B surface antigen (HBsAg) or functionally active fragment thereof may be directly or indirectly linked to the C-terminal of said RBD or functionally active fragment thereof.

In the present application, said RBD or functionally active fragment thereof may be fused in the ORF to said hepatitis B surface antigen (HBsAg) or functionally active fragment thereof.

In the present application, said "direct or indirect linking" generally refers to two different linking modes, i.e., the direct linking and indirect linking, wherein the direct linking refers to the linking between two sequences without any other artificially added sequences, such as a flexible linker, a rigid linker or a splicing linker, etc. Indirect linking means that two sequences are linked by artificially adding the linking sequences, such as a flexible linker, a rigid linker or a splicing linker. In some embodiments, said flexible linker may comprise the amino acid sequence of GSGSG (SEQ ID NO. 89). In some embodiments, the said linking sequence may comprise the amino acid sequence of SFTVEKGIYQTSNF (SEQ ID NO. 90).

In the present application, said fusion protein may further comprise a signal peptide. In some embodiments, said signal peptide sequence of MGWSCIILFLVATATGVHS (SEQ ID NO. 91) may be added to the N-terminal of said fusion protein.

In the present application, said fusion protein may comprise an amino acid sequence as set forth in any one of SEQ ID NOs. 1-17, 80 and 86.

In another aspect, the present application provides a fusion protein comprising: 1) the N-terminal domain (NTD) of the SARS-CoV-2 spike protein (S protein) or functionally active fragment thereof; and 2) one or more polypeptides selected from the groups consisting of P2 or functionally active fragment thereof, the foldon domain or functionally active fragment thereof, ferritin or functionally active fragment thereof, and hepatitis B surface antigen (HBsAg) or functionally active fragment thereof.

In the present application, said NTD or functionally active fragment thereof may be derived from the NTD of the wild-type S protein of the SARS-CoV-2 or from the NTD of the variant S protein of SARS-CoV-2. In some embodiments, said NTD or functionally active fragment thereof may comprise amino acid variant at one or more of the amino acid sites of L18, T19, T20, P26, D80, D138, R190, D215, L242-244 and R246. In some embodiments, said NTD or functionally active fragment thereof may comprise one or more amino acid variants of L18F, T20N, P26S, D80A, D138Y, R190S, D215G, L242-244del and R246I.

In the present application, said NTD or functionally active fragment thereof may comprise the NTD of the S protein of the Gamma variant. In some embodiments, said NTD may comprise the mutations at the amino acids of L18F, T20N, P26S, D138Y, and R190S. For example, said NTD may comprise the amino acid sequence as set forth in SEQ ID NO. 84.

In the present application, said NTD or functionally active fragment thereof may comprise, for example, the NTD of the S protein of a Beta variant. In some embodiments, said NTD may comprise mutations at L18F, D80A, D215G, L242-244del, and R246I. In some embodiments, said NTD may comprise the amino acid sequence as set forth in SEQ ID NO. 77.

In the present application, said P2 or functionally active fragment thereof may comprise an epitope peptide of tetanus toxin.

For example, P2 may be the peptide segment of the epitope peptides 830 to 844 of tetanus toxin or a variant thereof. For example, P2 may be a peptide segment of the epitope peptides 830 to 845 of tetanus toxin or a variant thereof. For example, P2 may be a peptide segment of the epitope peptides 829 to 844 of tetanus toxin or a variant thereof. For example, P2 may be a peptide segment obtained by truncating 1, 2, 3, 4, 5, or 6 or 10 amino acids of the N-terminal or C-terminal from the epitope peptides 829 to 844 of tetanus toxin or by adding those acids to the epitope peptides. For example, P2 may be a peptide segment obtained by truncating 1, 2, 3, 4, 5, or 6 or 10 amino acids of the N-terminal or C-terminal from the epitope peptides 830 to 845 of tetanus toxin or by adding those acids to the epitope peptides. For example, P2 may be a peptide segment obtained by truncating 1, 2, 3, 4, 5, or 6 or 10 amino acids of the N-terminal or C-terminal from the epitope peptides 830 to 844 of tetanus toxin or by adding those acids to the epitope peptides. In the present application, the term "variant" generally refers to a sequence that differs from the reference sequence by containing one or more differences (mutations). This difference may be the substitution, deletion, or insertion of one or more amino acids. For example, the epitope peptide of the said tetanus toxin comprises an amino acid sequence as set forth in any one of SEQ ID NOs. 64-66.

In the present application, said P2 or functionally active fragment thereof may be directly or indirectly linked to the N-terminal or the C-terminal of said NTD or functionally active fragment thereof.

In the present application, said P2 or functionally active fragment thereof may be directly or indirectly linked to the N-terminal of said NTD or functionally active fragment thereof.

In the present application, said P2 or functionally active fragment thereof is directly or indirectly linked to the C-terminal of said NTD or functionally active fragment thereof.

In the present application, said NTD or functionally active fragment thereof may be fused in the ORF to said P2 or functionally active fragment thereof.

In the present application, said foldon domain or functionally active fragment thereof may comprise amino acid residues at the C-terminal domain of bacteriophage T4 fibrin. In some embodiments, the amino acid residue at the C-terminal of said bacteriophage T4 fibrin may comprise a amino acid sequence as set forth in SEQ ID NO. 78.

In the present application, said foldon domain or functionally active fragment thereof may comprise 27 amino acid residues or mutations at the C-terminal domain of bacteriophage T4 fibrin. In the present application, foldon domain may be the truncated or elongated forms obtained by truncating or adding 1, 2, 3, 4, 5, or 6 or 10 amino acids at the N-terminal or C-terminal of 27 residues of bacteriophage T4 fibritin. In the present application, the term "variant" generally refers to a sequence that differs from the reference sequence by containing one or more differences (mutations). This difference may be the substitution, deletion, or insertion of one or more amino acids.

For example, said foldon domain or functionally active fragment thereof may comprise an amino acid sequence as set forth in any one of SEQ ID NOs. 67-69 and 78.

In the present application, said foldon domain or functionally active fragment thereof may be directly or indirectly linked to the N-terminal or C-terminal of said NTD or a functionally active fragment thereof.

In the present application, said foldon domain or functionally active fragment thereof may be directly or indirectly linked to the N-terminal of said NTD or a functionally active fragment thereof.

In the present application, said foldon domain or functionally active fragment thereof may be directly or indirectly linked to the C-terminal of said RBD or a functionally active fragment thereof.

In the present application, said NTD or functionally active fragment thereof may be fused in the ORF to said foldon domain or functionally active fragment thereof.

In the present application, said ferritin or functionally active fragment thereof comprises *trichoplusia ni* ferritin or functionally active fragment thereof.

In the present application, said *trichoplusia ni* ferritin or functionally active fragment thereof may comprise a heavy chain or a light chain of *trichoplusia ni* ferritin.

In the present application, said ferritin may be a variant of the ferritin from *trichoplusia ni* or *Helicobacter pylori.* In the present application, the term "variant" generally refers to a sequence that differs from the reference sequence by containing one or more differences (mutations). This difference may be the substitution, deletion, or insertion of one or more amino acids. In the present application, the "light chain of ferritin from *trichoplusia ni"* and "LC" can be interchangeably used with "ferritin LC". In the present application, the "heavy chain of ferritin from *trichoplusia ni"* and "HC" can be interchangeably used with "ferritin HC". In the present application, "*Helicobacter pylori* ferritin" can be used interchangeably with "HP ferritin".

In the present application, said heavy chain of the *trichoplusia ni* ferritin may comprise the amino acid sequence as set forth in SEQ ID NO. 70.

In the present application, said light chain of the *trichoplusia ni* ferritin may comprise the amino acid sequence as set forth in SEQ ID NO. 71.

In the present application, said ferritin or functionally active fragment thereof may comprise the *Helicobacter pylori* ferritin or a functionally active fragment thereof.

In the present application, said *Helicobacter pylori* ferritin or functionally active fragment thereof may comprise the amino acid sequence shown in SEQ ID NO. 72.

In the present application, the ferritin or functionally active fragment thereof comprises an amino acid sequence as set forth in any one of SEQ ID NOs. 70-72.

In the present application, said ferritin or functionally active fragment thereof may be directly or indirectly linked to the N-terminal or the C-terminal of said NTD or functionally active fragment thereof.

For example, said ferritin or functionally active fragment thereof may be directly or indirectly linked to the N-terminal of said NTD or functionally active fragment thereof.

For example, said ferritin or functionally active fragment thereof may be directly or indirectly linked to the C-terminal of said NTD or functionally active fragment thereof.

In the present application, said NTD or functionally active fragment thereof may be fused in the ORF to the ferritin or functionally active fragment thereof.

In the present application, the term "hepatitis B surface antigen (HBsAg)" generally refers to a capsid protein located in the outermost part of the envelope of hepatitis B virus. For example, the amino acid sequence of the hepatitis B surface antigen may be a sequence corresponding to the protein sequence accession number AAA45524, ANJ76941, CAA24234 or AAC34729 in NCBI, or the sequence obtained by truncating or adding 1, 2, 3, 4, 5, 6 or 10 amino acids at the N-terminal or C-terminal, or a mutation in the protein, such as the deletion, substitution, or insertion of one or more amino acids as appropriate.

For example, said hepatitis B surface antigen (HBsAg) or functionally active fragment thereof comprises the amino acid sequence as set forth in SEQ ID NO. 73.

In the present application, said hepatitis B surface antigen (HBsAg) or functionally active fragment thereof may be directly or indirectly linked to the N-terminal or the C-terminal of said NTD or functionally active fragment thereof.

In the present application, said hepatitis B surface antigen (HBsAg) or functionally active fragment thereof may be directly or indirectly linked to the N-terminal of said NTD or functionally active fragment thereof.

In the present application, said hepatitis B surface antigen (HBsAg) or functionally active fragment thereof may be directly or indirectly linked to the C-terminal of said NTD or functionally active fragment thereof.

In the present application, said NTD or functionally active fragment thereof may be fused in the ORF to the hepatitis B surface antigen (HBsAg) or functionally active fragment thereof.

In the present application, said "direct or indirect linking" generally refers to two different linking modes, i.e. the direct linking and indirect linking, wherein the direct linking refers to the linking between two sequences without any other artificially added sequences, such as a flexible linker, a rigid linker or a splicing linker, etc. Indirect linking means that two sequences are linked by artificially adding the linking sequences, such as a flexible linker, a rigid linker or a splicing linker. In some embodiments, said flexible linker may comprise an amino acid sequence of GSGSG (SEQ ID NO. 89). In some embodiments, the said linking sequence may comprise the amino acid sequence of SFTVEKGIYQTSNF (SEQ ID NO. 90).

In the present application, said fusion protein may further comprise a signal peptide. In some embodiments, the signal peptide sequence of MGWSCIILFLVATATGVHS (SEQ ID NO. 91) may be added to the N-terminal of said fusion protein.

In the present application, said fusion protein may comprise an amino acid sequence shown in any one of SEQ ID NOs. 20-36, 81 and 87.

In another aspect, the present application provides a fusion protein comprising: 1) the receptor-binding domain (RBD) of the SARS-CoV-2 spike protein (S protein) or a functionally active fragment thereof and 2) the N-terminal domain (NTD) of the SARS-CoV-2 spike protein (S protein) or functionally active fragment thereof.

In the present application, said RBD or functionally active fragment thereof may be derived from the RBD of the wild-type S protein of the SARS-CoV-2 or from the RBD of the variant S protein of SARS-CoV-2. In some embodiments, the said RBD or functionally active fragment thereof may comprise an amino acid variant at one or more of the amino acid sites of K417, L452, T478, E484 and N501.

In the present application, said RBD or a functionally active fragment thereof may comprise the RBD of the S protein of the Gamma variant. In some embodiments, said RBD or functionally active fragment thereof may comprise one or more amino acid variants of K417T, E484K and N501Y. For example, said RBD may comprise an amino acid sequence as set forth in SEQ ID NO. 83. In the present application, said RBD or functionally active fragment thereof may comprise the RBD of the S protein of the Beta variant. In some embodiments, said RBD or functionally active fragment thereof may comprise one or more amino acid variants of K417N, E484K and N501Y. For example, said RBD may comprise the amino acid sequence as set forth in SEQ ID NO. 76.

In the present application, said NTD or functionally active fragment thereof may be derived from the NTD of the wild-type S protein of the SARS-CoV-2 or from the NTD of the variant S protein of SARS-CoV-2. In some embodiments, said NTD or functionally active fragment thereof may comprise amino acid mutation at one or more of the amino acid sites of L18, T19, T20, P26, D80, D138, R190, D215, L242-244 and R246. In some embodiments, said NTD or functionally active fragment thereof may comprise one or more amino acid mutations of L18F, T19R, T20N, P26S, D80A, D138Y, R190S, D215G, L242-244del and R246I.

In the present application, said NTD or functionally active fragment thereof may comprise the NTD of the S protein of the Gamma variant. In some embodiments, said NTD may comprise the mutations of L18F, T20N, P26S, D138Y, and R190S. For example, the said NTD may comprise the amino acid sequence shown in SEQ ID NO. 84.

In the present application, said NTD or functionally active fragment thereof may comprise the NTD of the S protein of a Beta variant. In some embodiments, said NTD may comprise mutations of L18F, D80A, D215G, L242-244del, and R246I. For example, said NTD may comprise the amino acid sequence as set forth in SEQ ID NO. 77.

In the present application, said RBD or functionally active fragment thereof is directly or indirectly linked to said NTD or the functionally active fragment thereof.

In the present application, said N-terminal of the said RBD or functionally active fragment thereof is directly or indirectly linked to the C-terminal of said NTD or functionally active fragment thereof. In the present application, said C-terminal of the said RBD or functionally active fragment thereof is linked directly or indirectly to the N-terminal of said NTD or functionally active fragment thereof. In the present application, said RBD or functionally active fragment thereof is fused in the ORF to said NTD or functionally active fragment thereof.

In the present application, said fusion protein may also comprise one or more polypeptides selected from the following groups: P2 or a functionally active fragment thereof, foldon domain or a functionally active fragment thereof, ferritin or a functionally active fragment thereof, and hepatitis B surface antigen (HBsAg) or a functionally active fragment thereof.

In the present application, said P2 or functionally active fragment thereof may comprise an epitope peptide of tetanus toxin.

For example, P2 may be a peptide segment of the epitope peptides 830-844 of tetanus toxin or a variant thereof. For example, P2 may be a peptide segment of the epitope peptides 830-845 of tetanus toxin or a variant thereof. For example, P2 may be a peptide segment of the epitope peptides 829-844 of tetanus toxin or a variant thereof. For example, P2 may be a peptide segment obtained by truncating 1, 2, 3, 4, 5, or 6 or 10 amino acids of the N-terminal or C-terminal from the epitope peptides 829-844 of tetanus toxin or by adding those acids to the epitope peptides. For example, P2 may be a peptide segment obtained by truncating 1, 2, 3, 4, 5, or 6 or 10 amino acids of the N-terminal or C-terminal from the epitope peptides 830 to 845 of tetanus toxin or by adding those acids to the epitope peptides. For example, P2 may be a peptide segment obtained by truncating 1, 2, 3, 4, 5, or 6 or 10 amino acids of the N-terminal or C-terminal from the epitope peptides 830 to 844 of tetanus toxin or by adding those acids to the epitope peptides. In the present application, the term "variant" generally refers to a sequence that differs from the reference sequence by containing one or more differences (mutations). This difference may be the substitution, deletion, or insertion of one or more amino acids. For example, the epitope peptide of said tetanus toxin may comprise an amino acid sequence as set forth in any one of SEQ ID NOs. 64-66.

In the present application, said P2 or functionally active fragment thereof may be directly or indirectly linked to said RBD or its functional fragment thereof and/or said NTD or functionally active fragment thereof.

For example, the N-terminal of said RBD or functionally active fragment thereof may be directly or indirectly linked to the C- terminal of said NTD or functionally active fragment thereof, and the N-terminal of said P2 or functionally active fragment thereof may be directly or indirectly linked to the C-terminal of said RBD or functionally active fragment thereof.

In the present application, said P2 or functionally active fragment thereof is fused in the ORF to the RBD or functionally active fragment thereof and said NTD or functionally active fragment thereof.

In the present application, said foldon domain or functionally active fragment thereof may comprise amino acid residues at the C-terminal domain of bacteriophage T4 fibrin. In some embodiments, said amino acid residue at the C-terminal domain of bacteriophage T4 fibrin comprises the amino acid sequence as set forth in SEQ ID NO. 78.

In the present application, said foldon domain or functionally active fragment thereof may comprise 27 amino acid residues or mutations at the C-terminal domain of bacteriophage T4 fibrin. In the present application, the foldon domain may be the truncated or elongated forms of the sequence of bacteriophage T4 fibrin obtained by truncating 27 residues of the C-terminal of the protein or adding 1, 2, 3, 4, 5, or 6 or 10 amino acids to the N-terminal or C-terminal of the protein. In the present application, the term "variant" generally refers to a sequence that differs from the reference sequence by containing one or more differences (mutations). This difference may be the substitution, deletion, or insertion of one or more amino acids.

For example, said foldon domain or functionally active fragment thereof may comprise an amino acid sequence as set forth in any one of SEQ ID NOs. 67-69 and 78.

In the present application, said foldon domain or functionally active fragment thereof is directly or indirectly linked to said RBD or functionally active fragment thereof and/or said NTD or functionally active fragment thereof.

For example, the N-terminal of said foldon domain or functionally active fragment thereof may be directly or indirectly linked to the C-terminal of said RBD or functionally active fragment thereof, and the N-terminal of said RBD or functionally active N-terminal thereof is directly or indirectly linked to the C-terminal of said NTD or functionally active fragment thereof.

In the present application, said foldon domain or functionally active fragment thereof may be fused in the ORF to said RBD or functionally active fragment thereof as well as said NTD or functionally active fragment thereof.

In the present application, said N-terminal of said foldon domain or functionally active fragment thereof is directly or indirectly linked to the C-terminal of said P2 or functionally active fragment thereof, and the N-terminal of said P2 or functionally active fragment thereof is directly or indirectly linked to the C-terminal of said RBD or functionally active fragment thereof, while the N-terminal of said RBD or functionally active fragment thereof is directly or indirectly linked to the C-terminal of said NTD or functionally active fragment thereof.

In the present application, said P2 or functionally active fragment thereof, the foldon domain or functionally active fragment thereof and RBD or functionally active fragment thereof are fused in the ORF with said NTD or functionally active fragment thereof.

In the present application, said ferritin or functionally active fragment thereof comprises *Trichoplusia trichoplusia ni* ferritin or functionally active fragment thereof.

In the present application, said *trichoplusia ni* ferritin or functionally active fragment thereof comprises a heavy chain or a light chain of *trichoplusia ni* ferritin.

In the present application, the ferritin may be a variant of the ferritin from *trichoplusia ni* or *Helicobacter pylori.* In the present application, the term "variant" generally refers to a sequence that differs from the reference sequence by containing one or more differences (mutations). This difference may be the substitution, deletion, or insertion of one or more amino acids. In the present application, the "light chain of ferritin from *trichoplusia ni"* and "LC" can be interchangeably used with "ferritin LC". In the present application, the "heavy chain of ferritin from *trichoplusia ni"* and "HC" can be interchangeably used with "ferritin HC". In the present application, "*Helicobacter pylori* ferritin" can be used interchangeably with "HP ferritin".

In the present application, said heavy chain of the *trichoplusia ni* ferritin may comprise the amino acid sequence as set forth in SEQ ID NO. 70.

In the present application, said light chain of the *trichoplusia ni* ferritin may comprise the amino acid sequence as set forth in SEQ ID NO. 71.

In the present application, said ferritin or functionally active fragment thereof may comprise the *Helicobacter pylori* ferritin or functionally active fragment thereof.

In the present application, the ferritin or functionally active fragment thereof comprises an amino acid sequence as set forth in any one of SEQ ID NOs. 70-72.

In the present application, said ferritin or functionally active fragment thereof may be directly or indirectly linked to the RBD or functionally active fragment thereof and/or the said NTD or functionally active fragment thereof.

For example, the N-terminal of said ferritin or functionally active fragment thereof may be directly or indirectly linked to the C-terminal of the RBD or functionally active fragment thereof, and the N-terminal of said RBD or functionally active N-terminal thereof is directly or indirectly linked to the C-terminal of said NTD or functionally active fragment thereof.

For example, the N-terminal of said *Helicobacter pylori* ferritin or functionally active fragment thereof may be directly or indirectly linked to the C-terminal of the RBD or functionally active fragment thereof, and the N-terminal of said RBD or functionally active N-terminal thereof is directly or indirectly linked to the C-terminal of said NTD or functionally active fragment thereof.

In the present application, said *Helicobacter pylori* ferritin or functionally active fragment thereof is fused in the ORF to the RBD or functionally active fragment thereof and said NTD or functionally active fragment thereof.

In the present application, the term "hepatitis B surface antigen (HBsAg)" generally refers to a capsid protein located in the outermost part of the envelope of the hepatitis B virus. For example, the amino acid sequence of the hepatitis B surface antigen may be a sequence corresponding to the protein sequence accession number AAA45524, ANJ76941, CAA24234 or AAC34729 in the NCBI, or the sequence obtained by truncating or adding 1, 2, 3, 4, 5, 6 or 10 amino acids at the N-terminal or C-terminal, or a mutation in the protein, such as the deletion, substitution, or insertion of one or more amino acids as appropriate.

In the present application, said hepatitis B surface antigen or functionally active fragment thereof may comprise the amino acid sequence as set forth in SEQ ID NO. 73.

In the present application, said hepatitis B surface antigen or functionally active fragment thereof may be directly or indirectly linked to said RBD or functionally active fragment thereof and/or the said NTD or functionally active fragment thereof.

In the present application, the N-terminal of said hepatitis B surface antigen or functionally active fragment thereof may be directly or indirectly linked to the C-terminal of said RBD or functionally active fragment thereof, and the N-terminal of said RBD or functionally active fragment thereof is directly or indirectly linked to the C-terminal of said NTD or functionally active fragment thereof. In the present application, said hepatitis B surface antigen or functionally active fragment thereof may be fused in the ORF to said RBD or functionally active fragment thereof and said NTD or functionally active fragment thereof.

In the present application, the "direct or indirect linking" generally refers to two different linking modes, i.e., the direct linking and indirect linking, wherein the direct linking refers to the linking between two sequences without any other artificially added sequences, such as a flexible linker, a rigid linker or a splicing linker, etc. Indirect linking means that two sequences are linked by artificially adding the linking sequences, such as a flexible linker, a rigid linker or a splicing linker. In some embodiments, said flexible linker may comprise the amino acid sequence of GSGSG (SEQ ID NO. 89). In some embodiments, said linking sequence may comprise the amino acid sequence of SFTVEKGIYQTSNF (SEQ ID NO. 90).

In the present application, said fusion protein may further comprise a signal peptide. In some embodiments, said signal peptide sequence of MGWSCIILFLVATATGVHS (SEQ ID NO. 91) may be added to the N-terminal of said fusion protein.

In the present application, the fusion protein comprises an amino acid sequence as set forth in any one of SEQ ID NOs. 39-44, 79, 85 and 96.

### Immunogenic Composition

In another aspect, the present application provides an immunogenic composition comprising said fusion protein.

In the present application, the term "immunogenic composition" generally refers to a subunit composition. In the present application, said subunit composition refers to a composition in which the components have been isolated and purified to a purity of at least 50%, 60%, 70%, 80% or 90% prior to mixing the components to form an antigenic composition. For example, the subunit composition may be an aqueous solution of a water-soluble protein. For example, the subunit composition may comprise a detergent. For example, the subunit composition may comprise a non-ionic, zwitterionic, or ionic detergent. For example, the subunit composition may comprise a lipid. In some cases, the immunogenic composition may include the RBD or functionally active fragment thereof and the NTD or functionally active fragment thereof. In some cases, the immunogenic composition may include the RBD or functionally active fragment thereof and a fusion protein comprising the NTD or functionally active fragment thereof. In some cases, the immunogenic composition may include a fusion protein comprising an RBD or functionally active fragment thereof and an NTD or functionally active fragment thereof. In some cases, the immunogenic composition may include a fusion protein comprising the RBD or functionally active fragment thereof and a fusion protein comprising the NTD or functionally active fragment thereof. In some cases, said immunogenic composition may further comprise one or several human influenza virus hemagglutinin proteins HA. In the present application, said HA may comprise an amino acid sequence as set forth in any one of SEQ ID NOs. 92-95.

In some embodiments, said influenza strains from which the influenza hemagglutinin is obtained are the pandemic strains published on the WHO website every year. For example, the pandemic strains published on the WHO website in 2021 referred to type A (H1N1) EPI1661231|HA|A/Wisconsin/58 8/2019|EPI_ISL_404460, type A(H3N2)EPI1843589|HA|A/Cambodia/e0826360/20201EPI_ISL_94 4639, type B EPI1394970|HA|B/Washington/02/2019|EPI_ISL_347829 and type B EPI529345|HA| B/PHUKET/3073/2013 |EPI_ISL_161843. In some embodiments, the composition of the vaccines us ed to prevent influenza and coronaviruses includes 50 µg of each of the above types of HA protein a nd 50 µg of the mutated NTD-RBD-foldon protein and an adjuvant commonly used in vaccine comp osition, such as AS03 adjuvant.

For example, said immunogenic composition may comprise a first component and a second component, wherein said first component may comprise a fusion protein of said RBD or functionally active fragment thereof and NTD or functionally active fragment thereof as described in the present application, while said fusion protein may further comprise one or more polypeptides selected from the following group: P2 or functionally active fragment thereof, the foldon domain or functionally active fragment thereof, the ferritin or functionally active fragment thereof, and hepatitis B surface antigen (HBsAg) or functionally active fragment thereof. Said second component may comprise the said RBD or functionally active fragment thereof, said NTD or functionally active fragment thereof, or one or more arbitrarily selected from human influenza virus hemagglutinin protein HA.

In the present application, said RBD or functionally active fragment thereof may be derived from the RBD of the wild-type S protein of the SARS-CoV-2 or from the RBD of the variant S protein of SARS-CoV-2. In some embodiments, said RBD or functionally active fragment thereof may comprise an amino acid mutation at one or more of the amino acid sites of K417, L452, T478, E484 and N501. In the present application, said RBD or functionally active fragment thereof may comprise the RBD of the S protein of the Gamma variant. In some embodiments, said RBD or functionally active fragment thereof may comprise one or more amino acid mutations of K417T, E484K and N501Y. For example, said RBD may comprise the amino acid sequence as set forth in SEQ ID NO. 83. In the present application, said RBD or functionally active fragment thereof may comprise the RBD of the S protein of the Beta variant. In some embodiments, said RBD or functionally active fragment thereof may comprise one or more amino acid mutations of K417N, E484K and N501Y. In some embodiments, said RBD may comprise the amino acid sequence as set forth in SEQ ID NO. 76.

In the present application, said NTD or functionally active fragment thereof may be derived from the NTD of the wild-type S protein of the SARS-CoV-2 or from the NTD of the variant S protein of SARS-CoV-2. In some embodiments, said NTD or functionally active fragment thereof may comprise amino acid mutation at one or more of the amino acid sites of L18, T19, T20, P26, D80, D138, R190, D215, L242-244 and R246. In some embodiments, said NTD or functionally active fragment thereof may comprise one or more amino acid mutations of L18F, T19R, T20N, P26S, D80A, D138Y, R190S, D215G, L242-244del and R246I.

In the present application, said NTD or functionally active fragment thereof may comprise the NTD of the S protein of the Gamma variant. In some embodiments, NTD may comprise the mutations at the amino acids of L18F, T20N, P26S, D138Y, and R190S. For example, said NTD may comprise the amino acid sequence as set forth in SEQ ID NO.84.

In the present application, said NTD or functionally active fragment thereof may comprise the NTD of the S protein of a Beta variant. In some embodiments, said NTD may comprise mutations at L18F, D80A, D215G, L242-244del, and R246I. For example, said NTD may comprise the amino acid sequence as set forth in SEQ ID NO.77.

In the present application, said immunogenic composition may further include an adjuvant. For example, said adjuvant may comprise an aluminum salt, such as aluminium hydroxide gel (alum) or aluminium phosphate, but it may also contain a calcium salt, iron salt, or zinc salt, or it may be an insoluble suspension of an acylated tyrosine or an acylated sugar, a cationically or anionically derivatized polysaccharide, or a polyphosphazene. For example, said immunogenic composition may also be a selected preferential inducer of the Th1-type response. For example, said preferential inducer of Th1-type response may include monophosphoryl lipid A or a derivative thereof. For example, said adjuvant may be a composition of monophosphoryl lipid A (e.g., 3-de-O-acylated monophosphoryl lipid A (3D-MPL)) and an aluminium salt. An adjuvant enhancing system may comprise a composition of monophosphoryl lipid A and a saponin derivative, particularly the combination of QS21 and 3D-MPL as disclosed in WO 94/00153, or a composite in which QS21 is quenched with cholesterol to reduce the reactogenicity as disclosed in WO 96/33739. For example, said adjuvant may also be the adjuvant disclosed in WO 95/17210, which contains QS21, 3D-MPL and tocopherol in an oil-in-water emulsion. For example, the said adjuvant may be a homogeneous droplet-like emulsion formed by mixing Tween 80, sorbitan trioleate and squalene and then microfluidizing under the high pressure condition. For example, said adjuvant may comprise the unmethylated oligonucleotide CpG (WO96/02555).

In the present application, said immunogenic composition comprises a first component comprising the fusion protein, and a second component comprising the fusion protein.

In the present application, said immunogenic composition is formulated in the following weight ratios: 1) the first component (1-15 portions in weight ratio); and/or 2) the second component (1-15 portions in weight ratio).

In the present application, said immunogenic composition comprises: 1) 5-60 µg of the first component and/or 2) 5-60 µg of the second component.

In the present application, the term "weight ratio" generally refers to the ratio of the weight of the first component to the second component of the immunogenic composition. In the present application, the immunogenic composition may contain the following components per dose: 5-60 µg of the first component and 5-60 µg of the second component. For example, the immunogenic composition may comprise 5 µg, 10 µg, 20 µg, 30 µg or 40 µg of the first component. For example, the immunogenic composition may comprise 5 µg, 10 µg, 20 µg, 30 µg or 40 µg of the second component.

In another aspect, the present application provides an immunogenic composition comprising a first component comprising the receptor-binding domain (RBD) of the SARS-CoV-2 spike protein (S protein) or functionally active fragment thereof, and a second component comprising an N-terminal domain (NTD) of SARS-CoV-2 spike protein (S protein) or functionally active fragment thereof.

In the present application, said immunogenic composition is formulated in the following weight ratios: 1) the first component (1-15 portions in weight ratio); and/or 2) the second component (1-15 portions in weight ratio).

In the present application, said immunogenic composition comprises: 1) 5-60 µg of the first component; and/or 2) 5-60 µg of the second component.

In the present application, said RBD may comprise an amino acid sequence as set forth in any one of SEQ ID NOs. 18-19.

In the present application, said NTD comprises an amino acid sequence as set forth in any one of SEQ ID NOs. 37-38.

In the present application, the term "weight ratio" generally refers to the ratio of the weight of the first component to the second component of the immunogenic composition. In the present application, the immunogenic composition may contain the following components per dose: 5-60 µg of the first component and 5-60 µg of the second component. For example, the immunogenic composition may comprise 5 µg, 10 µg, 20 µg, 30 µg or 40 µg of the first component. For example, the immunogenic composition may comprise 5 µg, 10 µg, 20 µg, 30 µg or 40 µg of the second component.

In some cases, the immunogenic composition may comprise 20-100 µg of the first component NTD-RBD-foldon protein, and 20-100 µg of the second component NTD-foldon protein, or 20-100 µg of RBD-foldon protein, or 15-80 µg of influenza virus hemagglutinin protein.

In some embodiments, said immunogenic composition comprises 20-100 µg of NTD-RBD-foldon (Gamma variant) protein, 20-100 µg of NTD-foldon (Gamma variant) protein, and a lyophilized protectant; wherein said lyophilized protectant is prepared with the formula of 1% (w/v) sucrose, 2% (w/v) glycine, 0.02% (w/v) Tween 80 and 10 mM PB buffer (Na₂HPO₄, NaH₂PO₄) at pH 7.5.

In some embodiments, said immunogenic composition comprises 20-100 µg of NTD-RBD-foldon (Gamma variant) protein, 20-100 µg of NTD-foldon (Gamma variant) protein, and a lyophilized protectant; wherein the said lyophilized protectant is prepared with the formula of 1% (w/v) sucrose, 2% (w/v) glycine, 0.02% (w/v) Tween 80 and 10 mM PB buffer (Na₂HPO₄, NaH₂PO₄) at pH 7.5.

In some embodiments, said immunogenic composition comprises 20-100 µg of NTD-RBD-foldon (Gamma variant) protein, 15-180 µg of influenza virus hemagglutinin protein and a lyophilized protectant; wherein said lyophilized protectant is prepared with the formula of 1% (w/v) sucrose, 2% (w/v) glycine, 0.02% (w/v) Tween 80 and 10 mM PB buffer (Na₂HPO₄, NaH₂PO₄) at pH 7.5.

In another aspect, the present application provides a vaccine comprising the said fusion protein or said immunogenic composition, and arbitrarily selected pharmaceutically acceptable adjuvants.

In some embodiments, the said vaccine comprises 0.5 mL of the fusion protein or immunogenic composition, and 0.5 mL of arbitrarily selected pharmaceutically acceptable adjuvants.

The adjuvants of the present invention comprise one or more components selected from the group consisting of squalene, MF59, AS03, monophosphatidyl lipid A, flagellin, CpG-ODN, muramyl dipeptide, and aluminium salts or calcium salts. These adjuvants are well known in the art and are available through several commercial channels.

In some embodiments, said adjuvant is an aluminium hydroxide adjuvant.

In some embodiments, said adjuvant is an MF59 adjuvant.

In some embodiments, said adjuvant is preferably an AS03 adjuvant.

Said AS03 adjuvant comprises 10.69 mg of squalene, 11.86 mg of alpha-tocopherol, 4.86 mg of polysorbate 80, 3.53 mg of sodium chloride, 0.09 mg of potassium chloride, 0.51 mg of disodium hydrogen phosphate and 0.09 mg of potassium dihydrogen phosphate.

Said MF59 adjuvant comprises 4.5% squalene, 0.5% Tween 80 and 0.5% Span85.

In some embodiments, said vaccine comprises 20-100 µg of NTD-RBD-foldon protein, 1% (w/v) sucrose, 2% (w/v) glycine, 0.02% (w/v) Tween 80, and 10 mM PB buffer (Na₂HPO₄, NaH₂PO₄) at pH 7.5, and the AS03 adjuvant.

In some embodiments, said vaccine comprises 20-100 µg of NTD-RBD-foldon protein, 15- 45 µg of HA of H1N1 strain, 15-45 µg of HA of H3N2 strain, and 15-45 µg of HA of B/Washington/02/2019 strain, 1% (w/v) sucrose, 2% (w/v) glycine, 0.02% (w/v) Tween 80, and 10 mM PB buffer (Na₂HPO₄, NaH₂PO₄) at pH 7.5, and the AS03 adjuvant.

In some embodiments, said vaccine comprises 20-100 µg of NTD-RBD-foldon protein, 15-45 µg of HA of H1N1 strain, 15-45 µg ofHAofH3N2 strain, 15-45 µg of HA of B/Washington/02/2019 strain, and 15-45 µg of HA of B/PHUKET/3073/2013 strain, 1% (w/v) sucrose, 2% (w/v) glycine, 0.02% (w/v) Tween 80, and 10 mM PB buffer (Na₂HPO₄, NaH₂PO₄) at pH 7.5, and the AS03 adjuvant.

In some embodiments, said vaccine comprises 20-100 µg of NTD-RBD-foldon protein, 15-45 µg of HA of H1N1 strain, 15-45 µg ofHAofH3N2 strain, 15-45 µg of HA of B/Washington/02/2019 strain, 1% (w/v) sucrose, 2% (w/v) glycine, 0.02% (w/v) Tween 80, and 10 mM PB buffer (Na₂HPO₄, NaH₂PO₄) at pH 7.5, and adjuvant MF59.

In some embodiments, said vaccine comprises 20-100 µg of NTD-RBD-foldon protein, 15-45 µg of HA of H1N1 strain, 15-45 µg ofHAofH3N2 strain, 15-45 µg of HA of B/Washington/02/2019 strain, and 15-45 µg of HA of B/PHUKET/3073/2013 strain, 1% (w/v) sucrose, 2% (w/v) glycine, 0.02% (w/v) Tween 80, and 10 mM PB buffer (Na₂HPO₄, NaH₂PO₄) at pH 7.5, and the MF59 adjuvant.

In some preferable embodiments, said vaccine comprises 50 µg of NTD-RBD-foldon protein, 45 µg of HA of H1N1 strain, 45 µg of HA of H3N2 strain, 45 µg of HA of B/Washington/02/2019 strain, and 45 µg of HA of B/PHUKET/3073/2013 strain, 1% (w/v) sucrose, 2% (w/v) glycine, 0.02% (w/v) Tween 80, and 10 mM PB buffer (Na₂ HPO₄, NaH₂PO₄) at pH 7.5, and the AS03 adjuvant.

In some preferable embodiments, said vaccine comprises 50 µg of NTD-RBD-foldon protein, 45 µg of HA of H1N1 strain, 45 µg of HA of H3N2 strain, 45 µg of HA of B/Washington/02/2019 strain, and 45 µg of HA of B/PHUKET/3073/2013 strain, 1% (w/v) sucrose, 2% (w/v) glycine, 0.02% (w/v) Tween 80, and 10 mM PB buffer (Na₂HPO₄, NaH₂PO₄) at pH 7.5, and adjuvant MF59.

In the present application, the said RBD-NTD-foldon (Beta variant) may comprise the amino acid sequence shown in SEQ ID NO.79. In the present application, the said RBD-NTD-foldon (Gamma variant) may comprise the amino acid sequence shown in SEQ ID N.85. In the present application, the said RBD-NTD-foldon (WIV04-1) may comprise the amino acid sequence as set forth in SEQ ID NO. 96.

In the present application, said immunogenic composition may further comprise one or several kinds of hemagglutinin protein HA from human influenza viruses. In the present application, said HA may comprise the amino acid sequence as set forth in any one of SEQ ID NOs. 92-95.

In the present application, said RBD may also comprise an amino acid sequence as set forth in any one of SEQ ID NOs. 97-108.

In the present application, said RBD may also comprise an amino acid sequence as set forth in any one of SEQ ID NOs. 109-111.

### Nucleic Acid Molecules, Pharmaceutical Compositions and Applications

In another aspect, the present application also provides one or more isolated nucleic acid molecules that encode a fusion protein as described above. In some embodiments, said nucleic acid molecule may comprise mRNA.

In another aspect, the present application provides a pharmaceutical composition comprising said fusion protein, or said immunogenic composition, along with optionally a pharmaceutically acceptable excipient.

Said pharmaceutically acceptable vector may include buffers, antioxidants, preservatives, low-molecular-weight polypeptides, proteins, hydrophilic polymers, amino acids, sugars, chelating agents, counter-ions, metal complexes, and/or non-ionic surfactants, etc.

In the present application, said pharmaceutical compositions may be formulated for oral administration, intravenous administration, intramuscular administration, intra-lesion administration in situ, inhalation, rectal administration, vaginal administration, transdermal administration, or administration via a subcutaneous depot.

Said pharmaceutical compositions can be used to inhibit or postpone the development or progression of diseases or conditions, and/or can alleviate and/or stabilize the diseases or conditions.

Said pharmaceutical composition may comprise a prophylactically and/or therapeutically effective amount of said fusion protein or said immunogenic compositions.

Said prophylactically and/or therapeutically effective amount is defined as a dose required to prevent and/or treat (at least partially treat) a disease or a condition and/or any complication thereof in a subject having or at risk of developing such disease risks.

In the present application, the term "effective amount" generally refers to an amount of drug that can alleviate or eliminate a disease or symptom in a subject or that can prophylactically inhibit or prevent the occurrence of a disease or symptom. Typically, the specific effective amount can be determined based on the subject's body weight, age, gender, diet, excretion rate, past medical history, current treatments, dosing schedule, dosage form, method of administration, route of administration, combinational use of drugs, the health status of the said subject and potential of causing cross-infections, allergic reaction, hypersensitivity and side effects, and/or extent of progression of epithelial (or endothelial) tissue disease, etc. A person skilled in the art (e.g., a physician or veterinarian) may reduce or increase the dose proportionally according to these or other conditions or requirements.

In the present application, the said subjects may include humans or non-human animals. For example, the non-human animals may be selected from the group consisting of monkeys, chickens, geese, cats, dogs, mice and rats. In addition, non-human animals may also include any animal species other than humans, such as domestic animals, rodents, primates, household animals, or poultry animals. The said humans may be Caucasian, African, Asian, Semitic, or other races, or the hybridization of various races. For another example, the said humans may be an elderly, an adult, an adolescent, a child, or an infant.

The effective amount in humans can be extrapolated from the effective amount determined in experimental animals. For example, Freireich et al. described the interrelationship of the doses used in animals and those used in humans (based on mg/m² of body surface) (Freireich et al., Cancer Chemother Rep, 1966; 50, 219). Body surface area can be approximately determined based on the patient's body height and body weight. For example, see Scientific Tables, Geigy Pharmaceuticals, Ardsley, N.Y, 537 (1970).

In another aspect, the present application provides the use of said fusion protein or said immunogenic composition in the preparation of a vaccine.

In the present application, the vaccines may be used for the prevention and/or treatment of COVID-19 disease.

In another aspect, the present application provides the use of said fusion protein or the said immunogenic composition for the treatment and/or prevention of COVID-19.

In another aspect, the present application provides a method for making a COVID-19 subunit vaccine which comprises:
1) preparing said fusion protein or the said immunogenic composition; and
2) mixing said fusion protein or immunogenic composition described in 1) with a pharmaceutically acceptable adjuvant.

In another aspect, the present application provides a method for detecting SARS-CoV-2 neutralizing antibodies which comprises:
1) administering the COVID-19 subunit vaccine to the subjects; and
2) detecting the neutralizing antibodies produced in the said subjects described in 1) after receiving the COVID-19 subunit vaccine.

In another aspect, the present application provides a method of treating and/or preventing COVID-19 which comprises administering said fusion protein or said immunogenic composition or said COVID-19 subunit vaccine to the subjects.

The present application also relates to the following specific embodiments:
1. A fusion protein comprising: 1) the receptor-binding domain (RBD) of the SARS-CoV-2 spike protein (S protein) or functionally active fragment thereof; and 2) one or more polypeptides selected from the groups consisting of P2 or functionally active fragment thereof, foldon domain or functionally active fragment thereof, ferritin or functionally active fragment thereof, and hepatitis B surface antigen (HBsAg) or functionally active fragment thereof.
2. The fusion protein described in embodiment 1, wherein said RBD is derived from a wild-type SARS-CoV-2 strain or a variant thereof.
3. The fusion protein described in embodiment 2, wherein said SAR-CoV-2 variant is selected from any one of the groups consisting of a Gamma variant, a Beta variant, a Delta variant, and an Alpha variant.
4. The fusion protein in any one of embodiments 1-3, wherein said RBD has a mutation in one or more amino acid sites selected from the groups consisting of: K417, L452, T478, E484 and N501 as compared to the RBD of wild-type SARS-CoV-2.
5. The fusion protein of any one of embodiments 1-4, wherein said RBD comprises one or more of the amino acid mutations of K417T/N, L452R, T478K, E484K and N501Y
6. The fusion protein of any one of embodiments 1-5, wherein said RBD comprises the K417T, E484K and N501Y amino acid mutations.
7. The fusion protein of any one of embodiments 1-6, wherein said RBD comprises the K417T, E484K and N501Y amino acid mutations.
8. The fusion protein of any one of embodiments 1-7, wherein said RBD comprises the amino acid sequence as set forth in any one of the SEQ ID NOs.18, 19, 76, 83 and 97-108.
9. The fusion protein of any one of embodiments 1-8, wherein said P2 or functionally active fragment thereof comprises an epitope peptide of tetanus toxin.
10. The fusion protein of embodiment 9, wherein said epitope peptide of tetanus toxin comprises an amino acid sequence as set forth in any one of the SEQ ID NOs. 64-66.
11. The fusion protein of any one of embodiments 1-10, wherein said P2 or functionally active fragment thereof is directly or indirectly linked to the N-terminal or C-terminal of said RBD or functionally active fragment thereof.
12. The fusion protein in any one of embodiments 1-11, wherein said RBD or functionally active fragment thereof is fused in the ORF to said P2 or functionally active fragment thereof.
13. The fusion protein in any one of embodiments 1-12, wherein said foldon domain or functionally active fragment thereof comprises amino acid residues from the C-terminal domain of bacteriophage T4 fibrin.
14. The fusion protein of any one of embodiments 1-13, wherein said foldon domain or functionally active fragment thereof comprises an amino acid sequence as set forth in any one of SEQ ID NOs. 67-69 and 78.
15. The fusion protein of any one of embodiments 1-14, wherein said foldon domain or functionally active fragment thereof is directly or indirectly linked to the N-terminal or C-terminal of said RBD or functionally active fragment thereof.
16. The fusion protein of any one of embodiments 1-15, wherein said RBD or functionally active fragment thereof is fused in the ORF to said foldon domain or functionally active fragment thereof.
17. The fusion protein of any one of embodiments 1-16, wherein said ferritin or functionally active fragment thereof comprises *trichoplusia ni* ferritin or functionally active fragment thereof.
18. The fusion protein of embodiments 17, wherein said *trichoplusia ni* ferritin or functionally active fragment thereof comprises a heavy chain or a light chain of *trichoplusia ni* ferritin.
19. The fusion protein of embodiments 18, wherein said heavy chain of the *trichoplusia ni* ferritin comprises the amino acid sequence as set forth in SEQ ID NO. 70.
20. The fusion protein of any one of embodiments 18-19, wherein said light chain of the *trichoplusia ni* ferritin comprises the amino acid sequence as set forth in SEQ ID NO. 71.
21. The fusion protein of any one of embodiments 1-20, wherein said ferritin or functionally active fragment thereof comprises *Helicobacter pylori* ferritin or functionally active fragment thereof.
22. The fusion protein of any one of embodiments 1-21, wherein said ferritin or functionally active fragment thereof comprises an amino acid sequence as set forth in any one of SEQ ID NOs. 70-72.
23. The fusion protein of any one of embodiments 1-22, wherein said ferritin or functionally active fragment thereof is directly or indirectly linked to the N-terminal or C-terminal of said RBD or functionally active fragment thereof.
24. The fusion protein of any one of embodiments 1-23, wherein the said RBD or functionally active fragment thereof is fused in the ORF to the ferritin or functionally active fragment thereof.
25. The fusion protein of any one of embodiments 1-24, wherein said hepatitis B surface antigen or functionally active fragment thereof comprises an amino acid sequence as set forth in any one of SEQ ID NO. 73.
26. The fusion protein of any one of embodiments 1-25, wherein said hepatitis B surface antigen or functionally active fragment thereof is directly or indirectly linked to the N-terminal or C-terminal of said RBD or functionally active fragment thereof.
27. The fusion protein of any one of embodiments 1-26, wherein said RBD or functionally active fragment thereof is fused in the ORF to said hepatitis B surface antigen or functionally active fragment thereof.
28. The fusion protein of any one of embodiments 11-27, wherein said direct or indirect linking may comprise the linking via a linker.
29. The fusion protein of embodiment 28, wherein said linker may comprise a rigid linker, a flexible linker or other sequences.
30. The fusion protein of any one of embodiments 28-29, wherein said linker may comprise an amino acid sequence shown in any one of SEQ ID NOs. 89-90.
31. The fusion protein of any one of embodiments 1-30 comprises an amino acid sequence as set forth in any one of SEQ ID NOs. 1-17, 80 and 86.
32. A fusion protein comprising: 1) the N-terminal domain (NTD) of the SARS-CoV-2 spike protein (S protein) or functionally active fragment thereof; and 2) one or more polypeptides selected from the group consisting of P2 or functionally active fragment thereof, the foldon domain or functionally active fragment thereof, ferritin or functionally active fragment thereof, and hepatitis B surface antigen (HBsAg) or functionally active fragment thereof.
33. The fusion protein of embodiment 32, wherein said NTD is derived from said NTD of SARS-CoV-2 wild-type strain or a variant S protein thereof.
34. The fusion protein of embodiment 33, wherein said SAR-CoV-2 variant is selected from the group consisting of a Gamma variant, a Beta variant, a Delta variant, and an Alpha variant.
35. The fusion protein of any one of examples 32-34, wherein said NTD comprises a mutation at one or more amino acid sites selected from the groups consisting of L18, T19, T20, P26, D80, D138, R190, D215, L242-244, and R246.
36. The fusion protein of any one of embodiments 32-35, wherein said NTD comprises the mutations of one or more amino acids selected from the groups consisting of L18F, T19R, T20N, P26S, D80A, D138Y, R190S, D215G, L242-244del, and R246I.
37 The fusion protein of any one of embodiments 32-36, wherein said NTD comprises L18F, D80A, D215G, L242-244del and R246I amino acid mutations.
38 The fusion protein of any one of embodiments 32-37, wherein said NTD comprises L18F, D80A, D215G, L242-244del and R190S amino acid mutations.
39. The fusion protein of any one of embodiments 32-38, wherein said NTD comprises L18F, T20N, P26S, D138Y and R246I amino acid mutations.
40. The fusion protein of any one of embodiments 32-39, wherein said NTD comprises an amino acid sequence as set forth in any one of SEQ ID NOs. 37, 38, 77, 84 and 109-111.
41. The fusion protein of any one of embodiments 32-40, wherein said P2 or functionally active fragment thereof comprises an epitope peptide of tetanus toxin.
42. The fusion protein of embodiment 41, wherein said epitope peptide of tetanus toxin comprises an amino acid sequence as set forth in any one of the SEQ ID NOs. 64-66.
43. The fusion protein of any one of embodiments 32-42, wherein said P2 or functionally active fragment thereof is directly or indirectly linked to the N-terminal or C-terminal of said NTD or functionally active fragment thereof.
44. The fusion protein of any one of embodiments 32-43, wherein said NTD or functionally active fragment thereof is fused in the ORF to the P2 or functionally active fragment thereof.
45. The fusion protein of any one of embodiments 32-44, wherein said foldon domain or functionally active fragment thereof comprises amino acid residues at the C-terminal domain of bacteriophage T4 fibrin.
46. The fusion protein of any one of embodiments 32-45, wherein said foldon domain or functionally active fragment thereof comprises an amino acid sequence as set forth in any one of SEQ ID NOs. 67-69 and 78.
47. The fusion protein of any one of embodiments 32-46, wherein said foldon domain or functionally active fragment thereof is directly or indirectly linked to the N-terminal or C-terminal of said NTD or functionally active fragment thereof.
48. The fusion protein of any one of embodiments 32-47, wherein said NTD or functionally active fragment thereof is fused in the ORF to the foldon domain or functionally active fragment thereof.
49. The fusion protein of any one of embodiments 32-48, wherein the ferritin or functionally active fragment thereof comprises *trichoplusia ni* ferritin or functionally active fragment thereof.
50. The fusion protein of embodiment 49, wherein said *trichoplusia ni* ferritin or functionally active fragment thereof comprises a heavy chain or a light chain of *trichoplusia ni* ferritin.
51. The fusion protein of embodiment 50, wherein said heavy chain of the *trichoplusia ni* ferritin comprises the amino acid sequence as set forth in SEQ ID NO. 70.
52. The fusion protein of any one of embodiments 50-51, wherein said light chain of the *trichoplusia ni* ferritin comprises the amino acid sequence as set forth in SEQ ID NO. 71.
53. The fusion protein of any one of embodiments 32-52, wherein said ferritin or functionally active fragment thereof comprises *Helicobacter pylori* ferritin or functionally active fragment thereof.
54. The fusion protein of any one of embodiments 32-53, wherein said ferritin or functionally active fragment thereof comprises an amino acid sequence as set forth in any one of SEQ ID NOs. 70-72.
55. The fusion protein of any one of embodiments 32-54, wherein said ferritin or functionally active fragment thereof is directly or indirectly linked to the N-terminal or C-terminal of said NTD or functionally active fragment thereof.
56. The fusion protein of any one of embodiments 32-55, wherein said NTD or functionally active fragment thereof is fused in the ORF to the ferritin or functionally active fragment thereof.
57. The fusion protein of any one of embodiments 32-56, wherein said hepatitis B surface antigen or functionally active fragment thereof comprises an amino acid sequence as set forth in SEQ ID NO.73.
58. The fusion protein of any one of embodiments 32-57, wherein said hepatitis B surface antigen or functionally active fragment thereof is directly or indirectly linked to the N-terminal or C-terminal of said NTD or functionally active fragment thereof.
59. The fusion protein of any one of embodiments 32-58, wherein said NTD or functionally active fragment thereof is fused in the ORF to the hepatitis B surface antigen or functionally active fragment thereof.
60. The fusion protein of any one of embodiments 43-59, wherein said direct or indirect linking may comprise the linking via a linker.
61. The fusion protein of embodiment 60, wherein said linker may comprise a rigid linker, a flexible linker or other sequence.
62. The fusion protein of any one of embodiments 60-61, wherein said linker may comprise an amino acid sequence as set forth in any one of SEQ ID NOs. 89-90.
63. The fusion protein of any one of embodiments 32-62 comprises an amino acid sequence as set forth in any one of SEQ ID NOs. 20-36, 81 and 87.
64. A fusion protein comprising: 1) the receptor-binding domain (RBD) of the SARS-CoV-2 spike protein (S protein) or functionally active fragment thereof and 2) the N-terminal domain (NTD) of the SARS-CoV-2 spike protein (S protein) or functionally active fragment thereof.
65. The fusion protein of embodiment 64, wherein said RBD is derived from a wild-type SARS-CoV-2 strain or a variant thereof.
66. The fusion protein of embodiment 65, wherein said SAR-CoV-2 variant is selected from the group consisting of a Gamma variant, a Beta variant, a Delta variant, and an Alpha variant.
67. The fusion protein of any one of embodiments 64-66, wherein said RBD comprises a mutation in one or more amino acid sites selected from the group consisting of K417, L452, T478, E484 and N501.
68. The fusion protein of any one of embodiments 64-67, wherein said RBD comprises one or more of the amino acid mutations of K417T/N, L452R, T478K, E484K and N501Y
69. The fusion protein of any one of embodiments 64-68, wherein said RBD comprises the K417T, E484K and N501Y amino acid mutations.
70. The fusion protein of any one of embodiments 64-69, wherein said RBD comprises the K417T, E484K and N501Y amino acid mutations.
71. The fusion protein of any one of embodiments 64-70, wherein said RBD comprises the amino acid sequence as set forth in any one of the SEQ ID NOs. 18, 19, 76, 83 and 97-108.
72. The fusion protein of any of embodiments 64-71, wherein said NTD is derived from a wild-type SARS-CoV-2 strain or a variant thereof.
73. The fusion protein of embodiments 72, wherein said SAR-CoV-2 variant is selected from the group consisting of a Gamma variant, a Beta variant, a Delta variant, and an Alpha variant.
74. The fusion protein of any one of embodiments 64-73, wherein said NTD comprises a mutation at one or more amino acid sites selected from the group consisting of L18, T19, T20, P26, D80, D138, R190, D215, L242-244, and R246.
75. The fusion protein of any one of embodiments 64-74, wherein said NTD comprises mutations of one or more amino acids selected from the group consisting of L18F, T19R, T20N, P26S, D80A, D138Y, R190S, D215G, L242-244del, and R246I.
76. The fusion protein of any one of embodiments 64-75, wherein said NTD comprises L18F, D80A, D215G, L242-244del and R246I amino acid mutations.
77. The fusion protein of any one of embodiments 64-76, wherein the said NTD comprises L18F, D80A, D215G, L242-244del and R190S amino acid mutations.
78. The fusion protein of any one of embodiments 64-77, wherein the said NTD comprises L18F, T20N, P26S, D138Y and R246I amino acid mutations.
79. The fusion protein of any one of embodiments 64-78, wherein said NTD comprises an amino acid sequence as set forth in any one of SEQ ID NOs. 37, 38, 77, 84 and 109-111.
80. The fusion protein of any one of embodiments 64-79, wherein said fusion protein further includes one or more polypeptides selected from the following groups: P2 or functionally active fragment thereof, a foldon domain or functionally active fragment thereof, ferritin or functionally active fragment thereof, and hepatitis B surface antigen (HBsAg) or functionally active fragment thereof.
81. The fusion protein of any one of embodiments 64-80, wherein said RBD or functionally active fragment thereof is directly or indirectly linked to said NTD or functionally active fragment thereof.
82. The fusion protein of any one of embodiments 64-81, wherein said RBD or functionally active fragment thereof is fused in the ORF to said NTD or functionally active fragment thereof.
83. The fusion protein of any one of embodiments 64-82, wherein said P2 or functionally active fragment thereof comprises an epitope peptide of tetanus toxin.
84. The fusion protein of embodiment 83, wherein said epitope peptide of tetanus toxin comprises an amino acid sequence as set forth in any one of the SEQ ID NOs. 64-66.
85. The fusion protein of embodiments 64-84, wherein said P2 or functionally active fragment thereof is directly or indirectly linked to the RBD or its functional fragment thereof and/or the said NTD or functionally active fragment thereof.
86. The fusion protein of embodiments 64-85, wherein said P2 or functionally active fragment thereof is fused in the ORF to said RBD or functionally active fragment thereof and/or said NTD or functionally active fragment thereof.
87. The fusion protein of any one of embodiments 64-86, wherein the N-terminal of said P2 or functionally active fragment thereof is directly or indirectly linked to the C-terminal of said RBD or functionally active fragment thereof, and the N-terminal of said RBD or functionally active fragment thereof is directly or indirectly linked to the C-terminal of said NTD or functionally active fragment thereof.
88. The fusion protein of any one of embodiments 64-87, wherein said foldon domain or functionally active fragment thereof comprises amino acid residues from the C-terminal domain of bacteriophage T4 fibrin.
89. The fusion protein of any one of embodiments 64-88, wherein said foldon domain or functionally active fragment thereof comprises an amino acid sequence as set forth in any one of SEQ ID NOs. 67-69 and 78.
90. The fusion protein of embodiments 64-89, wherein said foldon domain or functionally active fragment thereof is directly or indirectly linked to said RBD or its functionally active fragment thereof and/or said NTD or functionally active fragment thereof.
91. The fusion protein of embodiments 64-90, wherein said foldon domain or functionally active fragment thereof is fused in the ORF to said RBD or functionally active fragment thereof and/or said NTD or functionally active fragment thereof.
92. The fusion protein of any one of embodiments 64-91, wherein the N-terminal of the said foldon domain or functionally active fragment thereof is directly or indirectly linked to the C-terminal of said RBD or functionally active fragment thereof, and the N-terminal of said RBD or functionally active fragment thereof is directly or indirectly linked to the C-terminal of said NTD or functionally active fragment thereof.
93. The fusion protein of any one of embodiments 64-92, wherein the N-terminal of said foldon domain or functionally active fragment thereof is directly or indirectly linked to the C-terminal of said P2 or functionally active fragment thereof, the N-terminal of said P2 or functionally active fragment thereof is directly or indirectly linked to the C-terminal of said RBD or functionally active fragment thereof, and the N-terminal of RBD or functionally active fragment thereof is directly or indirectly linked to the C-terminal of said NTD or functionally active fragment thereof.
94. The fusion protein of any one of embodiments 64-93, wherein said ferritin or functionally active fragment thereof comprises *trichoplusia ni* ferritin or functionally active fragment thereof.
95. The fusion protein of embodiment 94, wherein said *trichoplusia ni* ferritin or functionally active fragment thereof comprises a heavy chain or a light chain of *trichoplusia ni* ferritin.
96. The fusion protein of embodiments 95, wherein said heavy chain of the *trichoplusia ni* ferritin comprises the amino acid sequence as set forth in SEQ ID NO. 70.
97. The fusion protein of any one of embodiments 95-96, wherein said light chain of the *trichoplusia ni* ferritin comprises the amino acid sequence shown in SEQ ID NO. 71.
98. The fusion protein of any one of embodiments 64-97, wherein said ferritin or functionally active fragment thereof comprises *Helicobacter pylori* ferritin or functionally active fragment thereof.
99. The fusion protein of any one of embodiments 64-98, wherein said ferritin or functionally active fragment thereof comprises an amino acid sequence as set forth in any one of SEQ ID NOs. 70-72.
100.The fusion protein of embodiments 64-99, wherein said ferritin or functionally active fragment thereof is directly or indirectly linked to said RBD or its functional fragment thereof and/or said NTD or functionally active fragment thereof.
101. The fusion protein of embodiments 64-100, wherein said ferritin or functionally active fragment thereof is fused in the ORF with said RBD or its functionally active fragment thereof and/or said NTD or functionally active fragment thereof.
102.The fusion protein of any one of embodiments 64-101, wherein the N-terminal of said ferritin or functionally active fragment thereof is directly or indirectly linked to the C-terminal of said RBD or functionally active fragment thereof, and the N-terminal of said RBD or functionally active fragment thereof is directly or indirectly linked to the C-terminal of said NTD or functionally active fragment thereof.
103.The fusion protein of any one of embodiments 64-102, wherein said hepatitis B surface antigen or functionally active fragment thereof comprises an amino acid sequence as set forth in any one of SEQ ID NO. 73.
104.The fusion protein of embodiments 64-103, wherein said the hepatitis B surface antigen or functionally active fragment thereof is directly or indirectly linked to said RBD or its functional fragment thereof and/or said NTD or functionally active fragment thereof.
105.The fusion protein of embodiments 64-104, wherein said the hepatitis B surface antigen or functionally active fragment thereof is fused in the ORF with said RBD or its functional fragment thereof and/or said NTD or functionally active fragment thereof.
106.The fusion protein of any one of embodiments 81-105, wherein the direct or indirect linking may comprise the linking via a linker.
107. The fusion protein of embodiment 106, wherein said linker may comprise a rigid linker, a flexible linker or other sequence.
108.The fusion protein of any one of embodiments 81-107, wherein said linker may comprise an amino acid sequence as set forth in any one of SEQ ID NOs. 89-90.
109.The fusion protein of any one of embodiments 64-108 comprising an amino acid sequence as set forth in any one of SEQ ID NOs. 39-44, 79, 85 and 96.
110.An immunogenic composition, comprising a fusion protein of any one of embodiments 1-109.
111. Said immunogenic composition of embodiment 110 comprises a first component which comprises the receptor-binding domain (RBD) of the SARS-CoV-2 spike protein (S protein) or functionally active fragment thereof and a second component comprising said fusion protein of any one of examples 1-109.
112.The immunogenic composition of embodiment 110 comprises a first component which comprises the fusion protein of any one of embodiments 1-109 and a second component comprising the N-terminal domain (NTD) of SARS-CoV-2 spike protein (S protein) or functionally active fragment thereof.
113.The immunogenic composition of embodiment 110 comprises a first component which comprises the fusion protein of any one of embodiments 1-109 and a second component comprising the fusion protein of any one of embodiments 1-109.
114.The immunogenic composition of any one of embodiments 110-113 is formulated in the following weight ratios: 1) the first component (1-15 portions in weight ratio); and/or 2) the second component (1-15 portions in weight ratio).
115.The immunogenic composition of any one of embodiments 110-114 comprising: 1) 5-60 µg of the first component; and/or 2) 5-60 µg of the second component.
116. An immunogenic composition comprising the component comprising the receptor-binding domain (RBD) of the SARS-CoV-2 spike protein (S protein) or functionally active fragment thereof and a second component comprising a N-terminal domain (NTD) of SARS-CoV-2 spike protein (S protein) or functionally active fragment thereof.
117.The immunogenic composition of embodiment 116, wherein said RBD is derived from a wild-type SARS-CoV-2 strain or a variant thereof.
118. The immunogenic composition of embodiment 117, wherein said SAR-CoV-2 variant is selected from the group consisting of a Gamma variant, a Beta variant, a Delta variant, and an Alpha variant.
119.The immunogenic composition of any one of embodiments 116-118, wherein said RBD has a mutation in one or more amino acid sites selected from the group consisting of K417, L452, T478, E484 and N501.
120.The immunogenic composition of any one of embodiments 116-119, wherein said RBD comprises one or more of the amino acid mutations of K417T/N, L452R, T478K, E484K and N501Y.
121.The immunogenic composition of any one of embodiments 116-120, wherein said RBD comprises the K417T, E484K and N501Y amino acid mutations.
122.The immunogenic composition of any one of embodiments116-121, wherein said RBD comprises the K417T, E484K and N501Y amino acid mutations.
123.The immunogenic composition of any one of embodiments 116-122, wherein said RBD comprises the amino acid sequence as set forth in any one of the SEQ ID NOs. 18, 19, 76, 83 and 97-108.
124.The immunogenic composition of embodiments 116-123, wherein said NTD is derived from a wild-type SARS-CoV-2 strain or a variant thereof.
125.The immunogenic composition of embodiment 124, wherein said SAR-CoV-2 variant is selected from of the group consisting of a Gamma variant, a Beta variant, a Delta variant, and an Alpha variant.
126.The immunogenic composition of any one of embodiments 116-125, wherein said NTD comprises a mutation at one or more amino acid sites selected from the group consisting of L18, T20, P26, D80, D138, R190, D215, L242-244, and R246.
127.The immunogenic composition of any one of embodiments 116-126, wherein said NTD comprises the mutations of one or more amino acids selected from the groups consisting of L18F, T20N, P26S, D80A, D138Y, R190S, D215G, L242-244del, and R246I.
128.The immunogenic composition of any one of embodiments 116-127, wherein said NTD comprises L18F, D80A, D215G, L242-244del and R246I amino acid mutations.
129.The immunogenic composition of any one of embodiments 116-128, wherein said NTD comprises L18F, D80A, D215G, L242-244del and R190S amino acid mutations.
130.The immunogenic composition of any one of embodiments 116-129, wherein said NTD comprises L18F, T20N, P26S, D138Y and R246I amino acid mutations.
131.The immunogenic composition of any one of embodiments 116-130, wherein said NTD comprises an amino acid sequence as set forth in any one of SEQ ID NOs. 37, 38, 77, 84 and 109-111.
132.The immunogenic composition of any one of embodiments 116-131, which is formulated in the following weight ratios: 1) the first component (1-15 portions in weight ratio); and/or 2) the second component (1-15 portions in weight ratio).
133.The immunogenic composition of any one of embodiments 111-132 comprising: 1) 5-60 µg of the first component; and/or 2) 5-60 µg of the second component.
134.The immunogenic composition of any one of the embodiments 116-133, wherein said RBD comprises the amino acid sequence as set forth in any one of the SEQ ID NOs. 18-19.
135.The immunogenic composition of any one of embodiments 116-134, wherein said NTD comprises an amino acid sequence as set forth in any one of SEQ ID NOs. 37-38.
136.One or more isolated nucleic acid molecules, which encode (s) a fusion protein of any one of claims 1-109.
137.The nucleic acid molecule in claim 136 which comprises mRNA.
138.A pharmaceutical composition, which comprises a fusion protein of any one of embodiments 1-109, or an immunogenic composition of any one of embodiments 110-135, or a nucleic acid molecule of any one of embodiments 136-137, along with arbitrarily selected pharmaceutically acceptable excipient.
139. The use of said fusion protein of any one of embodiments 1-109 or said immunogenic composition of any one of embodiments 110-135 in the manufacturing of a vaccine.
140. The use of embodiment 139, wherein said vaccine is used for the prevention and/or treatment of COVID-19.
141.The fusion protein described any one of embodiments 1-109, or said immunogenic composition of any one of embodiments 110-135, or said nucleic acid molecule of any one of embodiments 136-137, which is used for the treatment and/or prevention of COVID-19.
142.A method for making a COVID-19 subunit vaccine, which comprises:
   1) preparing a fusion protein of any one of embodiments 1-109 or an immunogenic composition of any one of embodiments 110-135; and
   2) mixing said fusion protein or immunogenic composition of 1) with a pharmaceutically acceptable adjuvant.
143.A method for detecting SARS-CoV-2 neutralizing antibodies, which comprises:
   1) administering the COVID-19 subunit vaccine of embodiment 142 to the subjects; and
   2) detecting the neutralizing antibodies produced in said subjects of 1) after receiving the COVID-19 subunit vaccine.
144.A method for treating and/or preventing COVID-19, including administering to a subject said fusion protein of any one of embodiments 1-109, said immunogenic composition of any one of embodiments 110-135, said nucleic acid molecule of any one of embodiments 136-137, or said COVID-19 subunit vaccine of embodiments 142.

Not expected to be limited by any theory, the following exemplary examples are intended solely to illustrate the fusion proteins, immunogenic compositions, preparation methods and the uses, etc. of the present application, which are not intended to limit the scope of the present invention. In addition, whether there are 6 or 8 HIS tags or N-terminal signal peptides in the examples will not affect the scope of protection, and non-substantial improvements and adjustments made to the examples by those skilled in the art in accordance with the contents of the present invention are still within the scope of protection of the present invention.

### Example 1: Construction of recombinant expression plasmid of RBD-P2-6*HIS (GP101150-7)

Addition of signal peptide: MGVPAVPEASSPRWGTLLLAIFLAASRGLVAA (SEQ ID NO. 74) Vector selection: pcdna3.1 (+), codon optimization according to the CHO host cells.

Cloning of fragments: RBD-P2-6*HIS was amplified by PCR using the primer to obtain the fragment of PCR product, which was then recombined into pcdna3.1 (+) vector (BamHI-XhoI digested vector) by multi-fragment recombination to construct the recombinant expression plasmid of RBD-P2-6*HIS (GP101150-7). The reaction system is shown in Table 1.

**Table 1 Ligation Reaction System of Processed Target Fragment and Vector**

| Name | Volume |
|---|---|
| Enzyme digested vector | 5 µl |
| Purified PCR product | 5 µl |
| Seamless assembly of MIX | 10 µl |
| Total volume | 20 µl |

The above ligation solution was placed at 52°C for 30 min to obtain the RBD-P2-6*HIS (GP101150-7) recombinant expression plasmid.

Methods of transformation: 1) 1-3 µl of RBD-P2-6*HIS (GP101150-7) recombinant expression plasmid at a concentration of 100 ng/µl was pipetted into 100 µl of competent cells, and the mixture was shaken gently and rotated to mix well, and then was allowed to stand on ice for 3 min. 2) the mixture was placed in water bath at 42°C for 90 s without shaking. 3) the mixture was placed in an ice bath for approximately 3 mins. 4) then 500-800 µl of pre-warmed LB medium was added to each tube at 37°C, and the mixture was gently shaken with a shaker at 200 rpm at 37°C for 40 min. Verification of RBD-P2-6*HIS (GP101150-7) recombinant expression plasmid:1) agar plates containing the corresponding resistance were taken. 2) 100 µl of the bacterial solution was pipetted, and spread on the agar plate containing the corresponding resistance, then the bacteria was gently spread over the surface of the plate with a sterile glass spreader, and the plate was incubated at 37°C for 15 min. 3) the plate was inverted and incubated at 37°C for 12-16 h and bacterial colonies were expected to be formed. 4) the bacteria on the plate was picked and shaken at 250 rpm at 37°C for 14 h; after the bacteria solution was identified by the PCR, and the positive clone bacteria solution was sent for sequencing.

Method for identification of cloned plasmids: the RBD-P2-6*HIS fragment was amplified by the PCR using the primer sequence for identification (synthesized by the company's internal primer department with the length of the expected amplified fragment of 861bp) and the 20 µl PCR reaction system which includes 0.5 µL of primer, 2 µL of template bacteria solution, 0.5 µL of polymerase buffer, 3 µL of buffer and 14 µL of ddH₂O. Cycling parameters: pre-denaturation at 96°C for 3 min, at 95°C for 15 s, at 58°C for 15 s, and at 72°C for 20 s for 23 cycles, and then final extension at 72°C for 1 min. Positive clones with the bacterial solution were screened by the PCR. After the positive bacterial solution was obtained it was shaken at 37°C to extract the plasmids and perform the sequencing. The plasmids with the correct sequence, as confirmed by alignment, were double digested with BamHI and XhoI to obtain the two fragments of 861 bp and 5372 bp.

Extraction of RBD-P2-6*HIS (GP101150-7) recombinant expression plasmids: 1% Escherichia coli cells (stbl3) in RBD-P2-6*HIS (GP101150-7) recombinant expression plasmids were inoculated into 2 mL of LB medium and cultured overnight at 37°C with shaking. After treatment of the cells, a plasmid extraction kit was used to extract 100 µg of plasmids. The plasmids with the correct sequence, as confirmed by alignment, were double digested with BamHI and XhoI to obtain the two fragments of 861 bp and 5372 bp.

### ***Example 2 Cell Transfection and Purification of RBD-P2-6*HIS Protein

During all the operations performed on the cells, the host cells were mixed by gentle rotation to avoid vigorous mixing/pipetting. The CHO cells (ExpiCHO from Thermo) were subcultured and amplied until the cell density reached 4×10⁶ to 6×10⁶ cells/mL. Day-1: cell amplification, the cultured cells were amplified to 3×10⁶ to 4×10⁶ cells/mL and allowed to grow overnight. Day 0: cell transfection, the density and viability of the viable cells were determined, when the cell density reached 7×10⁶ to 10×10⁶ cells/mL and the viability rate reached 95%-99%, the cells were transfected. The fresh cell expression medium was taken and prewarmed to 37°C, and after the cells were diluted to a final density of 6×10⁶ cells/mL, they were incubated at 37°C in an 8% CO₂ incubator with 50 mm amplitude and shaken at 90 rpm. The OPti-PRO SFM medium was used to prepare the cell transfection reagent and RBD-P2-6*HIS (GP101150-7) recombinant expression plasmid complex (at 4°C). For example, in 1 mL of CHO cells, 40 µl of OPti-PRO SFM was prepared and added with 0.8 µg of RBD-P2-6*HIS (GP101150-7) recombinant expression plasmids, then mixed well and allowed to stand for 5 min; and 40 µl of OPti-PRO SFM was prepared and added with 3 µL of Expi Fectamine CHO reagent, and mixed well and allowed to stand for 5 min and then allowed to stand at room temperature for 1-5 min, and then the solution was slowly transferred to a shake flask and shaken gently while adding the solution. The cells were transferred to a 50 mm amplitude incubator containing 8% CO₂ and shaken at 90 rpm at 37°C. At 18-22 h after transfection, the Enhancer and ExpiCHO Feed was added to implement the standard experiment protocol. An exemple: 6 µL of Enhancer and 0.24 mL of ExpiCHO Feed were added to 1 mL of cells, which were transferred into a 50 mm amplitude incubator with 8% CO₂ at 90 rpm at 37°C. The cells were collected 8 days after transfection for subsequent purification.

Protein purification: the culture medium was centrifuged, and the supernatant was added to the Ni column, shaken and incubated for 2 h, and then purified by affinity chromatography with an empty gravity chromatography column. Equilibration buffer: "PBS", pH 7.4; washed with 10 CV; Wash buffer: "PBS", pH 7.4 with 20 mM imidazole; rinsed with 10 CV; Elution buffer: "PBS", pH 7.4 with 500 mM imidazole; eluted with 1 CV and repeated 5 times to obtain RBD-P2-6*HIS purified protein as set forth in Table 2.

**Table 2 Concentration and Volume of Purified Protein**

| P101150-7 | Concentration(mg/ml) | Volume(ml) |
|---|---|---|
| RBD-P2-6*HIS | 0.05 | 1 |

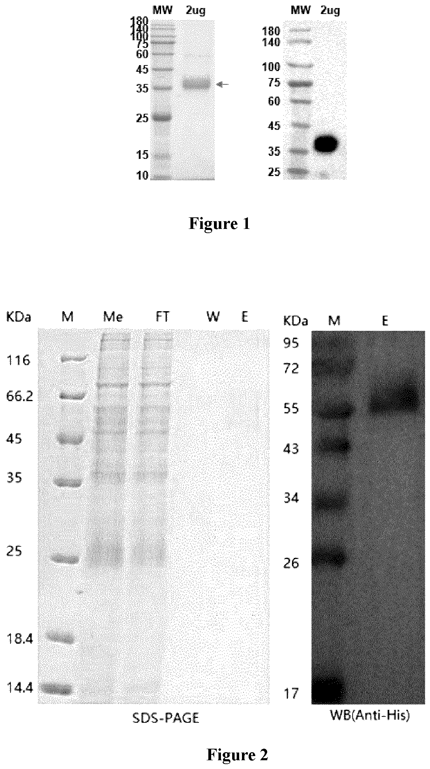

SDS-PAGE and Western blot validation. A. 1.0 mm PAGE gel; loading volume 8 µL; gel running sequence: M, molecular weight marker. Me, culture medium. FT, flow through. W, wash. E, eluted fractions. B. Electrophoresis: the RBD-P2-6*HIS purified protein was mixed with loading buffer (RBD-P2-6*HIS purified protein: 5 × loading buffer=4:1), boiled for 5 min, and spotted. Firstly, electrophoresis was performed at 100 V constant voltage, and it could be seen that the Marker gradually became a thin line. After the Marker entered the separation gel, the voltage was adjusted to 300 V and the electrophoresis was continued until the blue bromophenol blue band reached the bottom of the gel (approximately 25 min). C. Transfer: the glass plate was pried open, and the gel was carefully removed. The PVDF membrane was activated with methanol for 30s, then it was cut into the same size as the gel with the filter paper and soaked in the transfer solution, with the order of from bottom to top was: filter paper-PVDF membrane-gel-filter paper. It was ensured that there were no air bubbles between the layers, especially between the gel and the membrane. All the transfer solution in the plate was poured into the transfer cassette. The solution was transferred at 20V constant voltage. And this process required 20 min for 50 KDa protein. The higher the molecular weight, the longer the duration was required. D. Antibody incubation: the PVDF membrane was removed after transfer, and it could be seen that the Marker had been transferred onto the membrane. The membrane was blocked with 5% non-fat dry milk (1g of skimmed milk powder in 100 ml PBST) for 1 h and rinse with PBST for 2 × 5 min. The primary antibody (anti-His mab) was diluted with 5% non-fat dry milk (1 g of skimmed milk powder, 100 ml of TBS) and incubated in a small box and allowed to react for 2 h in a decolorization shaker at 4°C. The PVDF membrane was removed and rinsed with PBST for 4 × 10 min. similarly, the secondary antibody (goat anti-mouse) was diluted in 5% non-fat dry milk and incubated for 1 h and then rinsed for 4 × 10 min with PBST. All the samples were shaken with a shaker. E. Visualization with ECL: 1 mL of solution A and 10 µl of solution B were mixed and drippedonto each PBST membrane, and then photographed and recorded in the chemiluminescence imaging system after 5 min, as set forth in Figure 1.

### Example 3: Construction of NTD-P2-6*HIS (GP101150-8) Recombinant Expression Plasmid

Addition of signal peptide: MFVFLVLLPLVS (SEQ ID NO. 75), vector selection: pcdna3.1 (+). Codon optimization was performed according to the host CHO cell. NTD-P2-6*HIS was amplified by PCR using the primer to obtain the fragment PCR product, which was then recombined into pcdna3.1 (+) vector (BamHI-XhoI digested vector) by multi-fragment recombination to obtain the recombinant expression plasmid of NTD-P2-6*HIS (GP101150-8). The reaction system is shown in Table 3.

**Table 3 Ligation Reaction System of Processed Target Fragment and Vector**

| Name | Volume |
|---|---|
| Enzyme digested vector | 5 µl |
| Purified PCR product | 5 µl |
| Seamless assembly of MIX | 10 µl |
| Total volume | 20 µl |

Method of transformation: 1-3 µl of NTD-P2-6*HIS (GP101150-8) recombinant expression plasmid at a concentration of approximately 100 ng/µl was pipetted into approximately 100 µl of competent cells, and the mixture was shaken gently and rotated to mix well, and allowed to stand on ice for 3 min. The mixture was placed in a 42°C water bath for 90 s without being shaken andthen is was placed in an ice bath for about 3 min; 500-800 µl of pre-warmed LB medium was added at 37°C to each tube, which was gently shaken with a shaker at 200 rpm at 37°C for 40 min.

Validation of NTD-P2-6*HIS (GP101150-8) recombinant expression plasmid: 1) agar plates containing the corresponding antibody were taken. 2) 100 µl of the bacterial solution was pipetted and spread on the agar plate containing the corresponding antibody, and the bacteria was gently spread over the surface of the plate with a sterile glass spreader, and the plate was incubated at 37°C for 15 min. 3) the plate was inverted and incubated at 37°C for 12-16 h and bacterial colonies were formed. 4) the bacteria on the plate was picked and shaken at 250 rpm at 37°C for 14 h, and then identified with the bacteria solution by PCR, and the positive clone bacteria solution was sent for sequencing. Identification of cloned plasmids: the NTD-P2-6*HIS fragment was amplified by the PCR using the identification-primer sequence synthesized by the company's internal primer department with the length of the expected amplified fragment of 1005 bp and the 20 µl of PCR reaction system which included 0.5 µL of primer, 2 µL of template bacteria solution, 0.5 µL of polymerase buffer, 3 µL of buffer and 14 µL of ddHzO. Cycling parameters: pre-denaturation at 96°C for 3 min, at 95°C for 15 s, at 58°C for 15 s, at 72°C for 20 s for 23 cycles, and then final extension at 72°C for 1 min. Positive clones were screened with the bacterial solution by the PCR. After the positive bacterial solution was obtained, the bacteria was shaken at 37°C to extract the plasmids and the sequencing was performed. The plasmids with the correct sequences confirmed by sequence alignment double digested with BamHI and XhoI to obtain the two fragments of 1005 bp and 5372 bp.

Extraction of NTD-P2-6*HIS (GP101150-8) recombinant expression plasmid: 1% Escherichia coli cells (stbl3) in NTD-P2-6*HIS (GP101150-8) recombinant expression plasmids was inoculated into 2 mL of LB medium and cultured overnight at 37°C while shaking. After treatment of the cells, a plasmid extraction kit was used to extract 100 µg of plasmids. The plasmids with the correct sequence as confirmed by alignment were double digested with BamHI and XhoI to obtain the two fragments of 1005 bp and 5372 bp.

### ***Example 4: Cell Transfection and Purification of NTD-P2-6*HIS (GP101150-8) Protein

During all the operations performed on the cells, the host cells were mixed by gentle rotation to avoid vigorous mixing/pipetting. The CHO cells were subcultured and amplified until the cell density reached 4×10⁶ to 6×10⁶ cells/mL. Day-1: amplification of CHO cells, the cultured cells were amplified to 3×10⁶ to 4×10⁶ cells/mL and allowed to grow overnight. Day 0: cell transfection, the density and viability of the viable cells were determined, when the cell density reached 7×10⁶ to 10×10⁶ cells/mL and the viability rate to 95%-99%, the transfection was performed. The fresh cell expression medium was taken and prewarmed to 37°C, and the cells were diluted to a final density of 6×10⁶ cells/mL, and then incubated at 37°C in an 8% CO₂ incubator with 50 mm amplitude and shaken at 90 rpm. The OPti-PRO SFM medium was used to prepare the cell transfection reagent and NTD-P2-6*HIS (GP101150-8) recombinant expression plasmid complex (at 4°C). For example: for 1 mL of cells, 40 µl of OPti-PRO SFM was prepared, and 0.8 µg of NTD-P2-6*HIS (GP101150-8) recombinant expression plasmids was added and mixed well and allowed to stand for 5 min; 40 µl of OPti-PRO SFM was prepared, which was added with 3 µL of Expi Fectamine CHO reagent, and the mixture was mixed well and allowed to stand for 5 min and stand at room temperature for 1-5 min, then the solution was slowly transferred to a shake flask and shaken gently while adding the solution. The cells were transferred to a 50 mm amplitude incubator containing 8% CO₂ and shaken at 90 rpm at 37°C. At 18-22 h after transfection, the Enhancer and ExpiCHO Feed were added to implement the standard experiment protocol. Example: 6 µL of Enhancer and 0.24 mL of ExpiCHO Feed were added to 1 mL of cells, and the cells were placed in a 50 mm amplitude incubator with 8% CO₂ at 90 rpm for 37°C. And the cells were collected 8 days after transfection for subsequent purification.

Protein purification: the culture medium was centrifuged, and the supernatant was added to the Ni column, shaken and incubated for 2 h, and then purified by affinity chromatography on an empty gravity chromatography column. Equilibration buffer: "PBS", pH 7.4; washed with 10 CV Wash buffer: "PBS", pH 7.4 with 20 mM imidazole; rinsed with 10 CV Elution buffer: "PBS", pH 7.4 with 500 mM imidazole; eluted with 1 CV and repeated for 5 times to obtain NTD-P2-6*HIS (GP101150-8) purified protein, as set forth in Table 4.

**Table 4 Concentration and Volume of NTD-P2-6*HIS (GP101150-8) Purified Protein**

| P101150-8 | Concentration (mg/mL) | Volume (ml) |
|---|---|---|
| NTD-P2-6*HIS | 0.03 | 1 |

SDS-PAGE and Western blot validation. A. 1.0 mm PAGE gel; loading volume 8 µL; gel running sequence: M, molecular weight marker. Me, culture medium. FT, flow through. W, wash. E, eluted fractions. B. Electrophoresis: NTD-P2-6*HIS (GP101150-8) purified protein was mixed with loading buffer (NTD-P2-6*HIS (GP101150-8) purified protein: 5 × loading buffer=4: 1), boiled for 5 min, and spotted. Firstly, electrophoresis was performed at 100 V constant voltage, and it could be seen that the Marker gradually became a thin line. After the Marker entered the separation gel, the voltage was adjusted to 300 V and electrophoresis was continued until the blue bromophenol blue band reached the bottom of the gel (approximately 25 min). C. Transfer: the glass plate was pried open, and the gel was carefully removed. The PVDF membrane was activated with methanol for 30s, then it was cut into the same size as the gel with the filter paper and soaked in the transfer solution, with the order of from bottom to top was: filter paper-PVDF membrane-gel-filter paper. It was ensured that there were no air bubbles between the layers, especially between the gel and the membrane. All the transfer solution in the plate was poured into the transfer cassette. The solution was transferred at 20V constant voltage. And this process required 20 min for 50 KDa protein. The higher the molecular weight, the longer the duration was required. D. Antibody incubation: the PVDF membrane was removed after antibody incubation and transfer, and it could be seen that the Marker had been transferred onto the membrane. The membrane was blocked with 5% non-fat dry milk (1g of skimmed milk powder in 100 ml PBST) for 1 h and rinsed with PBST for 2 × 5 min. The primary antibody (anti-His mab) was diluted with 5% non-fat dry milk (1 g of skimmed milk powder, 100 ml of TBS) and incubated in a small box and allowed to react for 2 h in a decolorization shaker at 4°C. The PVDF membrane was removed and rinsed with PBST for 4 × 10 min. similarly, the secondary antibody (goat anti-mouse) was diluted in 5% non-fat dry milk and incubated for 1 h and then rinsed for 4 × 10 min with PBST. All the samples were shaken with a shaker. E. Visualization with ECL: 1 mL of solution A and 10 µl of solution B were mixed and dripped onto each PBST membrane, and then photographed and recorded in the chemiluminescence imaging system after 5 min, as set forth in Figure 2.

### Example 5: Construction of NTD-RBD-foldon-8*HIS Recombinant Expression Plasmid

Addition of signal peptide: MFVFLVLLPLVS (SEQ ID NO. 75), vector selection: pcdna3.1 (+). Codon optimization was performed according to host CHO cell. NTD-RBD-foldon-8*HIS was amplified by PCR using the primer to obtain the fragment PCR product, which was then recombined into pcdna3.1 (+) vector (BamHI-XhoI digested vector) by multi-fragment recombination to obtain the recombinant expression plasmid of NTD-RBD-foldon-8*HIS. The reaction system is shown in Table 5.

**Table 5 Ligation Reaction System of Processed Target Fragment and Vector**

| Name | Volume |
|---|---|
| Enzyme digested vector | 5 µl |
| Purified PCR product | 5 µl |
| Seamless assembly of MIX | 10 µl |
| Total volume | 20 µl |

Method of transformation: 1-3 µl of NTD-RBD-foldon-8*HIS recombinant expression plasmid at a concentration of approximately 100 ng/µl was pipetted into approximately 100 µl of competent cells, which was shaken gently and rotated to mix well, and allowed to stand on ice for 3 min. The mixture was placed in 42°C water bath for 90 s without shaking; and then placed in an ice bath for about 3 min; 500-800 µl of pre-warmed LB medium at 37°C was added to each tube, which were gently shaken with a shaker at 200 rpm at 37°C for 40 min.

Validation of NTD-RBD-foldon-8*HIS recombinant expression plasmid:
1) agar plates containing the corresponding antibody were taken. 2) 100 µl of the bacterial solution was pipetted, and spread on the agar plate containing the corresponding antibody, then the bacteria was gently spread over the surface of the plate with a sterile glass spreader, and the plate was incubated at 37°C for 15 min. 3) the plate was inverted and incubated at 37°C for 12-16 h and bacterial colonies were expected to be formed. 4) the bacteria on the plate was picked and shaken at 250 rpm at 37°C for 14 h; after the bacteria solution was identified by the PCR, and the positive clone bacteria solution was sent for sequencing.

Method for identification of cloned plasmids: the NTD-RBD-foldon-8*HIS fragment was amplified by PCR using the primer sequence for identification (synthesized by the company's internal primer department with the length of the expected amplified fragment of 1761 bp) and the 20 µl PCR reaction system which included 0.5 µL of primer, 2 µL of template bacteria solution, 0.5 µL of polymerase buffer, 3 µL of buffer and 14 µL of ddHzO. Cycling parameters: pre-denaturation at 96°C for 3 min, at 95°C for 15 s, at 58°C for 15 s, at 72°C for 20 s for 23 cycles, and then final extension at 72°C for 1 min. Positive clones were screened with the bacterial solution by PCR. After the positive bacterial solution was obtained, the bacteria was shaken at 37°C to extract the plasmids and perform sequencing. The plasmids with the correct sequence as confirmed by alignment were double digested with BamHI and XhoI to obtain the two fragments of 1761 bp and 5372 bp.

Extraction of NTD-RBD-foldon-8*HIS Recombinant Expression Plasmid: 1% Escherichia coli cells (stbl3) in NTD-RBD-foldon-8*HIS recombinant expression plasmids was inoculated into 2 mL of LB medium and cultured overnight at 37°C with shaking. After treatment of the cells, a plasmid extraction kit was used to extract 100 µg of plasmids. The plasmids with the correct sequence as confirmed by alignment were double digested with BamHI and XhoI to obtain the two fragments of 1761 bp and 5372 bp.

### ***Example 6: Cell Transfection and Protein Purification

During all the operations performed on the cells, the host cells were mixed by gentle rotation to avoid vigorous mixing/pipetting. The CHO cells (EXPICHO from Thermo) were subcultured and amplied until the cell density reached 4×10⁶ to 6×10⁶ cells/mL. Day-1: cell amplification, the cultured cells were amplified to 3×10⁶ to 4×10⁶ cells/mL and allowed to grow overnight. Day 0: cell transfection, the density and viability of the viable cells were determined, when the cell density reached 7×10⁶ to 10×10⁶ cells/mL and the viability rate reached 95%-99%, the cells were transfected. The fresh cell expression medium was taken and prewarmed to 37°C, and after the cells were diluted to a final density of 6×10⁶ cells/mL, they were incubated at 37°C in an 8% CO₂ incubator with 50 mm amplitude and shaken at 90 rpm.

The OPti-PRO SFM medium was used to prepare the cell transfection reagent and NTD-RBD-foldon-8*HIS recombinant expression plasmid complex (at 4°C). For example, for 1 mL of cells, 40 µl of OPti-PRO SFM was prepared and added with 0.8 µg of NTD-RBD-foldon-8*HIS recombinant expression plasmids, and the mixture was mixed well and allowed to stand for 5 min; 40 µl of OPti-PRO SFM was prepared, and added with 3 µL of Expi Fectamine CHO reagent, then mixed well and allowed to stand for 5 min and stand at room temperature for 1-5 min, then the solution was slowly transferred to a shake flask and shaken gently while adding the solution. The cells were transferred to a 50 mm amplitude incubator containing 8% CO₂ and shaken at 90 rpm at 37°C. At 18-22 h after transfection, the Enhancer and ExpiCHO Feed were added to implement the standard experiment protocol. For example, 6 µL of Enhancer and 0.24 mL of ExpiCHO Feed were added to 1 mL of cells, and the cells were placed in a 50 mm amplitude incubator with 8% CO₂ at 90 rpm for 37°C. The cells were collected 8 days after transfection for subsequent purification.

Protein purification: the culture medium was centrifuged, and the supernatant was added to the Ni column, shaken and incubated for 2 h, and then purified by affinity chromatography on an empty gravity chromatography column. Equilibration buffer: "PBS", pH 7.4; washed with 10 CV Wash buffer: "PBS", pH 7.4 with 20 mM imidazole; rinsed with 10 CV Elution buffer: "PBS", pH 7.4, with 500 mM imidazole; eluted with 1 CV and repeates for 5 times to obtain NTD-RBD-foldon-8*HIS purified protein. The results are shown in Table 6.

**Table 6 Concentration and Volume of NTD-RBD-foldon-8*HIS Purified Protein**

| P101150-5 | Concentration (mg/ml) | Volume (ml) |
|---|---|---|
| NR-foldon-8*HIS | 0.05 | 0.5 |

SDS-PAGE and Western blot validation. A. 1.0 mm PAGE gel; loading volume 8 µL; gel running sequence: M, molecular weight marker. Me, culture medium. FT, flow through. W, wash. E, eluted fractions. B. Electrophoresis: NTD-RBD-foldon-8*HIS purified protein was mixed with loading buffer (NR-foldon-8*HIS purified protein: 5 × loading buffer=4:1), boiled for 5 min, and spotted. Firstly, electrophoresis was performed at 100 V constant voltage, and it could be seen that the Marker gradually became a thin line. After the Marker entered the separation gel, the voltage was adjusted to 300 V and electrophoresis was continued until the blue bromophenol blue band reached the bottom of the gel (approximately 25 min). C. Transfer: the glass plate was pried open, and the gel was carefully removed. The PVDF membrane was activated with methanol for 30s, then it was cut into the same size as the gel with the filter paper and soaked in the transfer solution, with the order of from bottom to top was: filter paper-PVDF membrane-gel-filter paper. It was ensured that there were no air bubbles between the layers, especially between the gel and the membrane. All the transfer solution in the plate was poured into the transfer cassette. The solution was transferred at 20V constant voltage. And this process required 20 min for 50 KDa protein. The higher the molecular weight, the longer the duration was required. D. The PVDF membrane was removed after antibody incubation and transfer, and it could be seen that the Marker had been transferred onto the membrane.

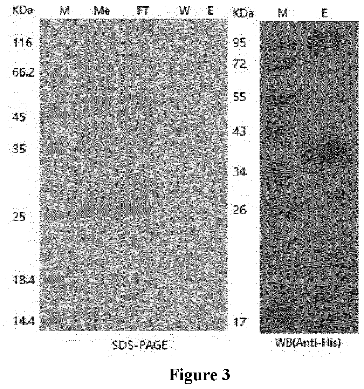

The membrane was blocked with 5% non-fat dry milk (1g of skimmed milk powder in 100 ml PBST) for 1 h and rinsed with PBST for 2 × 5 min. The primary antibody (anti-His mab) was diluted with 5% non-fat dry milk (1 g of skimmed milk powder, 100 ml of TBS) and incubated in a small box and allowed to react for 2 h in a decolorization shaker at 4°C. The PVDF membrane was removed and rinsed with PBST for 4 × 10 min. similarly, the secondary antibody (goat anti-mouse) was diluted in 5% non-fat dry milk and incubated for 1 h and then rinsed for 4 × 10 min with PBST. All the samples were shaken with a shaker. E. Visualization with ECL: 1 mL of solution A and 10 µl of solution B were mixed and dripped onto each PBST membrane, and then photographed and recorded in the chemiluminescence imaging system after 5 min, as set forth in Figure 3.

### Example 7: Construction of Vectors and Protein Expression

The following target fragments to be expressed were constructed and the purified protein was expressed with reference to the examples mentioned above, as set forth in Table 7.

**Table 7 Different Target Fragments**

| Gene name | Construction mode | SEQ ID NO. |
|---|---|---|
| RBD-HBsAg-6*HIS | Total gene synthesis | 45 |
| NTD-HBsAg-6*HIS | Total gene synthesis | 46 |
| RBD-foldon-6*HIS | Total gene synthesis | 47 |
| NTD-foldon-6*HIS | Total gene synthesis | 48 |
| NTD-RBD-foldon-8*HIS | Total gene synthesis | 49 |
| S-foldon-8*HIS | Total gene synthesis | 50 |
| RBD- P2-6*HIS | Total gene synthesis | 51 |
| NTD- P2-6*HIS | Total gene synthesis | 52 |
| S1-P2-8*HIS | Total gene synthesis | 53 |
| RBD- P2-foldon-6*HIS | Total gene synthesis | 54 |
| NTD- P2-foldon-6*HIS | Total gene synthesis | 55 |
| S1-P2- foldon-6*HIS | Total gene synthesis | 56 |
| RBD-ferritin LC-6*HIS | Total gene synthesis | 57 |
| RBD-ferritin HC-FLAG | Total gene synthesis | 58 |
| RBD194-HBsAg | PCR cloning | 59 |
| NTD-ferritin HC nucleic acid sequence | PCR cloning | 60 |
| NTD-ferritin LC nucleic acid sequence | PCR cloning | 61 |
| NTD ferritin nucleic acid sequence | Total gene synthesis | 62 |
| RBD ferritin nucleic acid sequence | Total gene synthesis | 63 |

### ***Example 8: Preparation of Vaccine Composition

0.04 mg/mL RBD protein, 0.04 mg/mLNTD protein, 2% to 15% (w/v) sucrose and 0.01% to 0.05% (w/v) Tween 80 and 5-25 mM histidine buffer at pH of 5.0-7.0 were pipetted, and the mixture was aliquoted into 2 ml vials with 0.5 ml per vial, placed in a lyophilizer for freeze-drying.

### ***Example 9: Preparation of Vaccine Composition

0.04 mg/mLRBD-P2 protein, 0.04 mg/mLNTD-P2 protein, 2% to 15% (w/v) sorbitol, 0.01% to 0.05% (w/v) polysorbate 20, 5-20 mM histidine buffer at pH 5.5-7.0 were pipetted, and the mixture was aliquoted into 2 ml vials with 0.5 ml per vial, placed in a lyophilizer for freeze-drying.

### ***Example 10: Preparation of Vaccine Composition

0.04 mg/mLRBD protein, 0.04 mg/mLNTD-P2 protein, 2% to 15% (w/v) sucrose and 0.01% to 0.05% (w/v) Tween 80 and 5-25 mM succinic acid buffer at pH of 4.5-5.5 were pipetted, and the mixture was aliquoted into 2 ml vials with 0.5 ml per vial, placed in a lyophilizer for freeze-drying.

### ***Example 11: Preparation of Vaccine Composition

0.04 mg/mL RBD-P2 protein, 0.04 mg/mLNTD protein, 2% to 15% (w/v) sorbitol, 0.01% to 0.05% (w/v) polysorbate 20, 5-20 mM histidine buffer at pH 5.5-7.0 were pipetted, and the mixture was aliquoted into 2 ml vials with 0.5 ml per vial, placed in a lyophilizer for freeze-drying.

### ***Example 12: Preparation of Vaccine Composition

The immunogenic composition of any one of the example 8-11 further comprises an aluminium hydroxide adjuvant at a concentration of 1 mg/mL.

CpG1018 adjuvant was added into the immunogenic composition of any one of the examples 8-11 at a concentration of 6 mg/mL. CpG adjuvant (CpG synthesized by Shanghai Sangon Biotech Co., Ltd. according to the CpG1018 sequence).

### ***Example 13: Preparation of Vaccine Composition

The said immunogenic composition of any one of the examples 8-11 further comprises an adjuvant, for example, the adjuvant vial is 0.5mL, and the components include 50 µg of MPL, 500 µg of aluminium hydroxide, 150 mM of NaCl, 8 mM disodium hydrogen phosphate dihydrate, and water for injection to 0.5 mL.

### ***Example 14: Preparation of Vaccine Composition

The said immunogenic composition of any one of the examples 8-11 further comprises an adjuvant, for example, a 0.25mL vial of adjuvant contains 10.69 mg of squalene, 11.86 mg of α-tocopherol, 4.86 mg of Tween 80, 3.53 mg of NaCl, 0.09 mg of KCl, 0.51 mg of Na₂HPO₄, 0.09 mg of KH₂PO₄ and water for injection.

### ***Example 15: Preparation of Vaccine Composition

The said immunogenic composition of any one of the examples 8-11 further comprises an adjuvant, for example, a 0.5mL vial of adjuvant contains 50 µg of MPL, 50 µg of QS-21, 1 mg of dioleoylphosphatidylcholine (DOPC), 0.25 mg of cholesterol, 0.15 mg of anhydrous disodium phosphate, 0.54 mg of monobasic potassium phosphate, 4.385 mg of sodium chloride, and water for injection.

### ***Example 16: Preparation of Vaccine Composition

The said immunogenic composition of any one of the examples 8-11 further comprises an adjuvant, for example, a 0.5mL vial of adjuvant contains 9.75 mg of squalene, 1.175 mg of span 85, 1.175 mg of Tween 80, 0.66 mg of trisodium citrate dihydrate and 0.04 mg of citric acid monohydrate, and water for injection.

### Example 17: Immunization Experiments Using Different Antigens and Adjuvants

The antigenic proteins were co-administered with different adjuvants to immunize mice, and the levels of neutralizing antibodies induced by the proteins were determined by ELISA assay. The grouping of mice and the immunization schedule are shown in Table 8.

**Table 8 Grouping of Mice**

| **Animal No.** | **Antigen** | **Adjuvant** | **Antigen dose** | **Immunizatio n procedure** | **Immunizat ion route** | **Number of animals** |
|---|---|---|---|---|---|---|
| 1 | RBD | - | (2+2)µg | 3 doses, 14 days apart | Muscle- 0.1 ml | 5 |
| 2 | RBD | Al(OH)₃ | (2+2)µg | 3 doses, 14 days apart | Muscle- 0.1 ml | 5 |
| 3 | RBD | CpG | (2+2)µg | 3 doses, 14 days apart | Muscle- 0.1 ml | 5 |
| 4 | RBD | CpG+Al(OH)₃ | (2+2)µg | 3 doses, 14 days apart | Muscle- 0.1 ml | 5 |
| 5 | RBD | AS01 | (2+2)µg | 3 doses, 14 days apart | Muscle- 0.1 ml | 5 |
| 6 | RBD | AS04 | (2+2)µg | 3 doses, 14 days apart | Muscle- 0.1 ml | 5 |
| 7 | NTD+RB D | - | (2+2)µg | 3 doses, 14 days apart | Muscle- 0.1 ml | 5 |
| 8 | NTD+RB D | Al(OH)₃ | (2+2)µg | 3 doses, 14 days apart | Muscle- 0.1 ml | 5 |
| 9 | NTD+RB D | CpG | (2+2)µg | 3 doses, 14 days apart | Muscle- 0.1 ml | 5 |
| 10 | NTD+RB D | CpG+Al(OH)₃ | (2+2)µg | 3 doses, 14 days apart | Muscle- 0.1 ml | 5 |
| 11 | NTD+RB D | AS01 | (2+2)µg | 3 doses, 14 days apart | Muscle- 0.1 ml | 5 |
| 12 | NTD+RB D | AS04 | (2+2)µg | 3 doses, 14 days apart | Muscle- 0.1 ml | 5 |
| 13 | NTD+RB D | - | - | 3 doses, 14 days apart | Muscle- 0.1 ml | 5 |

Wherein the NTD protein sequence was amino acid 1-291 in SEQ ID NO. 20, the RBD sequence was amino acid 1-223 in SEQ ID NO.1, the concentrations of adjuvants Al (OH) ₃, CpG and CpG + Al (OH) ₃ were determined with reference to example 12, the composition of AS01 was determined with reference to example 15, and the composition of AS04 was determined with reference to example 13; and the test results 28 days after immunization in mice are shown in Table 9. The results showed that RBD plus NTD induced higher levels of neutralizing antibodies.

**Table 9 Test Results 28 Days after Immunization of Mice**

| **Antigen** | **Adjuvant** | **Geometric mean titer (GMT)** | **Antigen** | **Adjuvant** | **Geometric mean titer (GMT)** |
|---|---|---|---|---|---|
| RBD | - | 4 | RBD+NTD | - | 4.916 |
| RBD | Al(OH)₃ | 11.24 | RBD+NTD | Al(OH)₃ | 19.54 |
| RBD | CpG | 4 | RBD+NTD | CpG | 4 |
| RBD | CpG+Al(OH)₃ | 58.89 | RBD+NTD | CpG+Al(OH)₃ | 117.5 |
| RBD | AS01 | 9.791 | RBD+NTD | AS01(B/c) | 11.64 |
| RBD | AS04 | 13.82 | RBD+NTD | AS04 | 83.18 |

### Example 18 Experiments Evaluating the Immunogenicity of Different Antigens with Adjuvants

The antigenic proteins were co-administered with adjuvant MF59 to immunize Japanese rabbits, and the levels of neutralizing antibodies induced by the proteins were determined by ELISA assay. The grouping and immunization schedule of Japanese rabbits are shown in Table 10.

**Table 10 Grouping of Japanese Rabbits for Immunization**

| **Anima l No.** | **Antigen** | **Adjuvant** | **Antigen dose** | **Immunization procedure** | **Immun ization volume** | **Number of animals** |
|---|---|---|---|---|---|---|
| 1 | NTD+RBD | MF59 | (10+10)µg | 3 doses, 14 days apart | 0.5ml | 6 |
| 2 | NTD-P2+RBD-P2 | MF59 | (10+10)µg | 3 doses, 14 days apart | 0.5ml | 6 |
| 3 | NTD-foldon+RBD-foldon | MF59 | (10+10)µg | 3 doses, 14 days apart | 0.5ml | 6 |
| 4 | NTD-ferritin LC+RBD-ferritin | MF59 | (10+10) µg | 3 doses, 14 days apart | 0.5ml | 6 |
| 5 | NTD-HBsAg+RBD-HBsAg | MF59 | (10+10) µg | 3 doses, 14 days apart | 0.5ml | 6 |
| 6 | NTD-RBD-P2 | MF59 | 20 µg | 3 doses, 14 days apart | 0.5ml | 6 |
| 7 | NTD-RBD-foldon | MF59 | 20 µg | 3 doses, 14 days apart | 0.5ml | 6 |
| 8 | NTD-RBD-P2-foldon | MF59 | 20 µg | 3 doses, 14 days apart | 0.5ml | 6 |
| 9 | NTD-RBD-ferritin | MF59 | 20 µg | 3 doses, 14 days apart | 0.5ml | 6 |
| 10 | NTD-RBD-HBsAg | MF59 | 20 µg | 3 doses, 14 days apart | 0.5ml | 6 |

The sequence of NTD-P2 was described in SEQ ID NO. 20, the sequence of RBD-P2 was described in SEQ ID NO. 1, the sequence of NTD-foldon was described in SEQ ID NO. 22, the sequence of RBD-foldon was described in SEQ ID NO. 3, the sequence of NTD-ferritin LC was described in SEQ ID NO. 23, the sequence of RBD-ferritin was determined with reference to SEQ ID NO. 5, the sequence of NTD-HBsAg was determined with reference to SEQ ID NO.25, the sequence of RBD-HBsAg was determined with reference to SEQ ID NO.6, and the adjuvant MF59 was determined with reference to example 16.

The sequence of NTD-RBD-P2 was determined with reference to that of SEQ ID NO.40 but with no HIS tag at the C-terminal; the sequence of NTD-RBD-foldon was determined with reference to that of SEQ ID NO. 41 but with no HIS tag at the C-terminal; the sequence of NTD-RBD-P2-foldon was determined with reference to that of SEQ ID NO.42 but with no HIS tag at the C-terminal; the sequence of NTD-RBD-ferritin was determined with reference to that of SEQ ID NO.43; and the sequence of NTD-RBD-HBsAg was determined with reference to that of SEQ ID NO. 44. The test results in Japanese rabbits 28 days after immunization are shown in Table 11, indicating that the fusion protein was immunogenic.

**Table 11 Test Results in Japanese rabbits 28 Days after Immunization**

| **Group** | **Antigen** | **Adjuvant** | **Dosage** | **Geometric mean titer (GMT)** |
|---|---|---|---|---|
| 1 | NTD+RBD | MF59 | (10+10) µg | 71.84 |
| 2 | NTD-P2+RBD-P2 | MF59 | (10+10) µg | 203.2 |
| 3 | NTD-foldon+RBD-foldon | MF59 | (10+10) µg | 45.25 |
| 4 | NTD-ferritin+RBD-ferritin | MF59 | (10+10) µg | 128 |
| 5 | NTD-HBsAg+ RBD-HBsAg | MF59 | (10+10) µg | 128 |
| 6 | NTD-RBD-P2 | MF59 | 20 µg | 456.1 |
| 7 | NTD-RBD-foldon | MF59 | 20 µg | 1024 |
| 8 | NTD-RBD-P2-foldon | MF59 | 20 µg | 1024 |
| 9 | NTD-RBD-ferritin | MF59 | 20 µg | 512 |
| 10 | NTD-RBD-HBsAg | MF59 | 20 µg | 456.1 |

### Example 19: Experiments Evaluating the Immunogenicity of Different Antigens and Adjuvants

The antigenic proteins were co-administered with different adjuvants to immunize rhesus monkeys, and the levels of neutralizing antibodies induced by the proteins were determined by ELISA assay. The grouping and immunization schedule of Rhesus monkeys are as follows, and the positive control was inactivated COVID-19 vaccine. The test results in rhesus monkeys 28 days after vaccination are shown in Table 12.

**Table 12 Immunization Results in Rhesus Monkeys**

| Group | Antigen | Adjuvant | Dosage | Geometric mean titer (GMT) |
|---|---|---|---|---|
| Gr1 | RBD+NTD-P2 | AS04 | (20+20) µg | 152.2 |
| Gr2 | RBD+NTD-P2 | AS04 | (10+10) µg | 256 |
| Gr3 | RBD+NTD-P2 | Al(OH)₃+CpG1.0 mg | (20+20) µg | 107.6 |
| Gr4 | RBD+NTD-P2 | AS03 | (20+20) µg | 608.9 |
| Gr5 | RBD+NTD-P2 | PIKA | (10+10) µg | 45.25 |
| Gr6 | Positive control inactivated vaccine | Al(OH)₃ | 3 µg | 90.51 |

### Example 20: Detection of the Effect of Candidate Antigens in Recombinant COVID-19 Vaccine in Inducing Neutralizing Antibody Production in Mice

NTD and RBD were selected as the candidate antigens based on the WIV04-1 sequence. The immunogenicity of NTD alone, RBD alone, co-immunization with NTD plus RBD, NTD-RBD fusion protein (hereinafter referred to as NR) and NTD-RBD-foldon fusion protein (hereinafter referred to as NR-foldon) was tested and the results are shown in Table 14. The results showed that the NR-foldon antigen combined with BFA03 adjuvant could significantly and effectively induce the body to produce high titers of neutralizing antibody (based on SARS-CoV-2 virus strain and VSV pseudovirus detection system) and antigen-specific IgG antibody in BALB/c mice. The neutralizing effect on WIV04 prototype strain, D614G and Gamma variants was significantly better than that on other antigens.

**Table 13 Overview of Experimental Design of Candidate Antigens in Recombinant COVID-19 Vaccine in BALB/c Mice**

| Antigen | Antigen dose | Immunization procedure | Blood sampling time point (d) |
|---|---|---|---|
| NTD | 4 µg | 2 doses 14 days apart | 0,14,28,42 |
| RBD | 4 µg | 2 doses 14 days apart | 0,14,28,42 |
| RBD+NTD | 2+2 µg | 2 doses 14 days apart | 0,14,28,42 |
| NR | 4 µg | 2 doses 14 days apart | 0,14,28,42 |
| NR-foldon | 4 µg | 2 doses 14 days apart | 0,14,28,42 |

**Table 14 Results of Screening of Candidate Antigens in Recombinant COVID-19 Vaccine in BALB/c mice**

| Antibody type | | NTD | RBD | RBD+NTD | NR | NR-foldon |
|---|---|---|---|---|---|---|
| Neutralizing antibody (live virus) ¹ titer (GMT) | | 16 | 588 | 512 | 891 | 1176 |
| Neutralizing antibody (pseudovirus) ² titer (GMT) | WIV04-1 | 168 | 2799 | 1132 | 5358 | 9234 |
| | D614G | 89 | 2947 | 1220 | 3600 | 8439 |
| | B.1.1.7 | 39 | 2250 | 891 | 2706 | 3587 |
| | B.1.351 | 56 | 3094 | 894 | 3130 | 1786 |
| | P.1 | 120 | 3262 | 900 | 5742 | 8263 |
| | B.1.617.2 | 32 | 1777 | 885 | 2125 | 1778 |
| | C.37 | 21 | 1977 | 765 | 2107 | 1997 |
| IgG antibody titer | | 200000 | 606287 | 696440 | 1206835 | 1741101 |

| | | | | | | |
|---|---|---|---|---|---|---|
| Note: 1. Neutralizing antibody test based on the anti-SARS-CoV-2 strain, similarly hereinafter. 2. Types of pseudovirus: prototype strain (WIV04-1), main epidemic strain (D614G); VOC variant: English variant (Alpha, B.1.1.7), South African variant (Beta, B.1.351), Brazilian variant (Gamma, P.1), Indian variant (Delta, B.1.617.2); VOI variant strain: Peru variant strain (Lambda, C.37), similarly hereinafter. | | | | | | |

### Example 21: Detection of Immunization Effect of Different Adjuvants

Al (OH)₃, BFA01, BFA02, BFA03, BFA04 and CpG/Al (OH) were selected respectively in combination with NR-foldon antigen to immunize the BALB/c mice. Then the neutralizing antibodies and antigen-specific IgG antibodies were detected. The results showed that among the candidate adjuvants, the immunization effect of adjuvant BFA03 was the best and significantly superior to the traditional aluminium adjuvant (aluminium hydroxide adjuvant) as well as the compound adjuvant system based on aluminium salt adjuvant.

**Table 15 Overview of Experimental Design of Candidate Adjuvants in Recombinant COVID-19 Vaccine in BALB/c Mice**

| Adjuvant | Antigen dose | Immunization procedure | Blood sampling time point (d) | Comments ¹ |
|---|---|---|---|---|
| Al(OH)₃ | 4µg | 2 doses 14 days apart | 0,14,28,42 | Aluminium content: 500 µg |
| BFA03 | 4µg | 2 doses 14 days apart | 0,14,28,42 | Tween 80: 4.86 mg,α -tocopherol: 11.86 mg, squalene: 10.69 mg |
| CpG/Al(OH)₃ | 4µg | 2 doses 14 days apart | 0,14,28,42 | CpG: 3000 µg, aluminium content: 500 µg |
| BFA02 | 4µg | 2 doses 14 days apart | 0,14,28,42 | Tween 80: 1.175 mg, span 85: 1.175 mg, squalene: 9.75 mg |
| BFA01 | 4µg | 2 doses 14 days apart | 0,14,28,42 | DOPC: 1 mg, cholesterol: 0.25 mg, MPL: 50 µg, QS-21: 50 µg |
| BFA04 | 4µg | 2 doses 14 days apart | 0,14,28,42 | MPL: 50 µg, aluminium content: 500 µg |

| | | | | |
|---|---|---|---|---|
| Note: 1. Content of main components in the adjuvant in vaccine at human dose (0.5 ml). The immunization dose in BALB/c mice is 1/5 HD. | | | | |

**Table 16 Experimental Results of Candidate Adjuvants in Recombinant COVID-19 Vaccine in BALB/c Mice**

| Adjuvant | Titer of anti-SARS-CoV-2 virus neutralizing antibody | Titer of anti-D614G pseudovirus neutralizing antibody | Titer of antigen-specific IgG antibody |
|---|---|---|---|
| Al(OH)₃ | 388 | 1356 | 303143 |
| CpG+Al(OH)₃ | 223 | 460 | 1600000 |
| BFA02 | 776 | 2039 | 870551 |
| BFA01 | 1024 | 4624 | 1000000 |
| BFA04 | 861 | 4054 | 672717 |
| BFA03 | 1176 | 8439 | 1741101 |

### Example 22: Experiments Detecting Immunization Effect of the Vaccine at Different Doses in in Mice

BALB/c mice were immunized with 1/10 and 1/5 of human dose (i.e., 4 µg and 8 µg) of NR-foldon antigen in combination with 1/5 or 1/10 of human dose of BFA03 adjuvant, respectively. The results showed that on the premise of fixed antigen dose, the titer of neutralizing antibody induced by 1/10 HD adjuvant was lower than that by the 1/5 HD adjuvant, with no statistical difference between the groups. To some extent, it is considered that stronger immune responses could be induced at a proper ratio between antigen and adjuvant.

**Table 17 Results of Dose-finding of Recombinant COVID-19 Vaccine in BALB/c Mice**

| Antigen dose | Adjuvant dose | Titer of pseudovirus neutralizing antibody |
|---|---|---|
| 1/5 HD | 1/10 HD | 36825 |
| 1/5 HD | 1/5 HD | 77315 |
| 1/10 HD | 1/10 HD | 27389 |
| 1/10 HD | 1/5 HD | 70673 |

### Example 23: Experiments Detecting Immunization Effect of the Vaccine at Different Doses in Rabbits

Japanese rabbits were immunized with one human dose of adjuvant BFA03 (0.5 ml) in combination with NR-foldon antigen at two different doses of 20 µg and 40 µg, respectively. The results are shown in Table 18, which shows that the antibody titer produced by immunization with 40 µg of antigen was higher.

**Table 18 Results of Immunization in Japanese rabbits with Different Doses of Adjuvants**

| Antigen dose | Adjuvant dose | Titer of anti-SARS-CoV-2 virus neutralizing antibody | Titer of pseudovirus neutralizing antibody | Titer of antigen-specific IgG antibody |
|---|---|---|---|---|
| 20µg | 1HD | 1024 | 9956 | 2674961 |
| 40µg | 1HD | 1783 | 30697 | 4827341 |

1 HD (0.5 mL), 1/2 HD and 1/4 HD adjuvant BFA03 were separately selected and combined with 1 HD NR-foldon antigen to immunize Japanese rabbits and the results are shown in Table 19. The combination of 1 HD adjuvant and 1 HD antigen induced the highest levels of neutralizing antibody and binding antibody and the decrease in the dose of adjuvant would reduce the immunizing effect.

**Table 19 Results of Immunization in Japanese rabbits with Different Doses of Adjuvants**

| Antigen dose | Adjuvant dose | Titer of pseudovirus neutralizing antibody | Antigen-specific IgG antibody |
|---|---|---|---|
| 1HD | 1HD | 30697 | 4827341 |
| 1HD | 1/2HD | 7200 | 2111213 |
| 1HD | 1/4HD | 6098 | 1829220 |

### Example 24: Detection of Neutralizing Effect of Antigen-adjuvant Combinations on Different COVID-19 Variants

The results of neutralizing antibody titer (GMT) produced by different species of animals immunized with ReCOV vaccine (NR-foldon antigenBFA03 adjuvant) are as follows. The table shows that the determined antigen-adjuvant combination had a good neutralizing effect on SARS-CoV-2 virus as well as different variants.

**Table 20 Results of Titers of Neutralizing Antibody Produced by Different Species**

| Type of neutralizing antibody | BALB/c mice | Japanese rabbits | Rhesus monkeys |
|---|---|---|---|
| SARS-CoV-2 | 6208 | 1783 | 1825 |
| WIV04-1 | 22626 | 9148 | 5958 |
| D614G | 58040 | 30697 | 18694 |
| B.1.1.7(Alpha) | 19722 | 8354 | 8156 |
| B.1.351(Beta) | 23488 | 1588 | 1048 |
| P.1(Gamma) | 69107 | 2768 | 3193 |
| B.1.617.2(Delta) | 16833 | 17400 | 8441 |

### Detection of the effect of the use of NTD and RBD of S protein of Gamma variant and NTD and RBD of S protein of Beta variant

### Example 25: Construction of Recombinant Expression Plasmids of NTD-RBD-foldon-6 × His (Gamma variant) and NTD-RBD-foldon-6 × His (Beta variant)

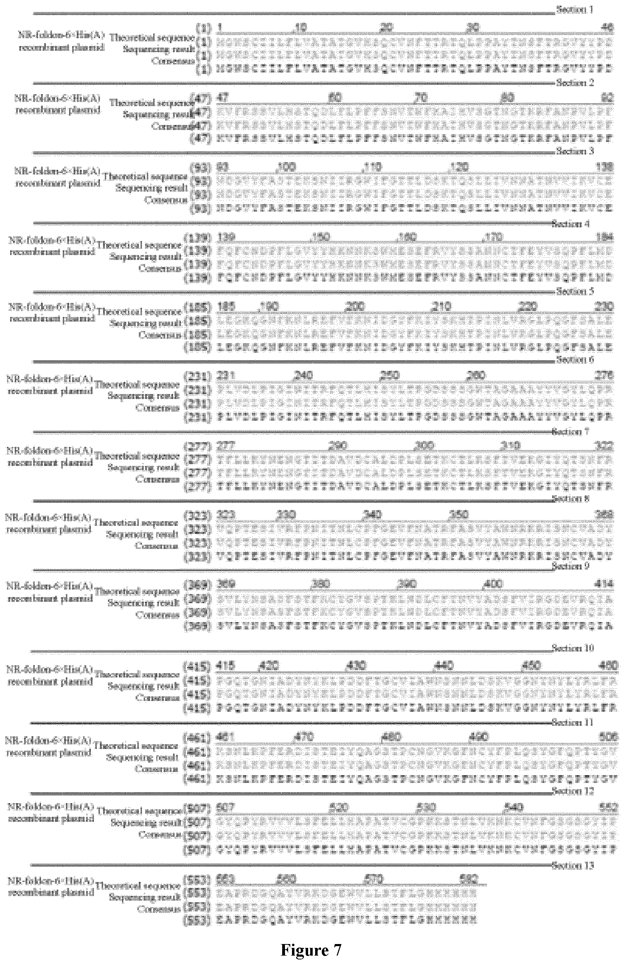

The NTD-RBD-foldon (Gamma variant) shown in SEQ ID NO.85 was added with signal peptide MGWSCIILFLVATATGVHS (SEQ ID NO. 91) and 6 × His; the NTD-RBD-foldon (Beta variant) shown in SEQ ID NO.79 was added with signal peptide MGWSCIILFLVATATGVHS (SEQ ID NO. 91) and 6 × His, and the codon of the full-length amino acid sequence was optimized by Wuxi AppTec Biopharmaceuticals Co., Ltd., and the DNAfragments of NTD-RBD-foldon-6 × His (Gamma variant) and NTD-RBD-foldon-6 × His (Beta variant) were obtained via whole gene synthesis by PCR. After gel purification, the purified DNA fragments were recombined into the target vector pcDNA3.1 (+) to obtain the recombinant expression plasmids of NTD-RBD-foldon-6 × His (Gamma variant) and NTD-RBD-foldon-6 × His (Beta variant). And the sequences were verified to be 100% correct by Sanger sequencing. The sequencing results are shown in Figure 1 and Figure 7.

Transformation of host bacteria: 1) 1-3 µL of recombinant expression plasmids of NTD-RBD-foldon-6 × His (Gamma variant) or NTD-RBD-foldon-6 × His (Beta variant) at the concentration of 100 ng/µL were added into 100 µl of top 10 *Escherichia coli* competent cells, mixed well by gentle shaking and rotation, and placed on ice for 3 min. 2) The solution was placed into water bath at 42°C for 90 s without shaking. 3) Place in an ice bath for approximately 3 min. 4) Add 500-800 µl of pre-warmed LB medium at 37°C to each tube, and gently shake with a shaker at 200 rpm at 37°C for 40 min. Extraction and sequencing of recombinant expression plasmids: 1) the agar plates containing 100 µg/mL ampicillin were prepared. 2) 100 µl of the bacterial solution was taken and the colonies on the LB agar plate containing 100 µg/mL ampicillin were gently streaked with a sterile glass spreader, and the plate was incubated at 37°C for 15 min. 3) the plate was inverted and incubated at 37°C for 12-16 h and bacterial colonies were formed. 4) a single clone was picked from the plate and inoculated into 300 mL LB medium for amplification, and then a large amout of plasmids were prepared using NucleoBond Xtra Maxi EF kit according to the instructions. The target genes were verified by sequencing, and the sequencing result was consistent with the designed genome sequence.

### ***Example 26: Cell Transfection and Protein Purification

### Cell transfection:

During all the operations performed on the cells, the host cells were mixed by gentle rotation to avoid vigorous mixing/pipetting. The CHO cells (EXPICHO from Thermo) were subcultured and amplified until the cell density reached 4×10⁶ to 6×10⁶ cells/mL. Transfection Day-1: the density of the cultured cells was adjusted to 3×10⁶ to 4×10⁶ cells/mL and the cells were allowed to grow overnight. Day 0: cell transfection, the viable cell density and survival rate were determined, the density of the cells was expected to reach 7×10⁶ to 10×10⁶ cells/mL, and the survival rate were expected to reach 95% to 99% before the transfection. Fresh cell expression medium was preheatedwarmed to 37°C, and the cells were diluted to a final density of 6 ×10⁶ cells/mL and incubated at 37°C in a 50 mm amplitude incubator containing 8% CO₂ at 90 rpm. OptiPRO^{™} SFM medium was used to prepare the transfection reagent and NTD-RBD-foldon-6×His (Gamma variant/Beta variant) recombinant expression plasmid complex (at 4°C). For example: for 1 mL of CHO cells, 40 µl of OptiPRO^{™} SFM was prepared and added with 0.8 µg of recombinant expression plasmids of NTD-RBD-foldon-6 × His (Gamma variant/Beta variant), the solution was mixed well and allowed to stand for 5 min; and 40 µl of OptiPRO^{™} SFM was prepared and added with 3 µL of ExpiFectamine^{™} CHO reagent and the solution was mixed well and allowed to stand for 5 min and placed at room temperature for 1-5 min, then the solution was slowly transferred to a shake flask and shaken gently during the addition process. The cells were transferred to a 50 mm amplitude incubator containing 8% CO₂ and shaken at 90 rpm at 37°C. At 18-22 h after transfection, the Enhancer and ExpiCHO Feed were added to implement the standard experiment protocol. Example: 6 µL of Enhancer and 0.24 mL of ExpiCHO Feed were added into 1 mL of cells, and the cells were placed and incubated in a 50 mm amplitude incubator containing 8% CO₂ at 90 rpm at 37°C. The cells were harvested 6 days after transfection for subsequent purification.

### Protein purification:

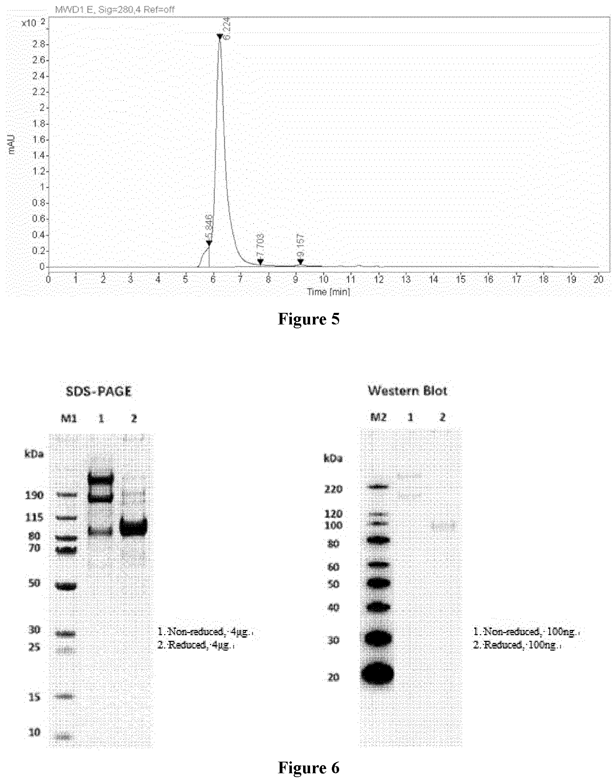

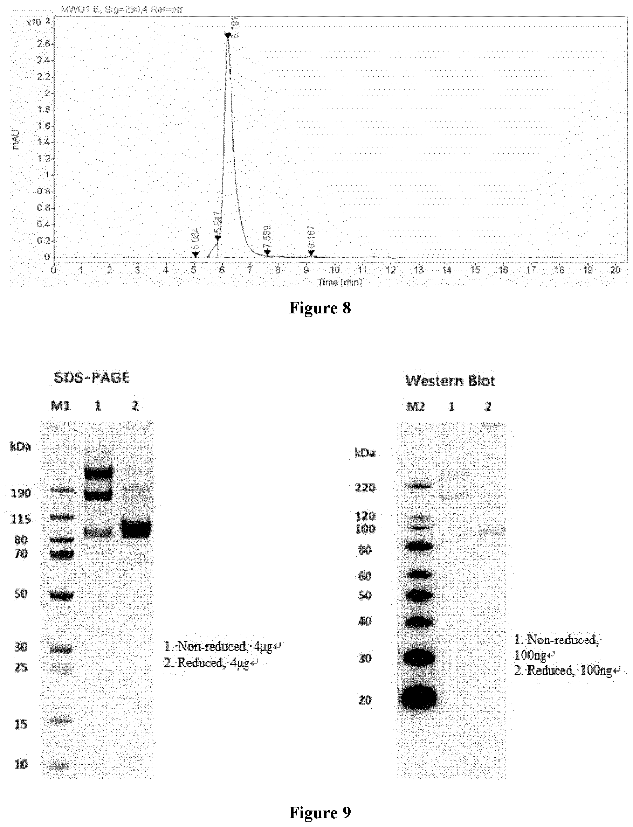

Immobilized-metal affinity chromatography (IMAC) and size-exclusion chromatography (SEC) were used for purification. A Hi Trap 5ml Chelating HP column (GE, USA) packed with NiSO₄ was used. After pretreatment with chelate chromatography medium, the column was loaded and washed with 50 mmol/L EDTA, 0.2 mol/L NaOH and ultrapure water, respectively. After equilibration with lysis buffer (5X), the supernatant of cell lysis was directly loaded onto the column, and gradient elution was performed with solution A (20 mmol/L PB, pH 7.4, 0.15 mol/L NaCl) and solution B (20 mmol/L PB, pH 7.4, 0.15 mol/L NaCl, 0.5 mol/L imidazole) as mobile phases (the concentration of solution B was increased from 4% (volume) to 100% (volume)). The fractions of interest were collected and proceeded to separation by SEC. HiLoad 26/60 Superdex-200 pre-grade gel column (GE, USA) was used. The solution C (20 mmol/L PB, pH 7.4, 0.15 mol/L NaCl) was used as the mobile phase and the elution rate was 2.5 mL/min. The chromatograms after purification by SEC are shown in Figure 5 and Figure 8.

The purified protein of NTD-RBD-foldon-6 × His (Gamma variant/Beta variant) was obtained. The protein purification results are shown in Table 21. The purity of target protein after purification was 93.6% (Gamma variant) and 95% (Beta variant).

**Table 21 Results of Protein Purification**

| Fusion protein | Purity (%) | Concentration (mg/mL) | Volume (mL) | Protein yield (mg) |
|---|---|---|---|---|
| NTD-RBD-foldon (Gamma variant) | 93.6 | 1.95 | 11.32 | 22.07 |
| NTD-RBD-foldon (Beta variant) | 95 | 1.87 | 9.89 | 18.49 |

### SDS-PAGE and Western blot validation:

A. 1.0 mm PAGE gel; loading volume 8 µL; gel running sequence: M, molecular weight marker. Me,culture medium. FT, flow through. W, wash. E, eluted fractions. B. Electrophoresis: the purified protein of NTD-RBD-foldon (Gamma variant/Beta variant) was mixed with loading buffer (NTD-RBD-foldon (Gamma variant/Beta variant) purified protein: 5 × loading buffer = 4: 1), boiled for 5 min, and then spotted. Firstly, electrophoresis was performed at 100 V constant voltage, and it could be seen that the Marker gradually became a thin line. After the Marker entered the separation gel, the voltage was adjusted to 300 V and electrophoresis was continued until the blue bromophenol blue band reached the bottom of the gel (approximately 25 min). C. Transfer: the glass plate was pried open, and the gel was carefully removed. The PVDF membrane was activated with methanol for 30s, then it was cut into the same size as the gel with the filter paper and soaked in the transfer solution, with the order of from bottom to top was: filter paper-PVDF membrane-gel-filter paper. It was ensured that there were no air bubbles between the layers, especially between the gel and the membrane. All the transfer solution in the plate was poured into the transfer cassette. The solution was transferred at 20V constant voltage. And this process required 20 min for 50 KDa protein. The higher the molecular weight, the longer the duration was required. D. Antibody incubation: the PVDF membrane was removed after antibody incubation and transfer, and it could be seen that the Marker had been transferred onto the membrane. The membrane was blocked with 5% non-fat dry milk (1g of skimmed milk powder in 100 ml PBST) for 1 h and rinsed with PBST for 2 × 5 min. The primary antibody (anti-His mab) was diluted with 5% non-fat dry milk (1 g of skimmed milk powder, 100 ml of TBS) and incubated in a small box and allowed to react for 2 h in a decolorization shaker at 4°C. The PVDF membrane was removed and rinsed with PBST for 4 × 10 min. similarly, the secondary antibody (goat anti-mouse) was diluted in 5% non-fat dry milk and incubated for 1 h and then rinsed for 4 × 10 min with PBST. All the samples were shaken with a shaker. E. Visualization with ECL: 1 mL of solution A and 10 µl of solution B were mixed and dripped onto each PBST membrane, and then photographed and recorded in the chemiluminescence imaging system after 5 min, as set forth in Figure 6 and Figure 9. As set forth in the SDS-PAGE and Western blot band patterns, the thickest band in the electrophoretic lane of NTD-RBD-foldon (Beta variant) solution after SEC separation was NTD-RBD-foldon (Beta variant), and the thickest band in the electrophoretic lane of NTD-RBD-foldon (Gamma variant) solution was NTD-RBD-foldon (Gamma variant), which mainly existed in the form of trimers. The corresponding target band could be seen in the Western blot result pattern.

### Example 27: Construction of Vectors and Protein Expression

Construct the following target fragments to be expressed, as set forth in Table 22, and express the purified protein with reference to the examples mentioned above.

**Table 22 Different Target Fragments**

| Gene name | Construction mode | SEQ ID NO. (not containing signal peptide and 6 × His) |
|---|---|---|
| NTD-RBD-foldon-6×His(WIV04-1) | Total gene synthesis | 96 |
| RBD-foldon-6× His (Beta variant) | Total gene synthesis | 80 |
| NTD-foldon-6× His (Beta variant) | Total gene synthesis | 81 |
| RBD-foldon-6×His (Gamma variant) | Total gene synthesis | 86 |
| NTD-foldon-6×His (Gamma variant) | Total gene synthesis | 87 |

### ***Example 28 Preparation of Immunogenic Compositions

50 µg of NTD-RBD-foldon (Gamma variant/Beta variant) purified protein, 1%(w/v) sucrose, 2%(w/v) glycine, 0.02%(w/v) Tween 80 and 10 mM PB buffer (Na₂HPO₄, NaH₂PO₄) at pH 7.5 were aliquoted into 2 mL vials with each vial containing 0.5 mL of the mixture, and placed into a lyophilizer for freeze-drying.

50 µg of NTD-RBD-foldon (Gamma variant/Beta variant) purified protein, 50 µg of RBD-foldon (Beta variant/Gamma variant) purified protein, 1%(w/v) sucrose, 2%(w/v) glycine, 0.02%(w/v) Tween 80 and 10 mM PB buffer (Na₂HPO₄, NaH₂PO₄) at pH 7.5 were aliquoted into 2 mL vials with each vial containing 0.5 mL of the mixture, and placed into a lyophilizer for freeze-drying.

50 µg of NTD-RBD-foldon (Gamma variant/Beta variant) purified protein, 50 µg of NTD-foldon (Beta variant/Gamma variant) purified protein, 1%(w/v) sucrose, 2%(w/v) glycine, 0.02%(w/v) Tween 80 and 10 mM PB buffer (Na₂HPO₄, NaH₂PO₄) at pH 7.5 were aliquoted into 2 mL vials with each vial containing 0.5 mL of the mixture, and placed into a lyophilizer for freeze-drying.

50 µg of NTD-RBD-foldon (Gamma variant/Beta variant) purified protein, 50 µg of NTD-RBD-foldon (WIV04-1) purified protein, 1%(w/v) sucrose, 2%(w/v) glycine, 0.02%(w/v) Tween 80 and 10 mM PB buffer (Na₂HPO₄, NaH₂PO₄) at pH 7.5 were aliquoted into 2 mL vials with each vial containing 0.5 mL of the mixture, and placed into a lyophilizer for freeze-drying.

50 µg of NTD-RBD-foldon (Gamma variant/Beta variant) purified protein, 135 µg of HA protein (45 µg of H1N1 HA, 45 µg of H3N2 HA, 45 µg of B/Washington/02/2019 HA), 1%(w/v) sucrose, 2%(w/v) glycine, 0.02%(w/v) Tween 80 and 10 mM PB buffer (Na₂HPO₄, NaH₂PO₄) at pH 7.5 were aliquoted into 2 mL vials with each vial containing 0.5 mL of the mixture, and placed into a lyophilizer for freeze-drying. The selected HA proteins are shown in Table 23.

50 µg of NTD-RBD-foldon (Gamma variant/Beta variant) purified protein, 180 µg of HA protein (45 µg of H1N1 HA, 45 µg of H3N2 HA, 45 µg of B/Washington/02/2019 HA and 45 µg of B/Phuket/3 073/2013 HA), 1%(w/v) sucrose, 2%(w/v) glycine, 0.02%(w/v) Tween 80 and 10 mM PB buffer (Na₂HPO₄, NaH₂PO₄) at pH 7.5 were aliquoted into 2 mL vials with each vial containing 0.5 mL of the mixture, and placed into a lyophilizer for freeze-drying. The selected HA proteins are shown in Table 23.

**Table 23 Influenza Strains Used in Combination**

| Influenza strain | | SEQ ID NO. |
|---|---|---|
| Type A (H1N1) | A/Wisconsin/588/2019 | 92 |
| Type A (H3N2) | A/Cambodia/e0826360/20 20 | 93 |
| Type B | B/Washington/02/2019 | 94 |
| Type B | B/PHUKET/3073/2013 | 95 |

### ***Example 29: Preparation of Vaccine

The immunogenic compositions of any one of the aforesaid embodiments further comprises an aluminium hydroxide adjuvant at a concentration of 1 mg/mL.

The immunogenic compositions of any one of the aforesaid embodiments further comprises adjuvant AS03. Each 0.5 mL adjuvant vial containing the components of 10.69 mg of squalene, 11.86 mg of α-tocopherol, 4.86 mg of polysorbate 80, 3.53 mg of sodium chloride, 0.09 mg of potassium chloride, 0.51 mg of dibasic sodium phosphate, 0.09 mg of monobasic potassium phosphate and water for injection.

The immunogenic compositions of any one of the aforesaid embodiments further comprises adjuvant MF59. Each 0.5 mL adjuvant vial containing the components of 4.5% squalene, 0.5% Tween 80, 0.5% span 85 and water for injection.

### Example 30: Immunization Experiment with Vaccine in Mice (Beta Variant)

The immunogenic compositions of Example 28 were selected and combined with adjuvant AS03 to immunize the mice at a dose of 2/25 of the human dose. The immunogenic composition prepared with NTD-RBD-foldon (WIV04-1) protein combined with adjuvant AS03 was used as the control. A VSV (vesicular stomatitis virus) system-based method for detecting the neutralizing antibody against SARS-CoV-2 pseudovirus was used to measure the level of neutralizing antibodies induced. The grouping of mice and the immunization schedule are shown in Table 24.

**Table 24 Grouping of Mice for Immunization**

| Antigen (NTD-RBD-foldon) | Adjuvant | Antigen dose | Immunization procedure | Immunization route | Number of animals |
|---|---|---|---|---|---|
| Beta variant | AS03 | 4µg | 2 doses 14 days apart | Intramuscular injection | 10 |
| WIV04-1 | AS03 | 4µg | 2 doses 14 days apart | Intramuscular injection | 10 |
| WIV04-1 + Beta variant | AS03 | (2+2) µg | 2 doses 14 days apart | Intramuscular injection | 10 |

The adjuvant AS03 was prepared with the reference to example 29. Serum was collected from the orbital vein of the mice 28 days after immunization. The titers of neutralizing antibody were detected by a VSV-based pseudovirus detection system. The neutralizing assay was performed with the pseudoviruses of the following SARS-COV-2 strains, namely the main epidemic strain (D614G), alpha variant strain (B.1.1.7), Beta variant strain (501Y. V2), and Gamma variant (501Y. V3), respectively, with the aim to investigate the neutralizing effect of the vaccine against different SARS-COV-2 variants. The test results are shown in Table 25. The results showed that the neutralizing antibody induced by NTD-RBD-foldon (WIV04-1) in combination with adjuvant AS03 had a good neutralizing effect with the GMT much higher than 10000 on the main epidemic pseudoviruses and Gamma variant pseudoviruses, but it exhibited a poor neutralizing effect on Alpha variant pseudoviruses and Beta variant pseudoviruses with the GMT much lower than 10000. The neutralizing antibody induced by NTD-RBD-foldon (Beta variant) in combination with adjuvant AS03 showed a good neutralizing effect on Beta variant pseudoviruses as well as Gamma variant pseudoviruses with the GMT much higher than 10000. More surprisingly, the GMT of the neutralizing antibodies against Gamma variant pseudoviruses was as high as 20944, but its neutralizing effect on major epidemic pseudoviruses and Alpha variant pseudoviruses was slightly weaker. It can be seen that the monovalent vaccine using NTD-RBD-foldon (Beta variant) as the immunogen has a good immunizing effect on all the 4 SARS-CoV-2 variants, and in particular, this protein can be used as a new COVID-19 vaccine candidate against SARS-CoV-2 variants (especially the South African and Gamma variant) to induce in a more efficient production manner of protective neutralizing antibodies to prevent the immune escape of SARS-CoV-2 variants.

Compared with the monovalent vaccine prepared with NTD-RBD-foldon (Beta variant) or NTD-RBD-foldon (WIV04-1), the bivalent vaccine prepared with NTD-RBD-foldon (Beta variant) combined with NTD-RBD-foldon (WIV04-1) as the immunogens could induce neutralizing antibodies at an overall increased level. So, it enhanced the effect of the monovalent vaccine of NTD-RBD-foldon (WIV04-1) against the Beta variant pseudoviruses and that of the monovalent vaccine of NTD-RBD-foldon (Beta variant) against both the main epidemic pseudoviruses and British epidemic strain pseudoviruses. This bivalent vaccine had the characteristics of wider scope of action and more potent effect and was more suitable as a candidate vaccine to prevent COVID-19.

**Table 25 Test Results 28 Days after Immunization of Mice**

| Antigen (NTD-RBD-foldon) | Adjuv ant | Dosage | Pseudovirus type | Geometric mean titer (GMT) |
|---|---|---|---|---|
| Beta variant | AS03 | 4µg | Main epidemic strain (D614G mutation) | 6905 |
| | | | Alpha variant (B.1.1.7) | 2848 |
| | | | Beta variant (501Y. V2) | 10464 |
| | | | Gamma variant (501Y. V3) | 20944 |
| WIV04-1 | | 4µg | Main epidemic strain (D614G mutation) | 14349 |
| | | | Alpha variant (B.1.1.7) | 5693 |
| | | | Beta variant (501Y. V2) | 7703 |
| | | | Gamma variant (501Y. V3) | 16967 |
| WIV04-1 + Beta variant | | (2+2)µg | Main epidemic strain (D614G mutation) | 13211 |
| | | | Alpha variant (B.1.1.7) | 5013 |
| | | | Beta variant (501Y. V2) | 11979 |
| | | | Gamma variant (501Y. V3) | 21795 |

### Example 31: Immunization Experiment with Vaccine in Mice (Gamma variant)

The immunogenic compositions of example 28 were selected and combined with adjuvant AS03 to immunize the mice at 2/25 of the human dose. The level of neutralizing antibodies induced was measured by a VSV (vesicular stomatitis virus) system-based method for detecting the neutralizing antibody against SARS-CoV-2 pseudovirus. The grouping of mice and the immunization schedule are shown in Table 26.

**Table 26 Grouping of Mice for Immunization**

| Antigen | Adjuvant | Antigen dose | Immunization procedure | Immunization route | Number of animals |
|---|---|---|---|---|---|
| NTD-RBD-foldon (Gamma variant) | AS03 | 4µg | 2 doses 14 days apart | Intramuscular injection | 10 |

The adjuvant AS03 was prepared with the reference to example 29. Serum was collected from the orbital vein of the mice 28 days after immunization. The titers of neutralizing antibody were detected by a VSV-based pseudovirus detection system. The neutralizing assay was performed with the pseudoviruses of the following SARS-COV-2 strains, namely the main epidemic strain (D614G), alpha variant strain (B.1.1.7), Beta variant strain (501Y. V2), and Gamma variant strain (501Y. V3), respectively, with the aim to investigate the neutralizing effect of the vaccine against different SARS-COV-2 variants. The test results, as set forth in Table 27, showed that NTD-RBD-foldon (Gamma variant) as the antigen combined with adjuvant AS03 induced titer of neutralizing antibodies against the Beta variant (501Y. V2) pseudovirus at the highest level, followed by that of against Gamma variant (501Y. V3) pseudovirus, and last comes that of the main epidemic strain (D614G) pseudovirus as well as Alpha variant (B. 1.1.7) pseudovirus. It can be seen that the monovalent vaccine using NTD-RBD-foldon (Gamma variant) as the immunogen has an immunizing effect on all the 4 SARS-CoV-2 variants, and this protein can be used as a new COVID-19 vaccine candidate against SARS-CoV-2 variants (especially the South African and Gamma variant) to induce in a more efficient production manner of protective neutralizing antibodies to prevent the immune escape of SARS-CoV-2 variants.

**Table 27 Test Results 28 Days after Immunization of Mice**

| Antigen | Pseudovirus type | Geometric mean titer (GMT) |
|---|---|---|
| NTD-RBD-foldon (Gamma variant) | Main epidemic strain (D614G mutation) | 1408 |
| | Alpha variant (B.1.1.7) | 615 |
| | Beta variant (501Y. V2) | 5544 |
| | Gamma variant (501Y. V3) | 5186 |

To sum up, it can be determined that higher titers of neutralizing antibodies may be produced by the bivalent vaccine prepared with NTD-RBD-foldon (Gamma variant) combined with NTD-RBD-foldon (Beta variant), or the bivalent vaccine prepared with NTD-RBD-foldon (Gamma variant) combined with NTD-RBD-foldon (WIV04-1), or the trivalent vaccine prepared with NTD-RBD-foldon (Gamma variant) combined with NTD-RBD-foldon (Beta variant) and NTD-RBD-foldon (WIV04-1), or the multivalent vaccine prepared by NTD-RBD-foldon (Gamma variant) combined with NTD-RBD-foldon (Beta variant), NTD-RBD-foldon (WIV04-1) and influenza hemagglutinin HA and so on.

## Claims

1. A fusion protein comprising: 1) the receptor-binding domain (RBD) of the SARS-CoV-2 spike protein (S protein) or functionally active fragment thereof, and 2) the N-terminal domain (NTD) of the SARS-CoV-2 spike protein (S protein) or functionally active fragment thereof.

2. The fusion protein of claim 1, wherein said RBD and NTD are derived from a wild-type SARS-CoV-2 strain or variants thereof.

3. The fusion protein of claim 2, wherein said SAR-CoV-2 variant is selected from any one of the following group consisting of a Gamma variant, a Beta variant, a Delta variant, and an Alpha variant.

4. The fusion protein of any one of claims 1-3, wherein said RBD has a mutation in one or more amino acid sites selected from the following group consisting of: K417, L452, T478, E484 and N501.

5. The fusion protein of any one of claims 1-4, wherein said RBD comprises one or more of the amino acid mutations of K417T/N, L452R, T478K, E484K and N501Y.

6. The fusion protein of any one of claims 1-5, wherein said RBD comprises the K417T, E484K and N501Y amino acid mutations.

7. The fusion protein of any one of claims 1-6, wherein said RBD comprises the K417N, E484K and N501Y amino acid mutations.

8. The fusion protein of any one of claims 1-7, wherein said RBD comprises the amino acid sequence as set forth in any one of SEQ ID NOs.: 18, 19, 76, 83 and 97-108.

9. The fusion protein of any one of claims 1-8, wherein said NTD comprises a mutation at one or more amino acid sites selected from the following groups consisting of L18, T19, T20, P26, D80, D138, R190, D215, L242-244, and R246.

10. The fusion protein of any one of claims 1-9, wherein said NTD comprises the mutations of one or more amino acids selected from the following group consisting of L18F, T19R, T20N, P26S, D80A, D138Y, R190S, D215G, L242-244del, and R246I.

11. The fusion protein of any one of claims 1-10, wherein said NTD comprises L18F, D80A, D215G, L242-244del and R246I amino acid mutations.

12. The fusion protein of any one of claims 1-11, wherein said NTD comprises L18F, T20N, P26S, D138Y and R246I amino acid mutations.

13. The fusion protein of any one of claims 1-12, wherein said NTD comprises an amino acid sequence as set forth in any one of SEQ ID NOs: 37, 38, 77, 84 and 109-111.

14. The fusion protein of any one of claims 1-13, which further includes one or more polypeptides selected from the following group: P2 or functionally active fragment thereof, foldon domain or functionally active fragment thereof, ferritin or functionally active fragment thereof, and hepatitis B surface antigen (HBsAg) or functionally active fragment thereof.

15. The fusion protein of any one of claims 1-14, wherein said RBD or functionally active fragment thereof is directly or indirectly linked to said NTD or functionally active fragment thereof.

16. The fusion protein of any one of claims 1-15, wherein said RBD or functionally active fragment thereof is in-frame fusion to said NTD domain or functionally active fragment thereof.

17. The fusion protein of any one of claims 14-16, wherein said polypeptide is linked directly or indirectly to said RBD or a functional fragment thereof and/or said NTD or functionally active fragment thereof.

18. The fusion protein of any one of claims 14-17, wherein said polypeptide is in-frame fusion to said RBD or functionally active fragment thereof and/or said NTD or functionally active fragment thereof.

19. The fusion protein of any one of claims 14-18, wherein said N-terminal of the polypeptide is directly or indirectly linked to the C-terminal of said RBD or functionally active fragment thereof, and the N-terminal of said RBD or functionally active fragment thereof is directly or indirectly linked to the C-terminal of said NTD or functionally active fragment thereof.

20. The fusion protein of any one of claims 14-19, wherein said P2 or functionally active fragment thereof comprises an epitope peptide of tetanus toxin.

21. The fusion protein of claim 20, wherein said epitope peptide of tetanus toxin comprises the amino acid sequence as set forth in any one of SEQ ID NOs.: 64-66.

22. The fusion protein of any one of claims 14-21, wherein said foldon domain or functionally active fragment thereof comprises C-terminal amino acid residues C-terminal domain of bacteriophage T4 fibrin.

23. The fusion protein of any one of claims 14-22, wherein said foldon domain or functionally active fragment thereof comprises an amino acid sequence as set forth in any one of SEQ ID NOs.: 67-69 and 78.

24. The fusion protein of any one of claims 14-23, wherein the N-terminal of said foldon domain or a functionally active fragment thereof is directly or indirectly linked to the C-terminal of said P2 or functionally active fragment thereof, the N-terminal of said P2 or functionally active fragment thereof is directly or indirectly linked to the C-terminal of said RBD or functionally active fragment thereof, and the N-terminal of said RBD or functionally active fragment thereof is directly or indirectly linked to the C-terminal of said NTD or functionally active fragment thereof.

25. The fusion protein of any one of claims 14-24, wherein said ferritin or functionally active fragment thereof comprises *trichoplusia ni* ferritin, *Helicobacter pylori* ferritin or functionally active fragment thereof.

26. The fusion protein of claim 25, wherein said *trichoplusia ni* ferritin or functionally active fragment thereof comprises a heavy chain or a light chain of *trichoplusia ni* ferritin.

27. The fusion of claim 26, wherein said heavy chain of *trichoplusia ni* ferritin comprises the amino acid sequence as set forth in SEQ ID NO: 70 and said light chain of *trichoplusia ni* ferritin comprises the amino acid sequence as set forth in SEQ ID NO: 71.

28. The fusion protein of any one of the claims 14-27, wherein said ferritin or functionally active fragment thereof comprises an amino acid sequence as set forth in any one of SEQ ID NOs.: 70-72.

29. The fusion protein of any one of claims 14-28, wherein said hepatitis B surface antigen or functionally active fragment thereof comprises an amino acid sequence as set forth in SEQ ID NO: 73.

30. The fusion protein of any one of claims 15-29, wherein said direct or indirect linking comprises the linking via a linker.

31. The fusion protein of claim 30, wherein said linker comprises a rigid linker, a flexible linker or other sequences.

32. The fusion protein of any one of claims 30-31, wherein said linker comprises an amino acid sequence as set forth in any one of SEQ ID NOs.: 89-90.

33. The fusion protein of any one of claims 1-32, comprising an amino acid sequence as set forth in any one of SEQ ID NOs.: 39-44, 79, 85 and 96.

34. An immunogenic composition, comprising the fusion protein of any one of claims 1-33.

35. The immunogenic composition of claim 34, which further comprises at least one kind of hemagglutinin protein HA from human influenza virus.

36. The immunogenic composition of claim 35, wherein said HA comprises an amino acid sequence in any one of SEQ ID NOs.: 92-95.

37. One or more isolated nucleic acid molecules, which encode(s) the fusion protein in any one of claims 1-33.

38. The nucleic acid molecule of claim 37, which comprises mRNA.

39. A pharmaceutical composition, which comprises the fusion protein of any one of claims 1-33, or the immunogenic composition of any one of claims 34-36, or the nucleic acid molecule of any one of claims 37-38, along with arbitrary pharmaceutically acceptable excipient.

40. The use of the fusion protein of any one of claims 1-33, or the immunogenic composition of any one of claims 34-36, or the nucleic acid molecule of any one of claims 37-38, in the preparation of a vaccine.

41. The use of claim 40, wherein said vaccine is used for the prevention and/or treatment of COVID-19.

42. The fusion protein of any one of claims 1-33, or the immunogenic composition of any one of claims 34-36, or the nucleic acid molecule of any one of claims 37-38 for use in the treatment and/or prevention of COVID-19.

43. A method for making a COVID-19 subunit vaccine, which comprises:
1) preparing the fusion protein of any one of claims 1-33, or the immunogenic composition of any one of claims 34-36, or the nucleic acid molecule of any one of claims 37-38; and
2) mixing the fusion protein, the immunogenic composition or the nucleic acid in 1) with a pharmaceutically acceptable adjuvant.

44. A method for detecting SARS-CoV-2 neutralizing antibodies, which comprises:
1) administering the COVID-19 subunit vaccine of claim 43 to the subjects; and
2) detecting the neutralizing antibodies produced in the subjects in 1) after receiving said COVID-19 subunit vaccine.

45. A method of treating and/or preventing COVID-19, which comprises administering the fusion protein of any one of claims 1-33, or the immunogenic composition of any one of claims 34-36, or the nucleic acid molecule of any one of claims 37-38, or the COVID-19 subunit vaccine of claim 43, to the subjects.
